(19) Europäisches Patentamt
European Patent Office
Office européen des brevets

(11) Veröffentlichungsnummer: **0 034 347**
**B1**

(12) # EUROPÄISCHE PATENTSCHRIFT

(45) Veröffentlichungstag der Patentschrift: **09.04.86**

(51) Int. Cl.⁴: **C 07 H 15/00**, A 61 K 31/70, C 07 K 5/00, A 23 K 1/16

(21) Anmeldenummer: **81101017.2**

(22) Anmeldetag: **13.02.81**

(54) **Derivate von Aldohexosen, Zwischenprodukte, Verfahren zu ihrer Herstellung, Präparate enthaltend solche Verbindungen und ihre Verwendung.**

(30) Priorität: **15.02.80 CH 1265/80**

(43) Veröffentlichungstag der Anmeldung: **26.08.81 Patentblatt 81/34**

(45) Bekanntmachung des Hinweises auf die Patenterteilung: **09.04.86 Patentblatt 86/15**

(84) Benannte Vertragsstaaten: **AT BE CH DE FR IT LI LU NL SE**

(56) Entgegenhaltungen:
**EP - A - 0 003 833**
**EP - A - 0 004 512**
**EP - A - 0 014 984**
**EP - A - 0 015 468**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Klybeckstrasse 141, CH-4002 Basel (CH)**

(72) Erfinder: **Hartmann, Albert, Dr., Steingasse 21A, D-7889 Grenzach (DE)**
Erfinder: **Baschang, Gerhard, Dr., Bückenweg 7, CH-4126 Bettingen (CH)**
Erfinder: **Wacker, Oskar, Dr., Löwenbergstrasse 60, CH-4059 Basel (CH)**
Erfinder: **Stanek, Jaroslav, Dr., Florastrasse 6, CH-4127 Birsfelden (CH)**

(74) Vertreter: **Zumstein, Fritz, Dr. et al, Bräuhausstrasse 4, D-8000 München 2 (DE)**

EP 0 034 347 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft neue Derivate von Aldohexosen, Zwischenprodukte und Verfahren zu ihrer Herstellung, pharmazeutische Präparate, die solche Verbindungen enthalten, und ihre Verwendung zur Herstellung von pharmazeutischen Präparaten oder als pharmakologisch wirksame Verbindungen.

Die Erfindung betrifft insbesondere Derivate von Pyranosen der Formel (I),

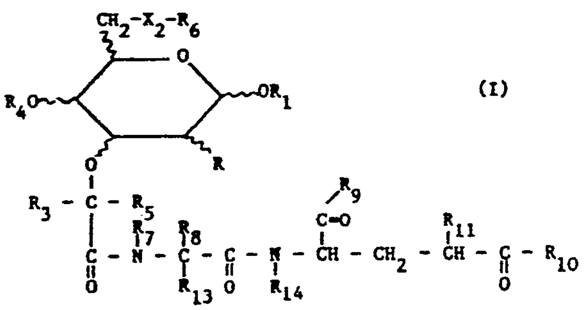

(I)

worin R für Hydroxy, Amino oder einen Rest der Formel

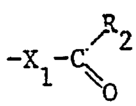

steht,

$X_1$ und $X_2$ unabhängig voneinander für $NR_{15}$ oder ein Sauerstoffatom stehen, wobei $R_{15}$ Wasserstoff oder Niederalkyl bedeutet, $R_1$ und $R_4$ unabhängig voneinander für Wasserstoff, Acyl oder eine Hydroxyschutzgruppe, $R_6$ für Wasserstoff, Acyl oder, falls $X_2$ eine Sauerstoffatom bedeutet, für eine Hydroxyschutzgruppe stehen, $R_2$ jeweils gegebenenfalls substituiertes Alkyl, Aryl oder Alkoxy darstellt, $R_3$, $R_5$, $R_7$, $R_{13}$ und $R_{14}$ unabhängig voneinander für Wasserstoff oder Niederalkyl stehen und $R_8$ einen Niederalkyl- oder Phenylniederalkylrest darstellt, der auch mit $R_7$ verbunden sein kann, und der eine Oxycarbonyl-, Mercaptocarbonyl- oder Aminocarbonylgruppe trägt, welche mit einem gegebenenfalls substituierten aliphatischen, cycloaliphatisch-aliphatischen, cycloaliphatischen oder aromatischen Kohlenwasserstoffrest $R_o$ verbunden ist, welch letzterer durch Oxycarbonyl, Mercaptocarbonyl und/oder Iminocarbonyl unterbrochen sein kann und der mehr als 5 C-Atome aufweisen muss, wenn $R_1$, $R_4$ und $R_6$ Wasserstoff bedeuten, worin die Reste $R_9$ und $R_{10}$ für gegebenenfalls veräthertes Hydroxy oder gegebenenfalls substituiertes Amino stehen, und $R_{11}$ Wasserstoff oder einen Rest der Formel -C(=O)-$R_{12}$ (Ia) bedeutet, worin $R_{12}$ für gegebenenfalls veräthertes Hydroxy oder gegebenenfalls substituiertes Amino steht, und Salze von Verbindungen der Formel I mit mindestens einer salzbildenden Gruppe, und Verfahren zur Herstellung von Verbindungen der Formel I und von Salzen von solchen Verbindungen mit salzbildenden Gruppen, ferner pharmazeutische Präparate enthaltend Verbindungen der Formel I oder Salze von solchen Verbindungen mit mindestens einer salzbildenden Gruppe, sowie die Verwendung der Verbindungen der Formel I und von Salzen von solchen Verbindungen mit salzbildenden Gruppen.

Die Formel I umschreibt Derivate einer Aldohexose, insbesondere beispielsweise der D-Mannose oder der D-Galaktose, in allererster Linie aber der D-Glucose.

Die Verbindungen der Formel (I) können als reine Isomere oder als Isomerengemische vorliegen. Beispielsweise kann am C-1 des Zuckerteils die α- oder β-Konfiguration vorliegen. Bei asymmetrischer Substitution sind die Konfigurationen am C-$R_3$ in erster Linie (D), daneben aber auch (L), am C-$R_5$-(D) oder (L), vornehmlich jedoch (L), und am CH-$NR_{14}$vornehmlich (D).

Als bevorzugte Verbindungen der Formel (I) seien diejenigen genannt, die sich von der D-Clucose ableiten und die Konfiguration (D) am C-$R_3$, die (L)-Konfiguration am C-$R_8$ und die (D)-Konfiguration am CH-$NR_{14}$ aufweisen.

Muramylverbindungen weisen am C-$R_3$ die (D)-Konfiguration, Isomuramylverbindungen die (L)-Konfiguration auf. Muraminsäure ist 2-Amino-2-desoxy-3-O-(1-carboxy-äthyl)-D-glucose. Normuraminsäure ist 2-Amino-2-desoxy-3-O-carboxymethyl-D-glucose.

Im vorliegenden Zusimmenhang enthalten mit "nieder" bezeichnete Reste und Verbindungen bis und mit 7, in erster Linie bis und mit 4 Kohlenstoffatome. Substituierte Reste können einen oder mehrere, gleiche oder verschiedene Substituenten aufweisen. Reste, bei denen die Anzahl der Atome nicht angegeben ist, enthalten nicht mehr als 90, insbesondere nicht mehr als 30, und vorzugsweise weniger als 20 C-Atome.

Die Reste $X_1$ und $X_2$ können die genannten Bedeutungen haben, $X_1$ steht aber vornehmlich für $NR_{15}$, insbesondere für NH, während $X_2$ vornehmlich für ein Sauerstoffatom steht.

Ein Acylrest $R_1$, $R_4$ oder $R_6$ kann für Formyl stehen, ist aber vorzugsweise gegebenenfalls substituiertes Hydrocarbylcarbonyl oder Heterocyclylcarbonyl, worin Hydrocarbyl einen aliphatischen, cycloaliphatischen, aromatischen, cycloaliphatisch-aliphatischen oder araliphatischen Kohlenwasserstoffrest und Heterocyclyl insbesondere einen 5-oder 6-gliedrigen Ring mit einem oder mehreren Sauerstoff-, Stickstoff- und/ oder Schwefelatomen als Ringgliedern bedeuten.

Insbesondere steht ein Acylrest $R_1$, $R_4$ oder $R_6$ für gegebenenfalls ungesättigtes Alkanoyl mit 2-80, in erster Linie mit 2-25 C-Atomen oder für gegebenenfalls substituiertes Benzoyl und stellt beispielsweise Niederalkanoyl, vornehmlich

Acetyl, Benzoyl oder den Acylrest einer Fettsäure, wie Stearoyl, Oleoyl oder Palmitoyl dar, wobei die letztgenannten Acylreste vornehmlich durch den Rest $R_6$ repräsentiert werden. Daneben kann beispielsweise vor allem der Rest $R_6$ für den obengenannten und weiter unten definierten aliphatischen, cycloaliphatischen oder cycloaliphatisch-aliphatischen Rest

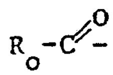

stehen. Als Beispiele für die letztgenannten Reste $R_6$ seien genannt: N-(D,L-2-Hydroxy-myristoyl)-glycyl, N-(Cholestan-3(α,β)-yl-acetyl)-glycyl, N-Cholyl-glycyl
N-(D,L-2-n-Hexadecanoylamino-n-hexadecanoyl)-glycyl, N-Stearoyl-L-alanyl und 11-Stearoylamino-n-undecanoyl.

Hydroxyschutzgruppen $R_1$, $R_4$ und/oder $R_6$ sind z.B. bestimmte Acylreste, wie gegebenenfalls, z.B. durch Halogen, substituiertes Niederalkanoyl, wie 2,2-Dichloracetyl, oder Acylreste von Kohlensäurehalbestern, insbesondere tert.-Butyloxycarbonyl, gegebenenfalls substituiertes Benzyloxycarbonyl, z.B. 4-Nitrobenzyloxycarbonyl, oder Diphenylmethoxycarbonyl oder 2-Halogen - niederalkoxycarbonyl, wie 2,2,2-Trichloräthoxycarbonyl, ferner Trityl oder Formyl, oder organische Silyl- oder Stannylreste, ferner leicht abspaltbare veräthernde Gruppen, wie tert.-Niederalkyl, z.B. tert.-Butyl, 2-oxa- oder 2-thiaaliphatische oder -cycloaliphatische Kohlenwasserstoffreste, in erster Linie 1-Niederalkoxyniederalkyl oder 1-Niederalkyl-thioniederalkyl, z.B. Methoxymethyl, 1-Methoxyäthyl, 1-Aethoxy-äthyl, 1-Methylthiomethyl, 1-Methylthioäthyl oder 1-Aethylthioäthyl, oder 2-Oxa- oder 2-Thiacycloalkyl mit 5-7 Ringatomen, z.B. 2-Tetrahydrofuryl oder 2-Tetrahydropyranyl oder entsprechende Thiaanaloge, sowie gegebenenfalls substitniertes-1-Phenylniederalkyl, wie gegebenenfalls substituiertes Benzyl oder Diphenylmethyl, wobei als Substituenten der Phenylreste z.B. Halogen, wie Chlor, Niederalkoxy, wie Methoxy, und/oder Nitro in Frage kommen.

Die oben erwähnten organischen Silyl- oder Stannylreste enthalten vorzugsweise Niederalkyl, insbesondere Methyl, als Substituenten der Silizium- oder Zinn-Atome. Entsprechende Silyl- oder Stannylgruppen sind in erster Linie Triniederalkylsilyl, insbesondere Trimethylsilyl, ferner Dimethyl-tert.-butyl-silyl, oder entsprechend substituiertes Stannyl, z.B. Tri-n-butylstannyl.

Hervorzuheben als Hydroxyschutzgruppen $R_4$ und $R_6$ sind gegebenenfalls substituierte Alkylidenreste, die die Sauerstoffatome in 4- und 6-Stel-lung verbinden. Alkylidenreste sind insbesondere Niederalkyliden, in erster Linie Metbyliden, Isopropyliden oder Propyliden oder auch ein gegebenenfalls substituierter Benzylidenrest. Hervorzuheben als Hydroxyschutzgruppe $R_1$ ist Benzyl.

Bevorzugterweise stehen die Reste $R_3$, $R_5$, $R_7$, $R_{13}$ und $R_{14}$für Wasserstoff oder Methyl.

In erster Linie stehen $R_3$ für Methyl und $R_5$ für Wasserstoff oder$R_3$ und $R_5$ beide jeweils für Wasserstoff bzw. für Methyl.

Substituenten von substituiertem Alkyl bzw. Alkoxy $R_2$ sowie von substituierten aliphatischen, cycloaliphatisch-aliphatischen oder cycloaliphatischen Resten in $R_8$ sind z.B. gegebenenfalls funktionell abgewandeltes, insbesondere gegebenenfalls veräthertes oder verestertes Aydroxy oder- Mercapto, wie Hydroxy, Alkoxy, z.B. Niederalkoxy, Alkanoyloxy, z.B. Niederalkanoyloxy, oder Halogen, ferner Mercapto oder niederes oder höheres Alkylthio, Oxo oder gegebenenfalls substituiertes Amino, wie Acylamino, z.B. Alkanoylamino.

Gegebenenfalls substituiertes Aryl $R_2$ oder ein gegebenenfalls substituierter aromatischer Kohlenwasserstoffrest in Rest $R_8$ ist in erster Linie gegebenenfalls substituiertes Phenyl. Gegebenenfalls substituiertes Alkoxy $R_2$ ist insbesondere Phenylniederalkoxy. Substituenten von pbenyl und Phenylniederalkoxy, wobei in letzterem sowohl der Phenylteil als aucb der Niederalkoxyteil substituiert sein kann, sind in einem Phenylteil z.B. Niederalkyl oder gegebenenfalls funktionell abgewandeltes, insbesondere gegebenenfalls verätbertes oder verestertes Hydroxy, wie Hydroxy, Niederalkoxy oder Halogen, und in einem Niederalkoxyteil z.B. gegebenenfalls funktionell abgewandeltes, insbesondere gegebenenfalls veräthertes oder verestertes Hydroxy, wie Hydroxy, Niederalkoxy, Niederalkanoyloxy oder Halogen.

Im Niederalkyl- oder Phenylniederalkylrest $R_8$ ist der Niederalkylrest gerade oder verzweigt. Er kann auch mit $R_7$ verbunden sein und bildet dann zusammen mit der Gruppierung

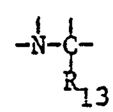

einen stickstoffhaltigen heterocyclischen Ring, der z.B. 5 und bis und mit 7 Ringglieder enthalten kann. Der Niederalkyl- oder Phenylniederalkylrest $R_8$ bzw. der genannte heterocyclische Ring trägt als Substituenten eine

Oxycarbonyl-

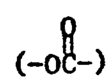,

Mercaptocarbonyl-

$$(-S\overset{\overset{O}{\|}}{C}-)$$

oder Aminocarbonyl-

$$(-NH\overset{\overset{O}{\|}}{C}-)$$

Gruppe, wobei als Verknüpfungsstelle sowohl das Sauerstoff-, Schwefelbzw. Stickstoffatom als auch das C-Atom der Carbonylgruppe in Frage kommt. Bevorzugterweise ist das Heteroatom mit dem Niederalkyl- oder Phenylniederalkylteil verknüpft, während das C-Atom der Carbonylgruppe mit den Kohlenwasserstoffrest verbunden ist. Auf der anderen Seite sind diese Substituenten mit einem gegebenenfalls substituierten aliphatischen, cycloaliphatisch-aliphatischen, cycloaliphatischen oder aromatischen Kohlenwasserstoffrest $R_o$ verbunden, der langkettig sein bzw. mehr als 5 Kohlenstoffatome haben muss, wenn $R_1$, $R_4$ und $R_6$ Wasserstoff bedeuten. Stellen dagegen beispielsweise $R_1$, $R_4$ und/oder $R_6$ einen Acyl, wie Alkanoylrest, z.B. einen Acetylrest dar, kann der obengenannte Kohlenwasserstoffrest auch weniger als 5 Kohlenstoffatome aufweisen.

Der langkettige aliphatische oder cycloaliphatisch-aliphatische Rest $R_o$ enthält z.B. bis und mit 90, vorzugsweise bis und mit 30 Kohlenstoffatome. Er kann verzweigt oder gerade, gesättigt oder ungesättigt sein und ist dann insbesondere ein gegebenenfalls cycloaliphatisch substituierter Alkyl- oder Alkenylrest. Diese Reste können sowohl im aliphatischen Teil als auch im cycloaliphatischen Teil wie oben erwähnt Substituenten tragen. Sie können auch durch weitere Oxycarbonyl-, Mercaptocarbonyl- oder Iminocarbonylgruppen unterbrochen sein. Dabei sitzt der cycloaliphatische Rest vorzugsweise direkt an einem dieser Trennglieder. Der zwischen dem ersten und dem gegebenenfalls vorhandenen zweiten Trennglied liegende aliphatische Rest ist insbesondere ein gerader oder verzweigter Alkylen- oder Alkenylenrest mit vorzugsweise 1 bis 15 Kohlenstoffatomen.

Der cycloaliphatische Rest, der Teil eines cycloaliphatisch-aliphatischen Restes sein kann, ist insbesondere ein mono- oder polycyclischer Rest, der gegebenenfalls durch freie, veresterte oder verätherte Hydroxygruppen, Halogenatome oder Oxogruppen substituiert und gesättigt oder ungesättigt sein kann. Er enthält z.B. bis zu 24 Kohlenstoffatome. Er ist insbesondere ein entsprechendes mono- oder polycyclisches Cycloalkyl oder Cycloalkenyl. Polycyclisches Cycloalkyl oder Cycloalkenyl ist in erster Linie tetracyclisches, z.B. Cyclopentanopolyhydrophenanthrenyl, das als Substituenten z.B. Hydroxy-, Alkoxy und/oder Halogen tragen und z.B. eine oder zwei Doppelbindungen aufweisen kann.

Als Beispiele für cycloaliphatisch-aliphatische Reste seien die Derivate der Cholansäure, wie Cholsäure und ihre Derivate genannt.

Ein gegebenenfalls substituierter aromatischer Koblenwasserstoffrest $R_o$ ist insbesondere ein gegebenenfalls substituierter Phenylrest, z.B. Phenyl oder 4-Methyl-phenyl.

Ein Niederalkylrest ist insbesondere ein Alkylrest mit 1-7 Kohlenstoffatomen, der z.B. in 2-Stellung gegebenenfalls funktionell abgewandeltes, insbesondere gegebenenfalls veräthertes oder verestertes Hydroxy oder Mercapto, wie Hydroxy, Alkoxy, z.B. Niederalkoxy, Alkanoyloxy, z.B. Niederalkanoyloxy oder Halogen, ferner Mercapto, oder Alkyl-, wie niederes oder höheres Alkylthio, oder gegebenenfalls substituiertes Amino, wie Acylamino, z.B. Alkamoylamino, tragen kann.

Im Phenylniederalkylrest $R_8$ enthält der Niederalkylrest insbesondere 1-3 Koblenstoffatome und ist in erster Linie Methyl.

Veräthertes Hydroxy $R_9$, $R_{10}$ oder $R_{12}$ ist z.B. durch einen aliphatischen Rest veräthertes Hydroxy, wie gegebenenfalls substituiertes Niederalkoxy oder durch eine Carboxylschutzgruppe geschütztes Hydroxy, wie Benzyloxy oder Silyloxy, z.B. Trimethylsilyloxy.

Gegebenenfalls substituiertes Amino $R_9$, $R_{10}$ oder $R_{12}$ enthält z.B. gegebenenfalls substituiertes Niederalkyl als Substituenten, wobei Niederalkyl z.B. durch gegebenenfalls funktionell abgewandeltes Hydroxy, Mercapto oder Carboxy, wie gegebenenfalls veräthertes oder verestertes Hydroxy oder Mercapto, z.B. Hydroxy, Niederalkoxy oder Niederalkylthio, oder gegebenenfalls verestertes oder amidiertes Carboxy, wie Carboxy, Niederalkoxycarbonyl oder Carbamoyl, substituiert sein kann.

Im vorliegenden Zusammenhang können die obengenannten Allgemeindefinitionen folgende Bedeutung haben:

Alkyl bedeutet u.a. Niederalkyl, z.B. Methyl, Aethyl, n-Propyl, Isopropyl, n-Butyl, Isobutyl oder tert.-Butyl, ferner n-Pentyl, Isopentyl, Neopentyl, n-Hexyl oder n-Heptyl, oder auch höheres Alkyl, wie geradkettiges oder verzweigtes Octyl, Nonyl, Decyl, Undecyl, Dodecyl, Tridecyl, Tetradecyl, Pentadecyl, Hexadecyl, Heptadecyl, Nonadecyl oder Heneicosyl, sowie höhere Alkylreste der Triacontyl-, Tetracontyl-, Pentacontyl-, Hexacontyl-, Heptacontyl-, Octacontyl- oder Nonacontylserien.

Alkenyl ist u.a. Niederalkenyl, z.B. Allyl oder Methallyl, oder höheres Alkenyl, z.B. Decenyl.

Alkoxy ist insbesondere Niederalkoxy, z.B. Methoxy, Aethoxy, n-Propyloxy, Isopropyloxy oder n-Butyloxy.

Phenylniederalkoxy ist z.B. Benzyloxy oder 1- oder 2-Phenyläthoxy.

Alkanoyloxy ist u.a. Niederalkanoyloxy, z.B. Acetyloxy, Propionyloxy oder Butyryloxy, ferner höheres Alkamoyloxy, z.B. Lauroyloxy, Myristoyloxy, Palmitoyloxy, Stearoyloxy oder

Behenoyloxy.

Halogen steht z.B. für Fluor, Chlor oder Brom.

Alkanoylamino ist u.a. Niederalkanoylamino, z.B. Acetylamino oder Propionylamino, sowie höheres Alkanoylamino, z.B. Palmitoylamino.

Niederalkylthio ist z.B. Methylthio oder Aethylthio, während höheres Alkylthio z.B. Tetradecylthio, Hexadecylthio oder Octadecylthio bedeutet. Niederalkyl-substituiertes Amino, das im Niederalkylteil gegebenenfalls substituiert sein kann, ist u.a. Niederalkylamino, z.B. Methylamino oder Aethylamino, Carboxyniederalkylamino, z.B. Carboxymethylamino oder Carbamoylniederalkylamino.

Niederalkoxycarbonyl ist z.B. Methoxycarbonyl oder Aethoxycarbonyl.

Salzbildende Gruppen in den Verbindungen der Formeln I und II sind insbesondere gegebenenfalls vorhandene Carboxylgruppen. Verbindungen der Formel I mit solchen Gruppen können deshalb in Form von Salzen, insbesondere pharmazeutisch verwendbaren Salzen, wie Metall-, insbesondere Alkali- oder Erdalkalimetall-, z.B. Natrium-, Kalium-, Calcium- oder Magnesiumsalzen, oder von Ammoniumsalzen, z.B. mit Ammoniak oder organischen Aminen, wie Niederalkylamin, z.B. Triäthylamin, vorliegen. Verbindungen der Formeln I und II mit einer basischen Gruppe, z.B. einer Aminogruppe, können Säureadditionssalze bilden.

**Stand der Technik**

Die Verbindungen der Formel I der vorliegenden Anmeldung unterscheiden sich in erster Linie durch die Definition des Restes $R_8$ vom Stand der Technik.

In der EP-A 0003833 sind in generischer Form Muramylpeptide vorbeschrieben, in denen der Rest $R_8$ gemäss Formel I der vorliegenden Anmeldung unter anderem freies, verestertes oder veräthertes Hydroxyniederalkyl, freies, verestertes oder veräthertes Mercaptoniederalkyl oder freies oder acyliertes Aminoniederalkyl bedeuten kann. In der EP-A 0004512 sind in generischer Form Muramylpeptide vorbeschrieben, in denen der dem Rest $R_8$ entsprechende Rest unter anderem freies Hydroxy-, Mercaptooder Aminoniederalkyl bedeutet. Ausserdem sind in dieser Anmeldung Einzelverbindungen genannt, in denen $R_8$ für Hydroxymethyl steht. In der nach dem Prioritätstag der vorliegenden Anmeldung veröffentlichten EP-A 0014984 sind Muramylpeptide generisch beschrieben, in denen der dem Rest $R_8$ entsprechende Rest u.a. freies Hydroxyniederalkyl oder Benzyloxyniederalkyl bedeutet. Auch sind dort Einzelverbindungen genannt, worin der Rest $R_8$ Hydroxymethyl oder Hydroxyethyl bedeutet.

In der nach dem Prioritätstag der vorliegenden Anmeldung publizierten EP-A 0015468 sind Muramylpeptide generisch beschrieben, in denen der dem Rest $R_8$ entsprechende Rest u.a. freies Hydroxyniederalkyl bedeutet.

In der nach dem Prioritätsdatum der vorliegenden Anmeldung publizierten EP-A 0014159 sind Pyranosederivate beschrieben, die sich auch von anderen Zuckern als von D-Glucose ableiten, und worin der dem Rest $R_8$ entsprechende Rest auch Hydroxy- oder Mercaptomethyl bedeuten kann.

Von M. Kiso et al.ist in Carbohydrate Research 79, C17-C19 (1980) eine Verbindung mit Adjuvansaktivität beschrieben, worin der dem Rest R von Formel I der vorliegenden Anmeldung entsprechende Rest für Hydroxy steht.

Jedoch sind keine Einzelverbindungen vorbeschrieben, die unter die Ansprüche der vorliegenden Anmeldung fallen.

Die Erfindung betrifft in erster Linie Peptid-Derivate von Glucosaminverbindungen, insbesondere der Formel (Ic)

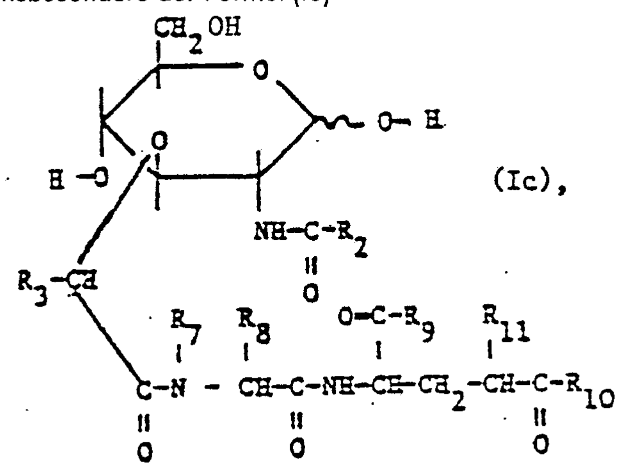

(Ic),

worin $R_2$ gegebenanfalls substituiertes Niederalkyl, Phenyl, Niederalkoxy oder Phenylniederalkoxy darstellt, $R_3$ für Wasserstoff oder Niederalkyl steht, $R_7$ Wasserstoff oder Niederalkyl bedeutet und $R_8$ einen Niederalkyl- oder Phenylniederalkylrest darstellt, der auch mit $R_7$ verbunden sein kann und der eine Oxycarbonyl-, Mercaptocarbonyl- oder Aminocarbonylgruppe trägt, welche mit einem gegebenenfalls substituierten, langkettigen aliphatischen oder cycloaliphatisch-aliphatischen Kohlenwasserstoffrest verbunden ist, welch letzterer durch Oxycarbonyl, Mercaptocarbonyl und/oder Iminocarbonyl unterbrochen sein kann, $R_9$ und $R_{10}$ für gegebenenfalls veräthertes Hydroxy oder gegebenenfalls substituiertes Amino stehen, und $R_{11}$ Wasserstoff oder einen Rest der Formel -C(=O)-$R_{12}$(Ia) bedeutet, worin $R_{12}$ für gegebenenfalls veräthertes Hydroxy oder gegebenenfalls substituiertes Amino steht, und Salze von Verbindungen der Formel Ic mit mindestens einer salzbildenden Gruppe.

Insbesondere betrifft die Erfindung die vorstehend genannten Verbindungen der Formel Ic, worin $R_2$ Niederalkyl mit bis zu 3 Kohlenstoffatomen, $R_3$ Wasserstoff oder Methyl, $R_7$ Wasserstoff $R_8$ Niederalkyl mit 1-4 C-Atomen, das als Substituenten eine OxycarbonyL-Mercaptocarbonyl- oder Aminocarbonylgruppe

trägt, welche über das Heteroatom mit dem Niederalkylrest verbunden ist und die ihrerseits mit Alkyl mit mehr als 10 und bis zu 50 Kohlenstoffatomen verbunden ist, das auch durch Iminocarbonyl unterbrochen sein kann, $R_9$ Amino, Hydroxy oder Niederalkoxy mit 1-3 C Atomen, $R_{10}$ Hydroxy, Niederalkoxy mit 1-3 C-Atomen oder Amino und $R_{11}$ Wasserstoff oder Carboxy bedeuten, und pharmazeutisch verwendbare Salze von solchen Verbindungen mit salzbildenden Gruppen, hauptsächlich solche Verbindungen, worin $R_8$ Alkanoyloxymethyl oder Alkanoyloxyäthyl mit mehr als 10 und bis zu 30 C-Atomen im Alkylteil des Alkanoylrestes, $R_9$ Amino und $R_{10}$ Hydroxy bedeuten, und ihre pharmazeutisch verwendbaren Salze.

Vor allem betrifft die Erfindung die Verbindungen der Formel Ic, worin $R_2$ für gegebenenfalls durch Hydroxy, Niederalkoxy, Niederalkanoyloxy oder Halogen substituiertes Niederalkyl oder Niederalkoxy, oder für gegebenenfalls durch Niederalkyl, Hydroxy, Niederalkoxy, Niederalkanoyloxy oder Halogen substituiertes Phenyl oder Phenylniederalkoxy steht, $R_3$ Wasserstoff oder Niederalkyl bedeutet, $R_7$ Wasserstoff oder Niederalkyl bedeutet, und $R_8$ einen Niederalkyl- oder Phenylniederalkylrest darstellt, der als Substituenten eine Oxycarbonyl-, Mercaptocarbonyl- oder Aminocarbonylgruppe trägt, welche ihrerseits wiederum mit einem gegebenenfalls durch Hydroxy, Niederalkoxy, Niederalkanoyloxy, Oxo oder Halogen substituierten Alkyl- oder Alkenylrest $R_o$ mit mehr als 6 und bis zu 90 Kohlenstoffatomen oder einem cycloaliphatisch substituierten Alkyloder Alkenylrest $R_o$ mit z.B. bis zu 30 Kohlenstoffatomen verbunden ist, und worin die Alkyl-, Alkenyl- und cycloaliphatisch substituierten Alkyl- bzw. Alkenylreste auch durch eine oder zwei Oxycarbonyl- oder Iminocarbonylgruppen unterbrochen sein können, $R_{11}$ Wasserstoff oder den Rest der Formel Ia bedeutet, und jeder der Reste $R_9$, $R_{10}$ und $R_{12}$ Hydroxy, Niederalkoxy, Amino oder Niederalkylamino, das gegebenenfalls durch Carboxy, Niederalkoxycarbonyl oder Carbamoyl substituiert ist, darstellen, wobei in solchen Verbindungen z.B. der Rest der Hydroxyessigsäure mit der Gruppierung der Formel -CH($R_3$)-, worin $R_3$ Niederalkyl darstellt, in der D-Form, der Rest der Aminoessigsäure mit der Gruppierung der Formel -CH($R_8$)-, in der L-Form, und der Rest der terminalen α-Aminoglutarsäureverbindung in der D-Form vorliegen, sowie Salze, insbesondere pharmazeutisch verwendbare Salze, von solchen Verbindungen mit salzbildenden Gruppen.

Vornehmlich betrifft die Erfindung Verbindungen der Formel Ic, worin $R_2$ Niederalkyl mit bis zu 4 Kohlenstoffatomen oder Phenyl darstellt, $R_3$ Wasserstoff oder Niederalkyl mit bis zu 4 Kohlenstoffatomen, insbesondere Methyl bedeutet, $R_7$ Wasserstoff darstellt und $R_8$ einen Niederalkylrest mit 1-4 Kohlenstoffatomen oder einen Phenylniederalkylrest darstellt, der als Substituenten eine Oxycarbonyl-, Mercaptocarbonyl- oder Aminocarbonylgruppe trägt, welche ihrerseits mit einem gegebenenfalls durch Hydroxy, Niederalkoxy, Oxo oder Halogen substituierten Alkyl- oder Alkenylrest $R_o$ mit mehr als 10 und bis zu 50 Kohlenstoffatomen oder einen gegebenenfalls durch Hydroxy, Niederalkoxy, Oxo oder Halogen substituierten tetracyclischen Cycloalkylalkylrest oder Cycloalkenylalkylrest $R_o$ mit mehr als 20 und bis zu 50 Kohlenstoffatomen verbunden ist, wobei solche Reste auch durch 1 oder 2 Oxycarbonyl-oder Iminocarbonylgruppen unterbrochen sein können, $R_9$ für Amino oder gegebenenfalls Carboxy oder Carbamoyl enthaltendes Niederalkylamino steht, $R_{10}$ Hydroxy bedeutet und $R_{11}$ Wasserstoff darstellt, wobei in solchen Verbindungen z.B. der Rest der Hydroxyessigsäure mit der Gruppierung der Formel -CH($R_3$)-, worin $R_3$ Niederalkyl darstellt, in der D-Form, der Rest der Aminoessigsäure mit der Gruppierung der Formel -CH($R_8$)- in der L-Form und der Rest der terminalen α-Aminoglutarsäureverbindung in der D-Form vorliegen, und Salze, insbesondere pharmazeutisch verwendbare Salze von solchen Verbindungen mit salzbildenden Gruppen.

Besonders zu erwähnen sind die Verbindungen der Formel Ic, worin $R_2$ Niederalkyl mit bis zu 4 Kohlenstoffatomen, insbesondere Methyl, oder Phenyl bedeutet, $R_3$ insbesondere Wasserstoff, ferner Niederalkyl mit bis zu 4 Kohlenstoffatomen, in erster Linie Methyl bedeutet, $R_7$ Wasserstoff darstellt, $R_8$ Niederalkyl mit 1-4 Kohlenstoffatomen oder Benzyl darstellt, die als Substituenten eine Oxycarbonyl-, Mercaptocarbonyl- oder Aminocarbonylgruppe tragen, welche vorzugsweise über das Heteroatom mit dem Niederalkyl- bzw. Benzylrest verbunden ist, und die ihrerseits mit einem gegebenenfalls durch Hydroxy, Niederalkoxy oder Oxo substituierten Alkyl oder Alkenyl mit mehr als 10 und bis zu 50 Kohlenstoffatomen oder einem gegebenenfalls durch Hydroxy oder Niederalkoxy substituierten tetracyclischen Cycloalkyl-alkyl- oder Cycloalkenylalkyl-rest mit mehr als 20 und bis zu 50 Kohlenstoffatomen verbunden ist, welche Reste auch durch eine Oxycarbonyl- oder Iminocarbonylgruppe unterbrochen sein können, $R_9$ Amino bedeutet, $R_{10}$ für Hydroxy steht, und $R_{11}$ Wasserstoff darstellt, wobei in solchen Verbindungen der Rest der Hydroxyessigsäure mit der Gruppierung der Formel -CH($R_3$)-, worin $R_3$ Niederalkyl darstellt, in der D-Form, der Rest der Aminoessigsäure mit der Gruppierung der Formel -CH($R_8$)- in der L-Form und der Rest der terminalen α-Amino-glutarsäureverbindung in der D-Form vorliegen, und Salze, insbesondere pharmazeutisch verwendbare Salze von solchen Verbindungen mit salzbildenden Gruppen.

Hervorzuheben sind die Verbindungen der Formel Ic, worin $R_2$ Niederalkyl mit bis zu 3 Kohlenstoffatomen oder Phenyl, $R_3$ Wasserstoff oder Methyl, $R_7$ Wasserstoff, $R_8$ Niederalkyl mit 1-3 Kohlenstoffatomen, das als Substituenten eine Oxycarbonyl-, Mercaptocarbonyl-oder

Aminocarbonylgruppe trägt, welche über das Heteroatom mit dem Niederalkylrest verbunden ist und die ihrerseits mit einem gegebenenfalls durch Hydroxy, Niederalkoxy oder Oxo substituierten Alkyl oder Alkenyl mit mehr als 10 und bis zu 50 Kohlenstoffatomen oder einem gegebenenfalls durch Hydroxy oder Niederalkoxy substituierten tetracyclischen Cycloalkyl-alkyl oder Cycloalkenyl-alkyl-rest mit mehr als 20 und bis zu 50 Kohlenstoffatomen verbunden ist, welche Reste auch durch Oxycarbonyl- oder Iminocarbonyl unterbrochen sein können, $R_9$ Amino, Niederalkylamino oder Carbamoylniederalkylamino oder Hydroxy, $R_{10}$ Hydroxy, Niederalkoxy, Amino oder Carbamoylniederalkylamino, und $R_{11}$ Wasserstoff bedeuten, wobei in solchen Verbindungen der Rest der Hydroxyessigsäure mit der Gruppierung -CH($R_3$)-, worin $R_3$ Methyl darstellt, in der D-Form, der Rest der Aminosäure mit der Gruppierung -CH($R_8$)-in der L-Form, und der Rest der terminalen α-Amino-glutarsäureverbindung in der D-Form vorliegen, und pharmszeutisch verwendbare Salze von solchen Verbindungen mit salzbildenden Gruppen.

Die Erfindung betrifft in allererster Linie die in den Beispielen beschriebenen Verbindungen und N-Acetyl-muramyl-L(O-behenoyl-N-methyl)-seryl-D-isoglutamin.

Die Erfindung betrifft auch die als Ausgangsstoffe zur Herstellung der Pyranosederivate der Formel I dienenden bzw. als Zwischenprodukte anfallenden Peptide der Formel II,

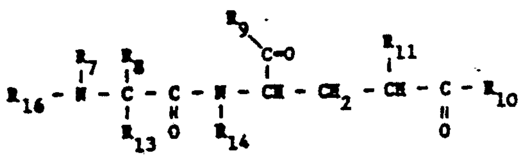 (II)

worin $R_{16}$ Wasserstoff oder eine Aminoschutzgruppe oder der Rest $R_{16}$

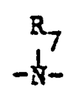

eine aktivierte Aminogruppe bedeuten und die übrigen Substituenten die obengenannten Bedeutungen haben, die Salze solcher Peptide mit mindestens einer salzbildenden Gruppe, Verfahren zu ihrer Herstellung und ihre Verwendung als Zwischenprodukte zur Herstellung der Verbindungen der Formel I.

Aminoschutzgruppen sind in erster Linie Acylreste, insbesondere solche von Kohlensäurehalbderivaten, wie -halbestern, z.B. gegebenenfalls durch Halogen, substituiertes Niederalkoxycarbonyl, wie tert.-Butyloxycarbonyl oder 2,2,2-Trichloräthoxycarbonyl, oder gegebenenfalls substituiertes 1-Phenylniederalkoxycarbonyl, wie Benzyloxycarbonyl.

Eine Aminogruppe, z.B. in einer Verbindung der Formel II, wird beispielsweise durch Reaktion mit einem Phosphit aktiviert.

Die neuen Verbindungen der Formel I können in an sich bekannter Weise hergestellt werden. Sie werden z.B. hergestellt, indem man in einer Verbindung der Formel I, worin funktionelle Gruppen gegebenenfalls in geschützter Form vorliegen, mindestens eine leicht durch Wasserstoff ersetzbare Gruppe, insbesondere eine Hydroxy-, Amino-, Carboxy- oder Mercaptoschutzgruppe, vor allem in den Resten R, $R_1$, $R_4$, $R_6$, $R_9$, $R_{11}$ oder $R_{10}$, abspaltet, oder indem man mindestens eine funktionelle Gruppe in die den Endstoffen entsprechenden Derivate überführt, beispielsweise freies Hydroxy oder Amino acyliert oder freies Carboxy verestert oder amidiert.

Schutzgruppen der genannten Art, sowie ihre Abspaltung sind beispielsweise beschrieben in "Protective Groups in Organic Chemistry", Plenum Press, London, New York, 1973, ferner in "The Peptides", Vol. I, Schröder and Lübke, Academic Press, London, New York, 1965, sowie in "Methoden der organischen Chemie", Houben-Weyl, 4. Auflage, Bd. 15/1, Georg Thieme Verlag, Stuttgart, 1974.

So sind Carboxylgruppen, wie die Carboxylgruppe

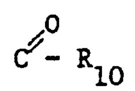 ,

üblicherweise in veresterter Form geschützt, wobei solche Estergruppierungen unter schonenden Bedingungen leicht spaltbar sind. In dieser Art geschützte Garboxylgruppen enthalten als veresternde Gruppen in erster Linie in 1-Stellung verzweigte oder in 1- oder 2-Stellung geeignet substituierte Niederalkylgruppen. Bevorzugte, in veresterter Form vorliegende Carboxylgruppen sind u.a. tert.-Niederalkoxycarbonyl, z.B. tert.-Butyloxycarbonyl, Arylmethoxycarbonyl, mit einem oder zwei Arylresten, wobei diese gegebenenfalls, z.B. durch Niederalkyl, wie tert.-Niederalkyl, z.B. tert.-Butyl, Niederalkoxy, wie Methoxy, Hydroxy, Halogen, z.B. Chlor, und/oder Nitro, mono- oder polysubstituierte Phenylreste darstellen, wie gegebenenfalls, z.B. wie oben erwähnt, substituiertes Benzyloxycarbonyl, z.B. 4-Nitrobenzyloxycarbonyl oder 4-Methoxybenzyloxycarbonyl, oder gegebenenfalls, z.B. wie oben erwähnt, substituiertes Diphenylmethoxycarbonyl, z.B. Diphenylmethoxycarbonyl oder Di-(4-methoxyphenyl)methoxycarbonyl, 1-Niederalkoxyniederalkoxycarbonyl, wie Methoxymethoxycarbonyl, 1-Methoxyäthoxycarbonyl oder 1-Aethoxymethoxycarbonyl, 1-Niederalkylthioniederalkoxycarbonyl, wie 1-Methylthiomethoxycarbonyl oder 1-Aethylthioäthoxycarbonyl, Aroylmethoxycarbonyl, worin die Aroylgruppe gegebenenfalls, z.B. durch Halogen, wie Brom, substituiertes Benzoyl darstellt, z.B. Phenacyloxycarbonyl, 2-Halogenniederalkoxycarbonyl, z.B. 2,2,2-Trichloräthoxycarbonyl, 2-Bronäthoxycarbonyl oder 2-Jodäthoxycarbonyl, oder 2-(trisubstituiertes Silyl)-äthoxycarbonyl, worin die Substituenten unabhängig voneinander je einen gegebenenfalls substituierten, z.B. durch Niederalkyl, Niederalkoxy, Aryl, Halogen und/oder Nitro substituierten, aliphatischen, araliphatischen, cycloaliphitischen oder aromitischen Kohlenwasserstoffrest mit z.B. bis zu 15 Kohlenstoffatomen, wie entsprechendes, gegebenenfalls substituiertes Niederalkyl, Phenylniederalkyl, Cycloalkyl oder Phenyl bedeuten, z.B. 2-Triniederalkylsilyläthoxycarbonyl, wie 2-Trimethylsilyläthoxycarbonyl oder 2-(Di-n-butylmethyl-silyl)-äthoxycarbonyl, oder 2-Triarylsilyläthoxycarbonyl, wie 2-Triphenylsilyläthoxycarbonyl.

Weitere, in veresterter Form vorliegende geschützte Carboxylgruppen sind entsprechende Silyloxycarbonyl-, insbesondere organische Silyloxycarbonylgruppen, ferner entsprechende Stannyloxycarbonylgruppen. In diesen enthält das Silicium- bzw. Zinnatom vorzugsweise Niederalkyl, insbesondere Methyl, ferner Niederalkoxy, z.B. Methoxy, und/oder Halogen, z.B. Chlor, als Substituenten. Geeignete Silyl- bzw. Stannylschutzgruppen sind in erster Linie Triniederalkylsilyl, insbesondere Trimethylsilyl, ferner Dimethyl-tert.butylsilyl, Niederalkoxyniederalkyl-halogen-silyl, z.B. Methoxy-methyl-chlor-silyl, oder Diniederalkylhalogen-silyl, z.B. Dimethyl-chlor-silyl, oder entsprechend substituierte Stannylverbindungen, z.B. Tri-n-butylstannyl.

Bevorzugte geschützte Carboxylgruppen sind tert.-Niederalkoxycarbonyl, wie tert.-Butyloxycarbonyl, und in erster Linie gegebenenfalls, z.B. wie oben erwähnt, substituiertes Benzyloxycarbonyl, wie 4-Nitrobenzyloxycarbonyl, oder Diphenylmethoxycarbonyl.

Eine geschützte Aminogruppe kann z.B. in Form einer leicht spaltbaren Acylamino-, Arylmethylamino-, verätherten Mercaptoamino-, 2-Acyl-niederalk-1-en-1-yl-amino-, Silylamino- oder Stannylaminogruppe oder als Azidogruppe vorliegen.

In einer entsprechenden Acylaminogruppe ist Acyl beispielsweise der Acylrest einer organischen Carbonsäure mit z.B. bis zu 18 Kohlenstoffatomen insbesondere einer gegebenenfalls, z.B. durch Halogen oder Aryl, substituierten Alkancarbonsäure oder gegebenenfalls, z.B. durch Halogen, Niederalkoxy oder Nitro, substituierten Benzoesäure, oder eines Kohlensäurehalbesters. Solche Acylgruppen sind beispielsweise Niederalkanoyl, wie Formyl, Acetyl, oder Propionyl, Halogen-niederalkanoyl, wie 2-Halogen-acetyl, insbesondere 2-Chlor-, 2-Brom-, 2-Jod-, 2,2,2-Trifluor- oder 2,2,2-Trichloracetyl, gegebenenfalls, z.B. durch Halogen, Niederalkoxy oder Nitro substituiertes Benzoyl, z.B. Benzoyl, 4-Chlorbenzoyl, 4-Methoxybenzoyl oder 4-Nitrobenzoyl, oder in 1-Stellung des Niederalkylrestes verzweigtes oder in 1- oder 2-Stellung geeignet substituiertes Niederalkoxycarbonyl, insbesondere tert.-Niederalkoxycarbonyl, z.B. tert.-Butyloxycarbonyl, Arylmethoxycarbonyl mit einem oder zwei Arylresten, die vorzugsweise gegebenenfalls, z.B. durch Niederalkyl, insbesondere tert.-Niederalkyl, wie tert.-Butyl, Niederalkoxy, wie Methoxy, Hydroxy, Halogen, z.B. Chlor, und/oder Nitro, mono- oder polysubstituiertes Phenyl darstellen, wie gegebenenfalls substituiertes Benzyloxycarbonyl, z.B. 4-Nitrobenzyloxycarbonyl, oder substituiertes Diphenylmethoxycarbonyl, z.B. Benzhydryloxycarbonyl, oder Di-(4-methoxyphenyl)methoxycarbonyl, Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl, 2-Halogenniederalkoxycarbonyl, z.B. 2,2,2-Trichloräthoxycarbonyl, 2-Bromäthoxycarbonyl oder 2-Jodäthoxy-carbonyl, oder 2-(trisubstituiertes Silyl)-äthoxycarbonyl, worin die Substituenten unabhängig voneinander je einen gegebenenfalls substituierten, z.B. durch Niederalkyl, Niederalkoxy, Aryl, Halogen oder Nitro, substituierten, aliphatischen, araliphatischen, cycloaliphatischen oder aromatischen Kohlenwasserstoffrest mit z.B. bis zu 15 C-Atomen, wie entsprechendes, gegebenenfalls substituiertes Niederalkyl, Phenylniederalkyl, Cycloalkyl oder Phenyl, bedeuten, z.B. 2-Triniederalkylsilyl-äthoxycarbonyl, wie 2-Trimethylsilyläthoxycarbonyl oder 2-(Di-n-butylmethyl-silyl)-äthoxycarbonyl, oder 2-Triarylsilyläthoxycarbonyl, wie 2-Triphenylsilyläthoxycarbonyl.

Weitere, als Aminoschutzgruppen in Frage kommende Acylreste sind auch entsprechende Reste organischer Fhosphor-, Phosphonoder Fhosphinsäuren, wie Diniederalkylphosphoryl, z.B. Dimethylphosphoryl, Diäthylphosphoryl, Di-n-propylphosphoryl oder Diisopropylphosphoryl, Dicycloalkylphosphoryl, z.B. Dicyclohexylphosphoryl, gegebenenfalls substituiertes Diphenylphosphoryl, z.B. Diphenylphosphoryl, gegebenenfalls, z.B. durch Nitro substituiertes Di-(phenylniederalkyl)-phosphoryl, z.B. Dibenzylphosphoryl oder Di-4-nitrobenzylphosphoryl, gegebenenfalls substituiertes Phenyloxy-phenyl-phosphonyl, z.B. Phenyloxy-phenyl-phosphonyl, Diniederalkylphosphinyl, z.B. Diäthylphosphinyl, oder gegebenenfalls substituiertes Diphenylphosphinyl, z.B. Diphenylphosphinyl.

In einer Arylmethylaminogruppe, die eine Mono-, Di- oder insbesondere Triarylmethylamino darstellt, sind die Arylreste insbesondere gegebenenfalls substituierte Phenylreste. Solche Gruppen sind beispielsweise Benzyl-, Diphenylmethyl- und insbesondere Tritylamino.

Eine verätherte Mercaptogruppe in einer mit einem solchen Rest ges-chützten Aminogruppe ist in erster Linie Arylthio oder Arylniederalkylthio, worin Aryl insbesondere gegebenenfalls, z.B. durch Niederalkyl, wie Methyl oder tert.-Butyl, Niederalkoxy, wie Methoxy, Halogen, wie Chlor, und/oder Nitro substituiertes Phenyl ist. Eine entsprechende Aminoschutzgruppe ist z.B. 4-Nitrophenylthio.

In einem als Aminoschutzgruppe verwendbaren 2-Acyl-niederalk-1-en-1-yl-rest ist Acyl z.B. der entsprechende Rest einer Niederalkancarbonsäure, einer gegebenenfalls, z.B. durch Niederalkyl, wie Methyl oder tert.-Butyl, Niederalkoxy, wie Methoxy, Halogen, wie Chlor, und/ oder Nitro substituierten Benzoesäure, oder insbesondere eines Kohlensäurehalbesters, wie eines Kohlensäureniederalkylhalbesters. Entsprechende Schutzgruppen sind in erster Linie 1-Niederalkanoyl-prop-1-en-2-yl, z.B. 1-Acetyl-prop-1-en-2-yl, oder 1-Niederalkoxycarbonyl-prop-1-en-2-yl, z.B. 1-Aethoxycarbonyl-prop-1-en-2-yl.

Eine Silyl- oder Stannylaminogruppe ist in erster Linie eine organische Silyl- bzw. Stannylaminogruppe, worin das Silicium- bzw. Zinnatom vorzugsweise Niederalkyl, insbesondere Methyl, ferner Niederalkoxy, z.B. Methoxy, und/oder Halogen, z.B. Chlor, als Substituenten enthält. Entsprechende Silyl- oder Stannylgruppen sind in erster Linie Triniederalkylsilyl, insbesondere Trimethylsilyl, ferner Dimethyl-tert.-butyl-silyl, Niederalkoxyniederalkyl-halogen-silyl, z.B. Methoxy-methyl-chlor-silyl, oder Diniederalkyl-halogensilyl, z.B. Dimethyl-chlor-silyl, oder entsprechend substituiertes Stannyl, z.B. Tri-n-butylstannyl.

Bevorzugte Aminoschutzgruppen sind Acylreste von Kohlensäurehalbestern, insbesondere tert.-Butyloxycarbonyl, gegebenenfalls, z.B. wie angegeben, substituiertes Benzyloxycarbonyl, z.B. 4-Nitrobenzyloxycarbonyl, oder Diphenylmethoxycarbonyl, oder 2-Halogenniederalkoxycarbonyl, wie 2,2,2-Trichloräthoxycarbonyl, ferner Trityl oder Formyl.

Eine Mercaptogruppe kann in analoger Weise wie eine Hydroxygruppe, z.B. wie oben für diese angegeben, geschützt werden.

Die Abspaltung der durch Wasserstoff ersetzbaren Gruppen wird unter milden, meist schwach sauren, mitunter auch neutralen oder schwach basischen Solvolysebedingungen hydrogenolytisch oder photolytisch durchgeführt. Silylgruppen können auch mit Fluorid-Ionen oder solche abgebende Verbindungen abgespalten werden.

Gewisse verätherte Hydroxygruppen, wie tert.-Butyloxy oder Tetrahydro-2-pyranyloxy, können solvolytisch, insbesondere durch Behandeln mit einem sauren Reagens, tert.-Butyloxy z.B. durch Behandeln mit einer starken organischen Garbonsäure, wie Trifluoressigsäure, und Tetrahydro-2-pyranyl-oxy z.B. durch Behandeln mit einer Mineralsäure, wie Chlorwasserstoff, gespalten werden. Andere, wie gegebenenfalls substituiertes 1-Phenyl-niederalkoxy, z.B. Benzyloxy oder Diphenylmethoxy, können hydrogenolytisch, z.B. durch Behandeln mit katalytisch aktiviertem Wasserstoff, wie Wasserstoff in Gegenwart eines Edelmetall-, z.B. Palladium-Katalysators, in die freie Hydroxygruppe überführt werden. Acyloxygruppen lassen sich solvolytisch (z.B. tert.Butyloxycarbonyl oder Diphenylmethoxycarbonyl durch Behandeln mit einer starken organischen Carbonsäure, wie Trifluoressigsäure), reduktiv (z.B. 2,2,2-Trichloräthoxycarbonyl- oder 2-Jodäthoxy-carbonyl, üblicherweise nach Bildung aus dem entsprechenden 2-Bromäthoxy-carbonyl-Derivat, durch Behandeln mit einem reduzierenden Metall, wie Zink, in Gegenwart von 90%iger wässriger Essigsäure), oder hydrogenolytisch (z.B. Benzyloxycarbonyl oder Diphenylmethoxycarbonyl durch Behandeln mit katalytisch aktiviertem Wasserstoff, wie Wasserstoff in Gegenwart eines Edelmetall-, z.B. Palladium oder Platinkatalysators spalten. In analoger Weise geschützte Mercaptogruppen lassen sich in entsprechender Weise in die freien Mercaptogruppen überführen. Als geschützte Aminogruppen verwendete Acylaminogruppen können in an sich bekannter Weise, insbesondere solvolytisch oder reduktiv, tert.-Butyloxycarbonylamino z.B. mittels Acidolyse (Behandeln mit Trifluoressigsäure), 2,2,2-Trichloräthoxycarbonylamino z.B. mittels metallischer Reduktion (Behandeln mit Zink in Gegenwart von Essigsäure), und Benzyloxycarbonylamino z.B. mittels Hydrogenolyse (Behandeln mit Wasserstoff in Gegenwart eines Hydrierkatalysators), in die freien Aminogruppen übergeführt werden. Geschützte Carboxylgruppen können je nach Art der Schutzgruppe, die üblicherweise eine veresternde Gruppe ist, in verschiedenartiger Weise freigesetzt werden, tert.-Butyloxycarbonyl oder Diphenylmethoxycarbonyl u.a. solvolytisch, z.B. durch Behandeln der entsprechenden Esterverbindung mit einer starken organischen Carbonsäure, wie Trifluoressigsäure, 2,2,2-Trichloräthoxycarbonyl oder 2-Jodäthoxycarbonyl (üblicherweise vor der Spaltung z.B. durch Behandeln der entsprechenden Esterverbindung mit einem Metalljodid, wie Natriumjodid, aus der entsprechenden 2-Bromäthoxycarbonylgruppe gebildet) reduktiv, z.B. durch Behandeln der Esterverbindung mit einem reduzierenden Metall, wie Zink in Gegenwart von 90%iger wässriger Essigsäure, und 1-Phenyl-niederalkoxycarbonyl, z.B. Benzyloxycarbonyl oder Diphenylmethoxycarbonyl, hydrogenolytisch, z.B. durch Behandeln der Esterverbindung mit

katalytisch aktiviertem Wasserstoff, wie Wasserstoff in Gegenwart eines Edelmetall-, wie Palladium- oder Platinkatalysators.

Verfahrensgemäss erhältliche Verbindungen der Formel I können in andere Verbindungen dieser Formel übergeführt werden. So kann man z.B. eine freie Hydroxygruppe in an sich bekannter Weise, u.a. wie weiter unten beschrieben, wie durch Behandeln mit einer, den Acylrest entsprechenden organischen Carbonsäure oder vorzugsweise einem geeigneten Derivat davon, wie mit einem Anhydrid, inkl. einem gemischten Anhydrid, oder einem aktivierten Ester davon, in eine Acyloxygruppe überführen.

Verbindungen der Formel I mit salzbildenden Gruppen können in an sich bekannter Weise in Salze überführt werden, z.B. eine entsprechende Verbindung mit einer Garboxylgruppe durch Behandeln mit einem geeigneten basischen Mittel, wie einem Alkalimetall- oder Erdalkalimetallhydroxid oder -carbonat, oder einem geeigneten Alkalimetallalkanoat.

Verfahrensgemäss erhältliche Isomerengemische von Verbindungen der Formel I können in an sich bekannter Weise in die einzelnen Isomeren aufgetrennt werden, z.B. ein entsprechendes Racemat einer Verbindung der Formel I mit einer Carboxylgruppe durch Bildung eines Salzes mit einer optisch aktiven Base, Trennung des Gemisches der diastereoisomeren Salze und Umwandeln des abgetrennten Salzes in die freie Säure.

Die neuen Verbindungen der Formel I können auch dadurch hergestellt werden, dass man ih einer Verbindung der Formel I, worin funktionelle Gruppen gegebenenfalls in geschützter Form vorhanden sind und worin $R_8$ einen Niederalkyl- oder Phenylniederalkylrest darstellt, der auch mit $R_7$ verbunden sein kann und der eine Hydroxy-, Mercapto-, Amino- oder Carboxygruppe trägt, die gegebenenfalls in derivatisierter Form vorliegen, und worin die übrigen Substituenten die obengenannte Bedeutung haben, gegebenenfalls stufenweise, über die Oxycarbonyl-, Mercaptocarbonylund/oder Iminocarbonyl-Gruppe den Rest $R_0$ einführt, und, wenn notwendig, in einer erhaltenen Verbindung geschützte funktionelle Gruppen in die freien funktionellen Gruppen überführt, und, wenn erwünscht, eine verfahrensgemäss erhältliche Verbindung der Formel I in eine andere Verbindung der Formel I überführt, und/oder, wenn erwünscht, ein verfahrensgemäss erhältliches Salz einer Verbindung der Formel I mit einer salzbildenden Gruppe in die entsprechende freie Verbindung der Formel I oder in ein anderes Salz umwandelt, und/oder, wenn erwünscht, eine verfahrensgemäss erhältliche Verbindung der Formel I mit einer salzbildenden Gruppe in ein Salz überführt, und/oder, wenn erwünscht, ein verfahrensgemäss erhältliches Isomerengemisch von Verbindungen der Formel I in die isomeren Verbindungen der Formel I auftrennt.

Die verfahrensmässige Einführung des Restes $R_0$ in solche Verbindungen kann in einem Schritt erfolgen, d.h. der Rest kann als ganzes, oder aber, falls darin weitere Oxycarbonyl-, Mercaptocarbonyl- oder Iminocarbonylgruppen vorhanden sind, stufenweise erfolgen, wobei man zuerst die erste funktionelle Bindung und dann die übrigen in an sich bekannter Weise herstellt.

Dabei handelt es sich entweder um eine Acylierungsreaktion, indem eine $R_0$-CO-gruppe in eine gegebenenfalls funktionell derivatisierte Hydroxy-, Mercapto- oder Aminoverbindung eingeführt wird, oder um die Veresterung bzw. Amidierung einer Garbonsäure oder einem reaktionsfähigen Derivat davon mit einer Verbindung der Formeln $R_0$-OH, $R_0$-SH oder $R^o$-$NH_2$ oder einem reaktiven funktionellen Derivat davon.

Funktionelle Gruppen im Ausgangsmaterial, die gegebenenfalls in geschützter Form vorliegen, sind in erster Linie freie Hydroxy-, Mercapto- oder Aminogruppen oder freie Carboxygruppen. Als Schutzgruppen dieser funktionellen Gruppen kommen insbesondere die in der Peptid- und/oder Zuckerchemie üblicherweise verwendeten, leicht abspaltbaren Hydroxy-, Mercapto-, Amino- und/oder Carboxy-Schutzgruppen in Frage, wobei in üblicher Weise, z.B. solvolytisch oder reduktiv, abspaltbare Schutzgruppen gewählt werden, die vorzugsweise unter neutralen oder sauren, gegebenenfalls unter schwach basischen Bedingungen, abspaltbar sind. Hydroxyschutzgruppen sind insbesondere eine Hydroxygruppe veräthernde Schutzgruppen, wie geeignetes, gegebenenfalls substituiertes Niederalkyl oder Phenylniederalkyl, insbesondere in 1-Stellung polyverzweigtes Niederalkyl, z.B. tert.-Butyl, oder gegebenenfalls substituiertes 1-Fhenyl-niederalkyl, z.B. Bemzyl, Diphenylmethyl oder Trityl, fermer die zusammen mit dem Sauerstoffatom der Hydroxygruppe eine Acetal- oder Halbacetalgruppierung bildenden, veräthernden Gruppen, insbesondere 2-Oxacycloalkyl, wie 2-Tetrahydrofuranyl oder 2-Tetrahydropyranyl, ferner über ein, dem Oxasauerstoffatom benachbarten, vorzugsweise nicht endständigen, Kohlenstoffatom gebundenes Oxaniederalkyl, z.B. 3-Oxa-2-n-pentan, sowie gegebenenfalls substituierte Alkylidenreste, die benachbarte Sauerstoffatome, wie die in 4- und 6-Stellumg,verbinden. Alkylidenreste sind insbesondere Niederalkyliden, in erster Linie Methyliden, Isopropyliden oder Propyliden oder auch ein gegebenenfalls substituierter Benzylidenrest. Weitere Hydroxyschutzgruppen sind veresternde Schutzgruppen, wie geeignetes, gegebenenfalls substituiertes Niederalkoxycarbonyl, insbesondere in α-Stellung zur Oxygruppe polyverzweigtes oder in β-Stellung zur Oxygruppe Halogen-enthaltendes Niederalkoxycarbonyl, z.B. tert.-Butyloxycarbonyl, 2,2,2,-Trichloräthoxycarbonyl, oder 2-Brom- oder 2-Jodäthoxycarbonyl, oder geeignetes, gegebenenfalls substituiertes

Phenylniederalkoxycarbonyl, wie 1-Phenylniederalkoxycarbonyl, z.B. Benzyloxycarbonyl oder Diphenylmethoxycarbonyl. Eine Mercaptogruppe kann in analoger Weise wie eine Hydroxygruppe geschützt sein.

Aminoschutzgruppen sind in erster Linie Acylreste, insbesondere solche von Kohlensäurehalbderivaten, wie -halbestern, z.B. gegebenenfalls durch Halogen, substituiertes Niederalkoxycarbonyl, wie tert.-Butyloxycarbonyl oder 2,2,2-Trichloräthoxycarbonyl, oder gegebenenfalls substituiertes 1-Phenylniederalkoxycarbonyl, wie Benzyloxycarbonyl.

Eine Carboxygruppe ist vorzugsweise in Form einer veresterten Carboxygruppe geschützt; als Schutzgruppen für Carboxylgruppen kommen u.a. geeignetes, gegebenenfalls substituiertes Niederalkyl, insbesondere in α-Stellung polyverzweigtes oder in β-Stellung Halogen-enthaltendes Niederalkyl, z.B. tert.-Butyl, 2,2,2-Trichloräthyl, oder 2-Brom-oder 2-Jodäthyl, oder geeignetes, gegebenenfalls substituiertes Phenylniederalkyl insbesondere 1-Phenylniederalkyl, wie Benzyl oder Diphenylmethyl, in Frage.

Ausgangsstoffe mit derivatisierter Hydroxyl-, Mercapto- oder Aminogruppe im Rest $R_8$ sind entsprechende Derivate, die anstelle der freien Hydroxy-, Mercapto- bzw. Amino-Verbindungen in der Acylierungsresktion eingesetzt werden können. Es handelt sich dabei z.B. um O-, S- oder N-Silyl-Derivate, wie O-, S- oder N-Organosilyl-, z.B. Triniederalkylsilyl-, insbesondere Trimethsylsilylderivate.

Weitere geeignete Derivate der Ausgsngsstoffe sind solche, in welchen die Hydroxy- bzw. Mercaptogruppe als reaktionsfähige veresterte Hydroxybzw. Mercaptogruppe vorliegt; geeignete reaktionsfähige veresterte Hydroxygruppen sind mit anorganischen Säuren veresterte Hydroxygruppen, wie Halogen-, z.B. Chlor-, Brom- oder Jodatome, oder mit starken organischen Säuren, wie entsprechenden Sulfonsäuren veresterte Hydroxygruppen, z.B. Niederalkylsulfonyloxy, insbesondere Methylsulfonyloxy, oder Arylsulfonyloxy, insbesondere p-Methylphenylsulfonyloxy.

Die Acylierung der gegebenenfalls derivatisierten Hydroxy-, Mercaptobzw. Aminogruppe im Rest $R_8$ kann in an sich bekannter Weise unter Verwendung von, den Rest $R_0$-CO einführenden Mitteln, erfolgen. Diese sind in erster Linie die entsprechenden Säuren oder vorzugsweise deren reaktionsfähige Derivate, wobei in diesen Mitteln gegebenenfalls vorhandene funktionelle Gruppen, wenn notwendig oder erwünscht, in geschützter Form vorliegen können.

Schutzgruppen von gegebenenfalls in solchen Säuren der Derivaten vorhandenen Hydroxy- oder Mercaptogruppen sind z.B. die oben erwähnten veräthernden oder veresternden Reste, während Aminogruppen z.B. durch die in der Peptidchemie üblicherweise verwendeten Schutzgruppen, insbesondere durch leicht, unter neutralen oder sauren, sowie unter schwach basischen Bedingungen abspaltbare Schutzgruppen, wie durch entsprechende Acylreste, insbesondere durch solche von monoveresterter Kohlensäure, oder in Salzform geschützt sein können.

Reaktionsfähige Derivate dieser Säuren sind in erster Linie reaktionsfähige aktivierte Ester oder reaktionsfähige Amhydride, ferner reaktionsfähige cyclische Amide dieser Säuren sowie deren Salze, wobei in solchen Säuren gegebenenfalls vorhandene funktionelle Gruppen, z.B. wie angegeben, geschützt sein können. Dabei können reaktionsfähige Derivate von solchen Säuren auch in situ gebildet werden.

Aktivierte Ester von Säuren der Formel $R_0$-COOH sind insbesondere am Verknüpfungskohlenstoffatom des veresternden Restes ungesättigte Ester, z.B. vom Vinylester-Typ, wie eigentliche Vinylester (die man z.B. durch Umesterung eines entsprechenden Esters mit Vinylacetat erhalten kann; Methode des aktivierten Vinylesters), Carbamoylvinylester (die man z.B. durch Behandeln der entsprechenden Säure mit einem Isoxazoliumreagens erhalten kamn; 1,2-Oxazolium- oder Woodward-Methode), oder 1-Niederalkoxy-vinylester (die man z.B. durch Behandeln der entsprechenden Säure mit einem Niederalkoxyacetylen erhalten kann; Aethoxyacetylen-Methode), oder Ester vom Amidinotyp, wie N,N'-disubstituierte Amidinoester (die man z.B. durch Behandeln der entsprechenden Saure, die bei Verwendung eines Säureadditionssalzes, z.B. des Hydrochlorids, auch in Form eines Salzes, wie eines Ammonium-, z.B. Benzyltrimethylammoniumsalzes, eingesetzt werden kann, mit einem geeigneten N,N'-disubstituierten Carbodiimid, z.B. N,N'-Dicyclohexyl-carbodiimid, erhalten kann; Carbodiimid-Methode), oder N,N-disubstituierte Amidinoester (die man z.B. durch Behandeln der entsprechenden Säure mit einem N,N-disubstituierten Cyanamid erhalten kann; Cyanamid-Methode), geeignete Arylester, insbesondere durch Elektronen-anziehende Substituenten geeignet substituierte Phenylester (die man z.B. durch Behandeln der entsprechenden Säure mit einem geeignet substituierten Phenol, z.B. 4-Nitrophenol, 4-Methylsulfonyl-phenol, 2,4,5-Trichlorphenol, 2,3,4,5,6-Pentachlor-phenol oder 4-Phenyldiazophenol, in Gegenwart eines Kondensationsmittels, wie N,N'-Dicyclohexyl-carbodiimid, erhalten kann; Methode der aktivierten Arylester), Cyanmethylester (die man z.B. durch Behandeln der entsprechenden Säure mit Chloracetonitril in Gegenwart einer Base erhalten kann; Cyanmethylester-Methode), Thioester, insbesondere gegebenenfalls, z.B. durch Nitro, substituierte Phenylthioester (die man z.B. durch Behandeln der entsprechenden Säure mit gegebenenfalls, z.B. durch Nitro, substituierten Thiophenolen, u.a. mit Hilfe der

Anhydrid- oder Carbodiimid-Methode, erhalten kann; Methode der aktivierten Thiolester), oder Amino- oder Amidoester (die man z.B. durch Behandeln der entsprechenden Säure mit einer N-Hydroxyamino- bzw. N-Hydroxy-amido-Verbindung, z.B. N-Hydroxy-piperidin, N-Hydroxy-succinimid, N-Hydroxy-phthalimid oder 1-Hydroxy-benztriazol, z.B. nach der Anhydrid- oder Carbodiimid-Methode, erhalten kann; Methode der aktivierten N-Hydroxyester).

Anhydride von Säuren der Formel $R_0$-COOH können symmetrische und vorzugsweise gemischte Amhydride dieser Säuren sein, so z.B. Anhydride mit anorganischen Säuren, wie Säurehalogenide, insbesondere Säurechloride (die man z.B. durch Behandeln der entsprechenden Säure mit Thionylchlorid, Phosphorpentachlorid oder Oxalylchlorid erhalten kann; Säurechlorid-Methode), Azide (die man z.B. aus einem entsprechenden Säureester über das entsprechende Hydrazid und dessen Behandlung mit salpetriger Säure erhalten kann; Azidmethode), Anhydride mit Kohlensäurehalbderivaten, wie mit entsprechenden Estern, z.B. Kohlensäureniederalkylhalbester (die man z.B. durch Behandeln der entsprechenden Säure mit Halogen-, wie Chlorameisensäureniederalkylestern oder mit einem 1-Niederalkoxycarbonyl-2-niederalkoxy-1,2-dihydrc-chinolin, z.B. 1-Niederalkoxycarbonyl-2-äthoxy-1,2-dihydrochinolin, erhalten kann; Methode der gemischten 0-Alkyl-kohlensäureanhydride), oder Anhydride mit dihalogenierter, insbesondere dichlorierter Phosphorsäure (die man z.B. durch Behandeln der entsprechenden Säure mit Phosphoroxychlorid erhalten kann; Phosphoroxychlorid-Methode), oder Anhydride mit organischen Säuren, wie gemischte Anhydride mit organischen Carbonsäuren (die man z.B. durch Behandeln der entsprechenden Säure mit einem gegebenenfalls substituierten Niederalkan- oder Phenylalkancarbonsäurehalogenid, z.B. Phenylessigsäure-, Pivalinsäure- oder Trifluoressigsäurechlorid erhalten kann; Methode der gemischten Carbonsäureanhydride) oder mit organischen Sulfonsäuren (die man z.B. durch Behandeln eines Salzes, wie eines Alkalimetallsalzes, der entsprechenden Säure, mit einem geeigneten organischen Sulfonsäurehalogenid, wie Niederalkanoder Aryl-, z.B. Methan- oder p-Toluolsulfonsäurechlorid, erhalten kann; Methode der gemischten Sulfonsäureanhydride), sowie symmetrische Anhydride (die man z.B. durch Kondensation der entsprechenden Säure in Gegenwart eines Carbodiimids oder von 1-Diäthylaminopropin erhalten kann; Methode der symmetrischen Anhydride).

Geeignete cyclische Amide sind insbesondere Amide mit fünfgliedrigen Diazacyclen aromatischen Charakters, wie Amide mit Imidazolen, z.B. Imidazol (die man z.B. durch Behandeln der entsprechenden Säure mit N,N'-Carbonyldiimidazol; Imidazolid-Methode), oder Pyrazolen, z.B. 3,5-Dimethyl-pyrazol (die man z.B. über das Säurehydrazid durch Behandeln mit Acetylaceton erhalten kann; Pyrazolid-Methode).

Salze von Säuren der Formel $R_0$-COOH sind insbesondere Metallsalze, wie Alkalimetall-, z.B. Natrium- oder Raliumsalze.

Man verwendet zur Acylierung der gegebenenfalls derivatisierten Hydroxygruppe insbesondere die aktivierten Ester, in erster Linie Amino- oder Amidoester, ferner auch die Amhydride, insbesondere die gemischten Anhydride. Ausgangsstoffe mit einer in reaktionsfähiger veresterter Form vorliegenden Hydroxygruppe werden üblicherweise mit einem Salz einer der Säuren der Formel $R_0$-COOH umgesetzt.

Wie erwähnt können Derivate der Säuren auch in situ gebildet werden. So kann man z.B. N,N'-disubstituierte Amidinoester in situ bilden, indem man das Gemisch des Ausgangsmaterials und der Säure der Formel $R_0$-COOH in Gegenwart des geeigneten N,N'-disubstituierten Carbodiimids, z.B. N,N'-Dicyclohexylcarbodiimid, zur Reaktion bringt. Ferner kann man Amino- oder Amidoester in Gegenwart des zu acylierenden Ausgangsmaterials bilden, indem man das Gemisch der gewünschten Säure und des entsprechenden Ausgangsmaterials in Gegenwart eines N,N'-disubstituierten Carbodiimids, z.B. N,N'-Dicyclohexyl-carbodiimid, und eines N-Hydroxy-amins oder N-Hydroxy-amids, z.B. N-Hydroxy-succinimid, gegebenenfalls in Anwesenheit einer geeigneten Base, z.B. 4-Dimethylamino-pyridin, umsetzt.

Die Acylierungsreaktionen können in an sich bekannter Weise durchgeführt werden, wobei die Reaktionsbedingungen in erster Linie von der Art des verwendeten Acylierungsmittels abhängen, üblicherweise in Gegenwart eines geeigneten Lösungs- oder Verdünnungsmittels oder eines Gemisches von solchen, und, falls notwendig, in Gegenwart eines Kondensationsmittels, das z.B. bei Verwendung eines Anhydrids als Acylierungsmittel gegebenenfalls auch ein Säurebindendes Mittel sein kann, unter Kühlen oder Erwärmen, z.B. in einem Temperaturbereich von etwa −30°C bis etwa +150°C, in einem geschlossenen Reaktionsgefäss und/oder in der Atmosphäre eines Inertgases, z.B. Stickstoff.

Die im obigen Verfahren verwendeten Ausgangsstoffe sind bekannt oder können in an sich bekannter Weise, die Acylierungsmittel z.B. wie angegeben, hergestellt werden.

Die Verbindungen der vorliegenden Erfindung können ebenfalls hergestellt werden, indem man in einer Verbindung der Formel IV,

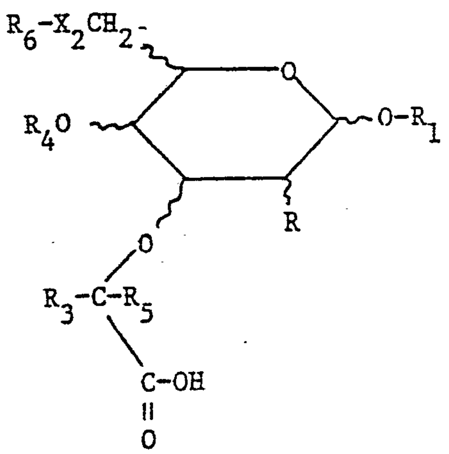

(IV)

worin die Substituenten die obengenannte Bedeutung haben, wobei freie funktionelle Gruppen gegebenenfalls in geschützter Form vorhanden sind, und worin die Carboxygruppe in derivatisierter Form vorliegen kann, die Carboxygruppe, gegebenenfalls stufenweise, mit einem den Rest der Formel

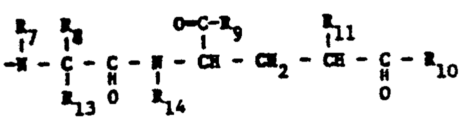

(IVa),

worin die Substituenten die obengenannte Bedeutung haben, wobei funktionelle Gruppen gegebenenfalls in geschützter Form vorhanden sind, übertragenden Mittel amidiert, und, wenn notwendig, in einer erhaltenen Verbindung geschützte funktionelle Gruppen in die freien funktionellen Gruppen überführt, und, wenn erwünscht, die zusätzlichen Verfahrensschritte durchführt.

Funktionelle Gruppen im Ausgangsmaterial der Formel IV bzw. im Rest der Formel IVa, die gegebenenfalls in geschützter Form vorliegen, sind in erster Linie die oben erwähnten, die in der beschriebenen Weise in geschützter Form vorliegen können.

Die verfahrensgemässe Amidierung der gegebenenfalls derivatisierten Carboxygruppe in einem Ausgangsmaterial der Formel IV kann in einem Schritt oder in mehreren Schritten erfolgen; d.h. die Gruppe der Formel IVa kann als ganze oder aber bruchstückweise eingeführt werden, z.B. unter Amidierung der gegebenenfalls derivatisierten Carboxygruppe im Ausgangsmaterial der Formel IV mit einem die Gruppe der Formel

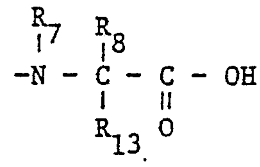

(IVb)

worin die Carboxygruppe in funktionell abgewandelter Form vorhanden sein kann, und gegebenenfalls vorhandene funktionelle Gruppen in geschützter Form vorliegen können, abgebenden Mittel und nachträglicher Amidierung der Carboxygruppe in einem Zwischenprodukt der Formel

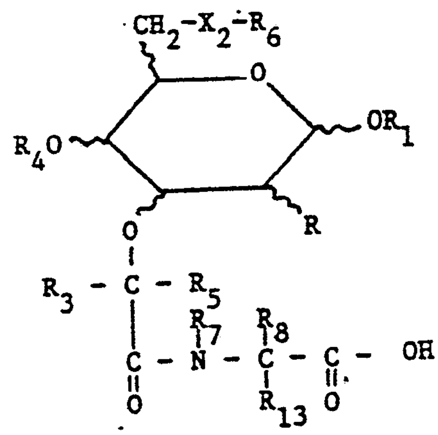

(V),

worin die Carboxygruppe in reaktionsfähiger derivatisierter Form vorhanden sein kann, und gegebenenfalls vorhandene funktionelle Gruppen in geschützter Form vorliegen können, mit einem den Rest der Formel

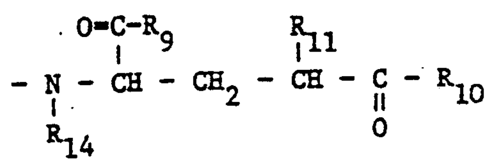

(Va),

worin gegebenenfalls vorhandene funktionelle Gruppen in geschützter Form vorliegen können, abgebenden Mittel.

Die Amidierung der gegebenenfalls derivatisierten Carboxygruppe in einem Ausgangsmaterial der Formel IV oder in einem Zwischenprodukt bzw. Ausgangsmaterial der Formel V kann in an sich bekannter Weise durchgeführt werden, wobei man üblicherweise von Verbindungen der Formel IV bzw. V ausgeht, in welchen die Carboxylgruppe in reaktionsfähiger derivatisierter Form vorliegt. Als amidierende Mittel verwendet man die den obigen Resten der Formeln IVa, IVb bzw. Va entsprechenden Dipeptide der Formel

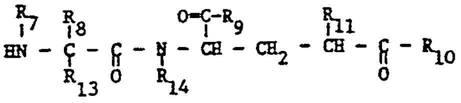

(IVaa)

bzw. Aminosäuren der Formeln

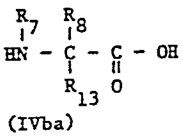

(IVba)

bzw.

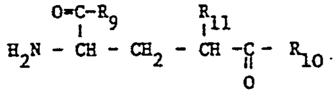

(Vaa)

worin vorhandene funktionelle Gruppen gegebenenfalls in geschützter Form vorliegen, und die an der Amidierungsreaktion teilnehmende Aminogruppe gegebenenfalls in reaktionsfähiger derivatisierter Form vorhanden sein kann.

Schutzgruppen von, in solchen Dipeptiden und Aminosäuren gegebenenfalls vorhandenen funktionellen Gruppen, wie den oben erwähnten Hydroxygruppen, sind vorzugsweise mittels veräthernden und veresternden Schutzgruppen, Aminogruppen vorzugsweise mittels acylierenden Schutzgruppen, geschützt, während Carboxygruppen üblicherweise in veresterter Form geschützt sind. Geeignete veresternde Schutzgruppen für Carboxyl sind leicht, vorzugsweise unter milden, vorzugsweise neutralen oder sauren, gegebenenfalls schwach basischen Bedingungen solvolytisch oder reduktiv abspaltbare Gruppen, wie am Verknüpfungskohlenstoff verzweigtes und/oder Aryl oder Aroyl, wie gegebenenfalls, z.B. durch Halogen, wie Chlor oder Brom, Niederalkoxy, z.B. Methoxy, und/oder Nitro, substituiertes Phenyl, Biphenylyl oder Benzoyl, aufweisendes Niederalkyl, z.B. tert.-Butyl, Benzyl, Diphenylmethyl, 2-Biphenylyl-2-propyl oder Phenacyl, oder am, dem Verknüpfungskohlenstoffatom benachbarten, Kohlenstoffatom geeignet substituiertes, wie Halogen, z.B. Chlor, Brom oder Jod, oder eine organische Silylgruppe, z.B. Trimethylsilyl, enthaltendes Niederalkyl, wie 2-Halogenniederalkyl, z.B. 2,2,2-Trichloräthyl, 2-Bromäthyl oder 2-Jodäthyl, oder 2-Trimethylsilyläthyl.

Reaktionsfähige, in derivatisierter Form vorliegende Aminogruppen in den Dipeptiden der Formel IVaa bzw. in den Aminosäuren der Formeln IVba und Vaa, sind in erster Linie silylierte Aminogruppen, die z.B. Trimethylsilyl als derivatisierenden Rest enthalten.

Reaktionsfähige Derivate der Ausgangsstoffe der Formel IV oder der Zwischenprodukte bzw. der Ausgangsstoffe der Formel V sind in erster Linie reaktionsfähige aktivierte Ester oder reaktionsfähige Anhydride, ferner reaktionsfähige cyclische Amide der Säuren der Formeln IV bzw. V, wobei solche reaktionsfähigen Derivate auch in situ gebildet werden können. Geeignete aktivierte Ester sind z.B. die obgenannten Ester, wie die am Verknüpfungskohlenstoffatom des veresternden Restes ungesättigten Ester, z.B. vom Vinylestertyp, wie eigentliche Vinylester, Carbamoylvinylester, oder 1-Niederalkoxy-vinylester, Ester vom Amidinotyp, wie N,N'- oder N,N-disubstituierte Amidinoester, Arylester, insbesondere durch Elektronen-anziehende Substituenten geeignet substituierte Phenylester, Cyanmethylester, Thioester, insbesondere gegebenenfalls, z.B. durch Nitro, substituierte Phenylthi-oester, oder Amino- oder Amidoester. Reaktionsfähige Anhydride der Säuren der Formeln IV und V können symmetrische und vorzugsweise gemischte Anhydride dieser Säuren sein, z.B. die obgenannten Anhydride mit anorganischen Säuren, wie die entsprechenden Säurehalogenide, insbesondere Chloride, Azide, Anhydride mit Kohlensäurehalbderivaten, wie entsprechenden Estern, z.B. Kohlensäureniederalkylhalbestern, oder Anhydride mit dihalogenierter, wie dichlorierter Phosphorsäure, oder Anhydride mit organischen Säuren, wie gemischte Anhydride mit organischen Carbon- oder Sulfonsäuren, oder symmetrische Anhydride. Geeignete cyclische Amide sind z.B. die oben beschriebenen und stellen insbesondere Amide mit fünfgliedrigen Diazacyclen aromatischen Charakters, wie Amide mit Imidazolen oder Pyrazolen, dar.

Wie erwähnt, können Derivate der Säuren der Formeln IV und V auch in situ gebildet werden. So kann mman z.B. N,N'-disubstituierte Amidinoester in situ bilden, indem man das Gemisch einer Säure der Formel IV bzw. V und eines Dipeptides der Formel IVaa oder einer Aminosäure der Formel IVba bzw. einer Aminosäure der Formel Vaa in Gegenwart des N,N'-disubstituierten Carbodiimids, z.B. N,N'-Dicyclohexylcarbodiimid, zur Reaktion bringt. Ferner kann man Amino- oder Amidoester einer Säure der Formeln IV bzw. V in Gegenwart eines Dipeptids der Formel IVaa oder einer Aminosäure der Formel IVa bzw. Vaa bilden, indem man das Gemisch der gewünschten Säure und der entsprechenden Dipeptid- bzw. der entsprechenden Aminosäure-Verbindung in Gegenwart eines N,N'-di-substituierten Carbodiimids, z.B. N,N'-Dicyclohexylcarbodiimid, und eines N-Hydroxy-amins oder N-Hydroxy-amids, z.B. N-Hydroxy-succimimid, gegebenenfalls in Anwesenheit einer geeigneten Base z.B. 4-Dimethylamino-pyridin, umsetzt.

Die Amidierungsreaktionen können in an sich bekannter Weise durchgeführt werden, wobei die Reaktionsbedingungen in erster Linie von der Art des verwendeten Derivats der Säure der Formel IV oder V abhängen, üblicherweise in Gegemwart eines geeigneten Lösungs- oder Verdünnungsmittels oder eines Gemisches von solchen, und, falls notwendig, in Gegenwart eines Kondensationsmittels, das z.B. bei Verwendung eines Anhydrids als Derivat einer Säure der Formel IV oder V gegebenenfalls auch ein Säurebindendes Mittel sein kann, unter Kühlen oder

Erwärmen, z.B. in einem Temperaturbereich von etwa -30°C bis etwa +150°C, im einem geschlossenen Reaktionsgefäss und/oder in der Atmosphäre eines Inertgases, z.B. Stickstoff.

Die im obigen Verfahren verwendeten Ausgangsstoffe können in an sich bekannter Weise hergestellt werden, z.B. durch Acylierung der freien Hydroxy- oder Mercaptogruppe im Rest $R_8$, analog der oben beschriebenen Weise.

Die Verbindungen der vorliegenden Erfindung können ebenfalls hergestellt werden, indem man eine Verbindung der Formel

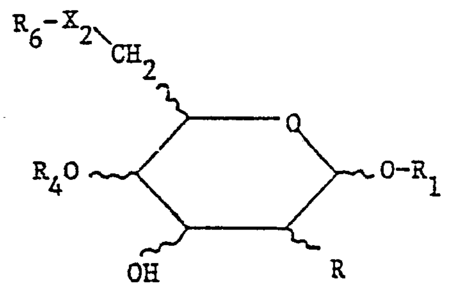

(VII),

worin die Substituenten die obengenannte Bedeutung haben, wobei funktionelle Gruppen vorzugsweise in geschützter Form vorliegen, oder ein reaktionsfähiges Derivat davon mit einer Verbindung der Formel

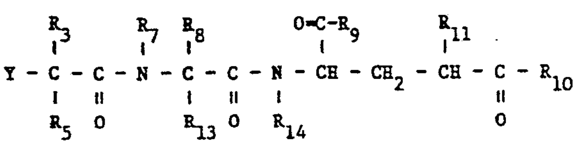

(VIII),

worin Y eine reaktionsfähige veresterte Hydroxygruppe darstellt, und die übrigen Substituenten die obengenannte Bedeutung haben, wobei funktionelle Gruppen vorzugsweise in geschützter Form vorliegen, umsetzt, und, wenn notwendig in einer erhaltenen Verbindung geschützte funktionelle Gruppen in die freien funktionellen Gruppen überführt, und, wenn erwünscht, die zusätzlichen Verfahrensschritte gegebenenfalls durchführt.

Funktionelle Gruppen in den Ausgangsstoffen der Formeln VII und VIII, die gegebenenfalls in geschützter Form vorliegen, sind in erster Linie die oben erwähnten, die in der beschriebenen Weise in geschützter Form vorliegen können.

Ein reaktionsfähiges Derivat des als Ausgangsmaterial verwendeten sekundären Alkohols der Formel VII ist in erster Linie eine entsprechende Metall-, insbesondere Alkalimetall-, z.B. Lithium-, Natrium- oder Kaliumverbindung.

Eine reaktionsfähige veresterte Hydroxygruppe Y ist eine mit einer starken Säure, wie einer entsprechenden anorganischen Säure, z.B. einer Halogenwasserstoffsäure, oder einer entsprechenden organischen Säure, wie organischen Sulfonsäure,veresterte Hydroxygruppe. Die Gruppe Y bedeutet dem nach in erster Linie Halogen, insbesondere Brom, ferner Chlor oder Jod.

Die obige Reaktion, in welcher das Ausgangsmaterial der Formel VII vorzugsweise in Form eines Derivates, wie einer Alkalimetallverbindung eingesetzt wird, kann in an sich bekannter Weise, z.B. in Gegenwart eines Lösungs- oder Verdünnungsmittels, wie Dimethylformsmid, und/oder eines Kondensationsmittels, und, falls erwünscht oder notwendig, unter Kühlen oder Erwärmen, z.B. in einem Temperaturbereich von etwa −30° bis etwa +150°C, in einem geschlossenen Reaktionsgefäss und/oder in der Atmosphäre eines Inertgases, z.B. Stickstoff, durchgeführt werden.

Die Ausgangsstoffe sind bekannt oder können in an sich bekannter Weise hergestellt werden, z.B., wie oben beschrieben, durch Acylierung einer freien Hydroxy-, Amino- oder Mercaptogruppe im Rest $R_8$ mit einer Säure

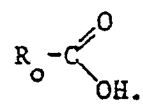

Die erfindungsgemässen Zwischenprodukte der Formel II werden in analoger Weise wie vorstehend beschrieben hergestellt, z.B., wenn erwünscht, durch Abspaltung von leicht durch Wasserstoff ersetzbaren Gruppen, vor allem in den Resten $R_{16}$, $R_9$, $R_{11}$ oder $R_{10}$ oder, wenn erwünscht, durchDerivatisierung freier funktioneller Gruppen oder durch Verknüpfen der Aminosäuren der Formeln (IVba) und (Vaa).

Ferner ist es auch möglich, die neuen Verbindun8en der Formel I, sofern R für den Rest der Formel

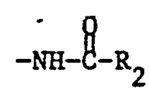

steht, worin $R_2$ gegebenenfalls substituiertes Niederalkyl oder Phenyl ist, $R_1$, $R_4$ und $R_6$ Wasserstoff und $X_2$ Sauerstoff bedeuten, und sie sich von der D-Glucose ableiten, zu erhalten, wenn in einer Verbindung der Formel

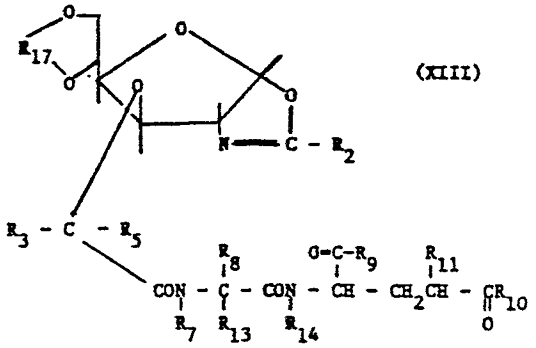

(XIII)

worin $R_{17}$ eine Alkyliden- oder Cycloalkylidengruppe ist und die übrigen Substituenten die obengenannte Bedeutung haben, den Oxazolin- und den Dioxolanring sauer aufspaltet, und gegebenenfalls vorhandene Schutzgruppen abspaltet.

Alkyliden ist darin insbesondere Niederalkyliden, wie Isopropyliden, und Cycloalkyliden in erster Linie Cyclopentyliden oder Cyclohexyliden.

Diese Spaltung erfolgt ebenfalls in an sich bekannter Weise, z.B. mit einem sauren Ionenaustauscher, insbesondere mit einem solchen mit Sulfonsäuregruppen, wie Amberlite IR-120 (ein Styrolharz mit stark sauren Sulfogruppen) oder Dowex 50 (Polystyrolsulfonsäure), oder einer starken anorganischen oder organischen Säure, wie Salzsäure, Bromwasserstoffsäure, Schwefelsäure oder einer Sulfonsäure, z.B. Methansulfonsäure, oder einer gegebenenfalls im aromatischen Ring substituierten Phenylsulfonsäure, wie p-Toluolsulfonsäure oder Trifluoressigsäure. Arbeitet man dabei in Gegenwart von Wasser, erhält man in 1-Stellung eine freie Hydroxygruppe. Ist auch eine der Carboxylgruppen $COR_9$ bzw. $COR_{10}$ und/oder $R_{11}$ mit einem Alkohol, insbesondere einem Niederalkanol verestert, lässt sie sich, insbesondere bei höherer Temperatur, mit wässriger Säure verseifen.

In den erhaltenen Verbindungen lassen sich Schutzgruppen am Peptidrest nachträglich, z.B. durch Hydrogenolyse, wie z.B. mit katalytisch angeregtem Wasserstoff oder Hydrolyse, abspalten.

Die dabei verwendeten Ausgangsstoffe lassen sich z.B. erhalten, wenn man in ein entsprechendes Oxazolin, mit einer freien Hydroxygruppe in 3-Stellung des Zuckerrestes den $R_3(R_5)$-Acetamidopeptidrest in einer oder mehreren Stufen einführt.

Die Peptide der Formel II werden erhalten, indem man

a) eine Aminosäure der Formel

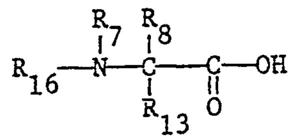

,

worin $R_{16}$ eine Aminoschutzgruppe bedeutet, während die übrigen Substituenten die obengenannte Bedeutung haben, und die Carboxylgruppe gegebenenfalls in aktivierter Form vorliegt, wobei andere funktionelle Gruppen gegebenenfalls in geschützter Form vorliegen, mit einer Aminosäure der Formel

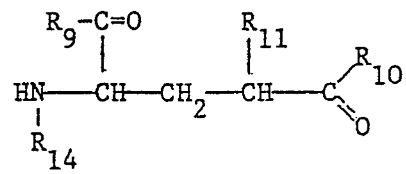

,

worin die

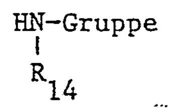

gegebenenfalls in aktivierter Form vorliegt, und die übrigen Substituenten die obengenannte Bedeutung haben, wobei darin vorhandene funktionelle Gruppen gegebenenfalls in geschützter Form vorliegen, umsetzt, und, wenn erwünscht, mindestens eine gegebenenfalls vorhandene Schutzgruppe abspaltet, und/oder, wenn erwünscht, eine erhaltene Verbindung mit mindestens einer salzbildenden Gruppe in ihr Salz überführt, oder

b) in einer Verbindung der Formel II, worin funktionelle Gruppen gegebenenfalls in geschützter Form vorliegen und worin $R_8$ einen Niederalkyloder Phenylniederalkylrest darstellt, der auch mit $R_7$ verbunden sein kann und der eine gegebenenfalls in reaktionsfähiger Form vorliegende Hydroxy-, Mercapto-, Amino- oder Carboxygruppe trägt, gegebenenfalls stufenweise, über die Oxycarbonyl-, Mercaptocarbonyl- oder Iminocarbonylgruppe den Rest $R_0$ einführt, und, wenn erwünscht, mindestens eine gegebenenfalls vorhandene Schutzgruppe abspaltet, und/oder, wenn erwünscht, eine erhaltene Verbindung mit mindestens einer salzbildenden Gruppe in ein Salz überführt, oder

c) in einer Verbindung der Formel II, worin funktionelle Gruppen gegebenenfalls in geschützter Form vorliegen, mindestens eine leicht durch Wasserstoff ersetzbare Gruppe abspaltet, oder mindestens eine funktionelle Gruppe in die den Endstoffen entsprechenden Derivate überführt.

Die neuen, ungeschützten lipophilen Pyranosederivate der Formel I, vor allem die

Muramyl- und Normuramylpeptide, und ihre Salze weisen eine Reihe wertvoller pharmakologischer Eigenschaften, insbesondere eine ausgeprägte immunpotenzierende Wirkung, auf.

So wird durch diese Verbindungen in vivo die Antikörperbildungsfähigkeit von Mäusen erheblich gesteigert:

NMRI-Mäusen werden durch intraperitoneale Injektion von 10 µg präzipitatfreiem BSA am Tag 0 immunisiert. 9, 15 und 29 Tage später werden Serumproben entnommmen und auf ihren Gehalt an anti-BSA-Antikörpern mit einer passiven Haemagglutinationstechnik untersucht. In der verwendeten Dosis ist lösliches BSA für die Empfängertiere subimmunogen, d.h. es vermag keine oder nur eine ganz geringfügige Produktion von Antikörpern auszulösen. Zusätzliche Behandlung der Mäuse mit gewissen immunpotenzierenden Stoffen vor oder nach der Antigengabe führt zu einem Anstieg der Antikörpertiter im Serum. Der Effekt der Behandlung wird durch den erreichten Scorewert, d.h. durch die Summe der $log_2$ Titerdifferenzen an den drei Blutungstagen ausgedrückt.

In diesem Test sind die Verbindungen der Formel (I) in der Lage, bei intraperitonealer oder subkutaner AppliRation von 0,5-5 mg/kg Tier an fünf aufeinander folgenden Tagen nach Immmunisierung mit BSA die Antikörperproduktion gegen BSA signifikant zu steigern. Sie sind diesbezüglich den herkömmlichen hydrophilen Muramylpeptiden hoch überlegen.

Auch Manifestationen der zellvermittelten Immunität können durch die genannten Verbindungen in vivo potenziert werden:

Während Sensibilisierung von Meerschweinchen mit BSA in inkomplettem Freundschen Adjuvans nur zu humoraler Antikörperbildung führt, induziert die Beimischung der erfindungsgemässen lipophilen Muramylpeptide in einem Dosisbereich von 5-50 µg zur Antigen-Oelemulsion Spättyp-Ueberempfindlichkeit gegenüber BSA: 3 Wochen nach Immunisierung führt die intrakutane Injektion von BSA bei diesen Tieren zu einer lokalen Entzündungsreaktion mit Erythem und Verdickung der Haut, die innert 24 bis 48 Stunden ihr Maximum erreicht. Diese Spättyp-Reaktionen entsprechen quantitativ und qualitativ denjenigen, die üblicherweise durch Immunisierung mit BSA in komplettem Freund'schen Adjuvans (d.h. mit Zusatz von Mykobakterien) erhalten werden. Die $ED_{50}$-Werte (benötigte µg/Tier zur Induktion einer Differenz des Reaktionsvolumens (Erythemfläche x Hautdickenzunahme) bei behandelten und unbehandelten Tieren von 200µl, 24 Stunden nach Auslösung) betragen 10-20 µg.

Besonders hervorzuheben ist auch die Fähigkeit solcher Pyranosederivate, durch Applikation zusammen mit BSA in Liposomen (4:1-Gemisch von Eilecithin und Cholesterin oder Eilecithin allein; 4 mg/Tier) und ohne die toxi-sche Mineralöl-Komponente, in Meerschweinchen eine Spättypüberempfindlichkeit gegen BSA zu induzieren. Quantitativ und qualitativ sind diese Spättyp-Reaktionen ebenfalls identisch mit denjenigen, die durch Immunisierung mit BSA in komplettem Freund'schen Ajuvans erhalten werden. Die $ED_{50}$-Werte betragen 100-300 µg pro Tier.

Die neuen Verbindungen der Formel (I) zeigen, soweit sie keine Schutzgruppen enthalten, im Vergleich zu hydrophilen Muramyldipeptiden weitere zusätzliche Qualitätsverbesserungen:

Balb/c Mäuse werden durch intraperitoneale Injektion von $2x10^4$ P815 Mastocytomzellen am Tag 0 immunisiert. Am Tag 15 werden die Milzzellen der so immunisierten Tiere in vitro auf das Vorhandensein zytotoxischer, gegen P815 Mastocytom-Zellen gerichteter T-Lymphocyten untersucht. Dazu werden die P815 Zielzellen mit $^{51}Cr$ markiert und das Ausmass der zytotoxischen Reaktion durch-Messen der Radioaktivität im Kulturüberstand ermittelt. In der verwendeten Dosis sind die P815 Mastozytomzellen für die Empfänger-Mäuse subimmunogen, d.h. sie induzieren keine oder nur ganz geringe Bildung zytotoxischer T-Zellen. Gleichzeitige intraperitoneale Applikation von 1 bis 50 µg der genannten Muramylpeptide der Formel I führen zu einer signifikanten Steigerung der Bildung zytotoxischer T-Zellen (Faktor 10 bis 30 gegenüber unbehandelten Mäusen).

Die immunpotenzierenden Eigenschaften der ungeschützten Verbindungen der Formel (I) lassen sich auch im Falle der Induktion spezifischer Immuntoleranz gegen Transplantationsantigene durch Immunisierung mit adjuvierten Autoblasten bei der Maus darstellen:

In einer gemischten Lymphocytemkultur werden Milzlymphocyten des zukünftigen Transplantat-Empfängers (Cr7Bl/6J Mäuse) mit bestrahlten Milzzellen des zukünftigen Transplantat-Spenders (CBA/J Mäuse) inkubiert. T-Lymphocyten mit spezifischen Rezeptoren für die Histokompatibilitätsamtigene des Spenders proliferieren und werden zu Blasten; diese können durch Sedimentation von den andern Zellen abgetrennt werden. Die spezifischen Blasten exprimieren die relevanten idiotypischen Spezifitäten der Membranrezeptoren und werden adjuviert mit komplettem Freund'schem Adjuvans (CFA) als Autoimmunogene zur Induktion spezifischer Toleranz gegen die betreffenden Transplantationsantigene in die prospektiven Transplantat-Empfänger (C57 Bl/6J) injiziert. Die Immunisierung erfolgt viermal in vierwöchigen Abständen mit autologen anti- CBA/J T Lymphoblasten. Adsorbate von T-Autoblasten mit den neuen Verbindungen der Formel (I) ($10^9$ Blasten werden in einer Lösung von 20 mg Substanz in 20 ml PBS suspendiert. Nach zweistündiger Inkubation werden die Zellen zentrifugiert und zweimal mit PBS gewaschen) sind in der Lage, spezifische Immuntoleranz in Abwesenheit von CFA zu induzieren, wobei die

Adsorbate gleich wirksam sind wie Lymphoblasten in CFA.

Die ungeschützten Verbindungen der Formel (I) sind ausserdem in der Lage, in Konzentrationen von 0,5-100 µg/ml in Milzzellkulturen von normalen Mäusen die Bildung antikörperproduzierender Zellen zu induzieren (Vermehrung der 19S-plaquebildenden Zellen um einen Faktor 10 bis 30 über den Kontrollwert (in Abwesenheit der stimulierenden Substanzen): So werden in Anwesenheit der genannten Verbindungen z.B. spezifische Antikörper gegen Schaferythrocyten gebildet, ohne dass den Kulturen Schaferythrocyten zur Immunisierung zugesetzt werden. Andererseits vermögen die genannten Substanzen im selben Konzentrationsbereich auch die immunologische Reaktivität von T-zellverarmten Milzzellkulturen (von kongential athymischen nu/nu Mäusen) gegenüber einem normalerweise thymusabhängigen Antigen (Schaferythrocyten) zu steigern (Faktor 10 bis 30 gegenüber unbehandelten Kontrollkulturen). Durchdiegenannten Verbindungen werden aber in vitro direkt oder indirekt nicht nur Proliferations- und Syntheseleistungen von B-Lymphocyten (d.h. von potentiell antikörperbildenden Zellen) induziert, sondern auch Effekte auf T-Lymphocyten (zu denen regulatorisch aktive Helfer- und Suppressorzellen sowie cytotoxische Effektorzellen gehören), vermittelt. So vermögen z.B. die erwähnten Verbindungen in einem Konzentrationsbereich von 1-20 µg/ml die Reaktivität von cortisonresistenten Thymuszellen gegenüber allogenen bestrahlten Stimulatorlymphocyten erheblich (bis zu 10-fach) zu potenzieren.

Die oben erwähnten Wirkungen kommen wahrscheinlich indirekt dadurch zustande, dass solche lipophilen Verbindungen Makrophagen aktivieren, die ihrerseits die Reaktivität von T- und B-Lymphocyten fördern. Tatsächlich kann man zeigen, dass die genannten Verbindungen bereits in geringen Konzentrationen (0,5-10 µg/ml) grosse Mengen "colony stimulating activity" (CSA) aus Maus-Makrophagen freisetzen (Induktion von bis zu 150-200 Kolonien innert 7 Tagen aus $10^5$ Mäuse-Knochenmarkzellen, nach Zugabe von 20% Ueberstand aus während 24 Stunden mit Substanz inkubierten Makrophagenkulturen, im Vergleich zu 0-5 Kolonien bei Zugabe von Ueberständen unbehandelter Makrophagenkulturen). CSA ist ein biologischer Mediator, der für die Differenzierung von Knochenmark-Stammzellen zu Makrophagen und polymorphkernigen Leucocyten notwendig ist. Damit bewirken die genannten Verbindungen einen erhöhten Nachschub von Zellen, die für die unspezifische Resistenz und für die Induktion, Amplifikation und Expression spezifischer (lymphocytenvermittelter) Immunreaktionen von zentraler Bedeutung sind.

Die immunpotenzierende Wirkung der neuen Verbindungen kann in vivo nachgewiesen werden: So führt die Injektion eines erfindungsgemässen Muramylpeptidderivats der Formel I innert 3-9 Stunden zu einem hohen Anstieg der CSA-Konzentration im Serum (bis zu 120 Kolonien pro $10^5$ Mäuseknochenmarkzellen nach Zugabe von Chloroform extrahiertem Serum [5% Endkonzentration] im Vergleich zu 0-5 Kolonien bei unbehandelten Tieren). Dementsprechend wird durch Verabreichung derselben Verbindungen in vivo die Amtikörperbildungsfähigkeit von Mäusen erheblich potenziert.

Die immunpotenzierenden Eigenschaften der neuen Verbindungen der Formel I und ihrer Salze lassen sich auch in Tumormodellen demonstrieren, so z.B. beim Ehrlich Ascites Tumor in der Maus.

Die Verbindungen gemäss der vorliegenden Erfindung sind zudem wenig toxisch: Auch 5-malige intraperitoneale Applikation in einer Dosis von 100 mg/kg/Tag an fünf aufeinanderfolgenden Tagen wurde von Mäusen anscheinend symptomlos vertragen. Da die für die Immunstimulation benötigen Dosen sehr gering sind, ist die therapeutische Breite der neuen Verbindungen sehr gross.

Die neuen Verbindungen gemäss der vorliegenden Erfindung können somit die zelluläre und besonders die humorale Immunität erheblich steigern, und zwar sowohl in Mischung mit dem Antigen selber (Adjuvanseffekt im engeren Sinne) als auch bei zeitlich und örtlich von der Antigeninjektion getrennter Zufuhr (systemische Immunpotenzierung).

Die neuen Verbindungen gemäss der vorliegenden Erfindung können somit als Adjuvantien in Mischung mit Impfstoffen dazu benützt werden, den Impferfolg zu verbessern und den durch humorale Antikörper und/oder zelluläre Immunität vermittelten Infektionsschutz gegenüber bakteriellen, viralen oder parasitären Erregern zu verbessern.

Schliesslich eignen sich die beschriebenen Verbindungen in Mischung mit verschiedenen Antigenen als Adjuwantien bei der experimentellen und industriellen Herstellung von Antiseren für Therapie und Diagnostik und bei der Induktion von imm unologisch aktivierten Lymphocyten-populationen für Zelltransferverfahren.

Darüberhinaus können die neuen Verbindungen auch ohne gleichzeitige Antigenzufuhr dazu benützt werden, bereits unterschwellig ablaufende Immunreaktionen bei Mensch und Tier zu fördern. Die Verbindungen eignen sich demnach besonders für die Stimulation der körperlichen Abwehr, z.B. bei chronischen und akuten Infektionen oder bei selektiven (antigenspezifischen) immunologischen Defekten, sowie bei angeborenen, aber auch bei erworbenen allgemeinen (d.h. nicht antigenspezifischen) immunologischen Defektzuständen, wie sie im Alter, im Verlauf schwerer Primärerkrankungen und vor allem nach Therapie mit ionisierenden Strahlen oder mit immunosuppressiv wirkenden Hormonen auftreten. Die genannten Stoffe können somit

vorzugsweise auch in Kombination mit Antibiotika, Chemotherapeutika oder anderen Heilmitteln verabreicht werden Schliesslich sind die beschriebenen Stoffe auch zur allgemeinen Prophylaxe von Infektionskrankheiten bei Mensch und Tier geeignet.

Die geschützten Verbindungen der Formel I als auch die Verbindungen der Formel II sind Zwischenprodukte zur Herstellung der Verbindungen der Formel I. Darüberhinaus sind auch die Verbindungen der Formel II pharmakologisch wirksam.

Die Kombination der ungeschützten Pyranosederivate der Formel I mit Antibiotika gehört zum Gegenstand der Erfindung und bewirkt eine Steigerung der antibiotischen Aktivität.

Die Erfindung betrifft auch ein Verfahren zur Steigerung der antibiotischen Aktivität von Antibiotika, dadurch gekennzeichnet, dass man ein Pyranosederivat der Formel I, insbesondere eine Muramyl- oder Normuramylverbindung mit einem Antibiotikum zusammen verabreicht. Dabei kann das Pyranosederivat bis zu 24 Stunden vor oder nach, vorzugsweise jedoch etwa gleichzeitig mit dem Antibiotikum verabreicht werden. Als Kombinationspartner verwendet man insbesondere die oben als bevorzugte Verbindungen genannten Muramyl- und Normuramylverbindungen.

Die Verabreichung der Antibiotika erfolgt auf dem üblichen Wege, wie subkutan, intravenös oder oral, während man die Muramylpeptide, insbesondere wenn sie getrennt von den Antibiotika verabreicht werden, meist subkutan verabfolgt.

Von den zur Kombination mit den erfindungsgemässen Pyranosederivaten in Frage kommenden Antibiotika sind besonders solche aus den Gruppen der β-Lactamantibiotika, Aminoglycoside, Tetracycline, Macrolide, Lincomycine, Polyenantibiotika, Polypeptidantibiotika, Anthracycline, Chlor- und Thiamphenicole, Cycloserine, Fusidinsäuren oder Rifamycine zu nennen.

Als bevorzugte Antibiotika seien unter den β-Lactamen die Penicilline Cephalosporine, Peneme, Nocardicine, Thienamycine und Clavulanverbindungen, z.B. die Clavulansäuren, genannt.

Penicillin-Antibiotika sind in erster Linie Amoxycillin, Ampicillin, Carbenicillin, Cloxacillin, Cyclacilli-n, Dicloxacillin, Mecillinam, Methicillin, Penicillin G, Penicillin V, Pivampicillin, Sulbenicillin, Azlocillin, Ticarcillin, Mezlocillin, Pivmecillinam oder 6-(4-endo-Azatricyclo[5.2.2.$0^{2,6}$]undec-8-enyl)-methylenaminopenicillansäure genannt.

Aus der Gruppe der Cephalosporine können z.B. Cefaclor, Cefazaflur, Cefazolin, Cefadroxil, Cefoxitin, Cefuroxim, Cephacetril, Cephalexin, Cephaloglycin, Cephaloridine, Cephalotin, Cefamandol, Cephanon, Cephapirin, Cefatrizin, Cephradin, Cefroxadin (7 β-[D-2-amino-2-(1,4-cyclohexadienyl)-acetamido]-3-methoxy-3-cephem-4-carbonsäure = CGP 9000), Cefsulodin, Cefotaxim, Cefotiam, Ceftezol oder Cefazedon genannt werden.

Unter den Nocardicinen ist z.B. Nocardicin A und unter den Thienamycinen und Clavulansäuren z.B. Thienamycin bzw. Clavulansäure zu erwähnen.

Unter den Aminoglycosiden sind insbesondere Streptomycine, z.B. Streptomycin und Streptomycin A, Neomycine, z.B. Neomycin B, Tobramycine, z.B. Tobramycin oder Dibekacin, Kanamycine (z.B. Gemische von Kanamycin A, B und C), sowie Amicacine, Gentamycine (z.B. Gemische von Gentamycin A, $C_1$, $C_2$ oder $C_{1a}$) oder Sisomicine, wie Sisomicin oder Netilmicin, ferner Lividomycin, Ribocamycin und Paromomycin zu erwähnen.

Als Tetracycline sind insbesondere Tetracyclin, Doxycyclin, Chlortetracyclin, Oxytetracyclin oder Methacyclin zu nennen.

Als Macrolide sind z.B. Maridomycin, Spiramycine, wie Spiramycin I, II und III, Erythomycine, z.B. Erythromycin, Oleandomycine, z.B. Oleandomycin und Tetraacetyloleandomycin, und als Lincomycine z.B. Lincomycin und Clindamycin zu erwähnen.

Als Polyen-Antibiotika sind besonders Amphotericin B und dessen Methylester oder Nystatin zu nennen.

Als Polypeptid-Antibiotika können z.B. Colistin, Gramicidin S, Polymyxin B, Virginiamycin,Tyrothricin, Viomycin oder Vancomycin besonders genannt werden.

Die erfindungsgemässen Kombinationspräparate weisen pro Dosiseinheit die üblichen Mengen an Antibiotika auf z.B. zwischen 50 und 1000 mg, in der Regel zwischen 100 und 500 mg. Die Pyranosederivatmenge richtet sich nach dem vorgesehenen Applikationsort. Sie ist für oral verabreichbare Präparate höher als für injizierbare. Oral applizierbare Präparate enthalten zwischen 1 mg bis zur Hälfte der Antibiotikamenge an Pyranosederivat der Formel I, in der Regel zwischen 5 und 50 mg. Bei Verwendung von magensaftbeständigen Manteltabletten kann die Dosierung auch weniger als 1 mg (bis zu 0,01 mg) Muramylpeptid pro Tablette betragen. Injizierbare Präparate enthalten zwischen 10 µg und 50 mg, bevorzugt zwischen 100 µg und 10 mg Pyranosederivat. Diese Präparate können, insbesondere wenn sie zur oralen Gabe vorgesehen sind, ausserdem noch die üblichen Mengen an pharmakologischen Trägerstoffen, Streck- und/ oder Verdünnungsmitteln enthalten. Insbesondere für injizierbare Präparate sind auch liposomale Applikationsformen geeignet.

Besonders hervorzuheben sind pharmazeutische oder veterinärmedizinische Präparate, sowie Futtermittel oder Futtermittelzusätze, die eine wirksame oder unterwirksame Dosis der genannten Antibiotika und zusätzlich ein Muramylpeptid der Formel I enthalten.

Im Verfahren der vorliegenden Erfindung

verwendet man eine wirksame oder unterwirksame Dosis des Antibiotikums, je nach Art des letzteren z.B. von etwa 10 bis etwa 1000 mg, insbesondere von etwa 50 bis etwa 500 mg pro Einzeldosis.

Die Dosierung der Pyranosederivate der Formel 1 und ihrer Salze richtet sich nach dem Applikationsort, entspricht der für die pharmazeutischen Präparate genannten Dosierung und liegt z.B. für 70 kg schwere Warmblüter zwischen 1 mg und 100 mg täglich (oral).

Die hohe antibiotische Wirkung der neuen Präparate und des neuen Verfahrens kann durch "in vivo"-Versuche nachgewiesen werden, die am verschiedenen Tierarten, insbesondere an Säugetieren, wie Mäusen, durchgeführt werden. Dazu infiziert mman sie mit einer letalen oder subletalen Dosis eines pathogenen Mikroorgamismus und gibt dann das genannte neue Präparat bzw. die einzelnen Dosen am Muramylpeptid und Antibiotikum. Die Wirkung wird als $ED_{50}$ bestimmt, das ist diejenige Dosis, bei der 50% der Tiere überleben.

Es wurde nun überraschenderweise gefunden, dass die Infektion mit pathogenen Keimen, insbesondere der weniger beeinflussbaren gramnegativen Bakterien, wie z.B. Stämmen der Aerobakter, Brucella, Escherichia, Klebsiella, Malleomyces, Neisseria, Pasteurella, Proteus, Pseudomonas, Shigella und Vibrio, aber auch von gram-positiven Bakterien, wie von Aktinomycetes, Clostridia, Corynebakterien, Diplokokken, Mycobakterien oder Staphylokokken, oder von Pilzen, wie Candida Albicans, Cryptokokkus neoformans, Plastomyces dermatitides oder Hystoplasma capsulatum, in erhöhtem Masse gehemmt und bekämpft wird.

Die pharmazeutischen Präparate der vorliegenden Erfindung sind vorzugsweise Tabletten oder Gelatinekapseln, welche die Wirkstoffe zusammen mit Verdünnungsmitteln, z.B. Laktose, Dextrose, Saccharose, Mannit, Sorbit, Cellulose und/oder Glucose und Schmiermitteln, z.B. Kieselerde, Talk, Stearinsäure oder Salze davon, wie Magnesium- oder Calciumstearat, und/oder Polyäthylenglykol, enthalten. Tabletten enthalten ebenfalls Bindemittel, z.B. Magnesiumaluminiumsilikat, Stärken, wie Mais-, Weizen-, Reis- oder Pfeilwurzstärke, Gelatine, Traganth, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat und/oder Brausemischungen, oder Adsorptionsmittel, Farbstoffe, Geschmackstoffe und Süssmittel. Injizierbare Präparate sind vorzugsweise isotonische wässrige Lösungen oder Suspensionen. Suppositorien, Salben oder Cremen sind in erster Linie Fettemulsionen oder -suspensionen. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wertvolle Stoffe enthalten können, werden in an sich bekannter Weise, z.B. mittels konventioneller Misch-, Granulieroder Dragierverfahren, hergestellt und enthalten von etwa 0,1% bis etwa 75%, insbesondere von etwa 1% bis etwa 50%, der genannten Aktivstoffe.

Die oral applizierbaren Präparate der vorliegenden Erfindung können auch mit einem gegen Magensaft beständigen Ueberzug versehen sein.

Die nachfolgenden Beispiele illustrieren die Erfindung, ohne sie in irgendeiner Form einzuschränken.

Allgemeines: Die angegebenen $R_f$-Werte wurden auf Kieselgeldünnschichtplatten der Firma Merck ermittelt. Das Verhältnis der Laufmittel in den verwendeten Laufmittelgemischen zueinander ist in Volumenanteilen (V/V) angegeben. Temperaturen sind in Grad Celsius angegeben.

**Beispiel 1:**

Zu 1,9 g N-Acetyl-1α-0-benzyl-4,6-0-isopropyliden-normuramyl-L-O-[N-behenoyl-glycyl]-seryl-D-i-soglutamin-tert.butylester, gelöst in 20 ml abs. Essigsäureäthylester, werden bei Raumtemperatur unter Rühren und Ausschluss von Feuchtigkeit 20 ml einer 4,5 normalen Lösung von Chlorwasserstoffsäure im gleichen Lösungsmittel gegeben. Das nach wenigen Minuten kristallin ausfallende Produkt wird nach lstündigem Rühren mit 30 ml Aether versetzt, der Niederschlag abgesaugt, gewaschen und über Natronasbest getrocknet, Ausbeute 1,6 g mit $R_f$ = 0,55 (Chloroform:Methanol:Wasser = 70:30:5).

1 g (1,05 mmol) des oben erhaltenen Produkts, gelöst in 20 ml Dimethoxyäthan/Wasser 20:1, werden in Gegenwart von 0,2 g Pd-Kohle (10%ig) während 20 Stunden bei 20° und 24 Stunden bei 35° hydriert. Der Katalysator wird abfiltriert, mit dem gleichen Lösungsmittelgemisch gewaschen und das Filtrat, das laut Dünnschichtchromatographie nur Nebenprodukte enthält, verworfen.

Das Produkt wird durch intensive Extraktion des Kntalysators mit Chloroform/Essigsäureäthylester 1:1 und Methanol gewonnen (0,53 g) und wird durch Chromatographie an Kieselgel (1:100) im System Chloroform/ Methanol/Wasser 70:30:5 (3 ml Fraktionen) weitergereinigt. Das in den Fraktionen 66-125 anfallende Material, teilweise als Na-Salz(= aus Kieselgel) vorliegend, wird in Wasser gelöst, auf eine stark saure Ionenaustauschersäule (Dowex 50, H-Form) gegeben und erst mit Wasser, dann mit Dimethoxyäthan/Wasser 1:1 eluiert (2 ml Fraktionen). Die Eluate werden vereinigt und das Dimethoxyäthan im Vakuum verdampft. Diese Suspension wird durch Zugabe von tert. Butanol

in Lösung gebracht, durch ein Milliporfilter (Teflon; 0,2 µ) filtriert und lyophilisiert.

Es verbleiben 0,327 g (36%) N-Acetyl-normuramyl-L-(O-[N-behenoylglycyl])-seryl-D-isoglutamin als lockeres Pulver mit $R_f$ = 0,19 (Chloroform:Methanol:Wasser = 70:30:5), $R_f$ = 0,38 (Acetonitril:Wasser = 3:1), $R_f$ = 0,45 (Essigsäureäthylester:n-Butanol:Pyridin:Eisessig: Wasser = 42:21:21:6:10).

Das im vorliegenden Beispiel verwendete Ausgangsmaterial kann wie folgt erhalten werden:

2,3 g N-Acetyl-1α-0-benzyl-4,6-0-isopropyliden-normuramyl-L-(O-[N-carbobenzoxy-glycyl])-seryl-D-i-soglutamin-tert.butylester werden in 50 ml Dimethoxyätham/Wasser 20:1 gelöst und nach Zugabe von 0,5 g Pd-Kohle (10%ig) während 20 Minuten mit Wasserstoff behandelt. Der pH der Lösung wird durch Zugabe von 0,1 N HCl (wässrig) auf 5 gehalten. Der Katalysator wird abfiltriert, die Reaktionslösung eingedampft und der Rückstand über Phosphorpentoxid getrocknet.

2 g des so gewonnenen Materials werden in 20 ml Pyridin gelöst, 0,26 g N-Methylmorpholin zugefügt und bei 0° unter Rühren 1,9 g Behensäurechlorid,gelöst in 1 ml Methylenchlorid, zugefügt.

Nach 4stündigem Rühren bei Raumtemperatur wird das Pyridin unter vermindertem Druck abgedampft, der Rückstand in 200 ml Essigsäureäthylester aufgenommen und mit jeweils 20 ml Portionen wie folgt extrahiert: 2mal mit Wasser, dann 2mal mit Natriumhydrogencarbonatlösung (0,3%ig) und schliesslich noch 3mal mit Wasser. Nach Trocknen über Natriumsulfat wird das Lösungsmittel verdampft und der Rückstand am Kieselgel (1:50) im System Chloroform/Isopropanol = 95:5 (15 ml Fraktionen) chromatographiert. Die entsprechenden Fraktionen (265-370) werden vereinigt und zur Trockne verdampft und ergeben 2,0 g N-Acetyl-1α-0-benzyl-4,6-0-isopropyliden-normuramyl-L-O-[N-behenoyl-glycyl]-seryl-D-isoglutamintert.butylester als schwach gelblichen Schaum,

$R_f$ = 0,85 (Chloroform:Methamol:Wasser = 70:30:5),

$R_f$ = 0,27 (Chloroform:Isopropanol:Eisessig = 70:8:2).

2,5 g (3,7 mmol) N-Acetyl-1α-0-benzyl-4,6-0-isopropyliden-normuramyl-L-seryl-D-isoglutamin-tert.butylester, 0,75 g Carbobenzoxyglycin, 0,45 g Dimethylaminopyridin und 1,0 g 1-Hydroxybenztriazol werden in 25 ml Dimethylformamid gelöst. Mam kühlt auf 0° ab, fügt 0,85 g Dicyclohexylcarbodiimid hinzu und rührt das Ganze während 22 Stunden bei Raumtemperatur. Die Suspension wird filtriert und der Niederschlag mit Essigsäureäthylester gewaschen. Die vereinigten Filtrate werden durch weitere Zugabe von Essigsäureäthylester auf ein Volumen von 250 ml gebracht, 3mal mit je 20 ml Wasser, 3mal mit je 20 ml 2%iger Natriumhydrogencarbonatlösung und schliesslich 4mal mit Wasser gewaschen. Nach Trocknen über Natriumsulfat wird das Lösungsmittel verdampft und der Rückstand an Kieselgel in einer Mischung von Chloroform/Isopropanol = 9:1 chromatographiert (10 ml Fraktionen). Die Fraktionen 75 bis 190 werden gesammelt, zur Trockne verdampft und der farblose Schaum nach Lösen in 20 ml Essigsäureäthylester und Zugabe von 20 ml Aether und 100 ml Petroläther zur Kristallisation gebracht. Nach Absaugen und Trocknen im Vakuum verbleiben 2,7 g (87%) N-Acetyl-1α-0-benzyl-4,6-0-isopropyliden-normuramyl-L-(O[N-carbobenzoxy-glycyl])-seryl-D-isoglutamin-tert.-butylester mit Zersetzungspunkt 104°,

$$[\alpha]_D^{20}$$

= + 75 ± 1° (c=0,9; Essigsäureäthylester), $R_f$ = 0,78 (Chloroform: Methanol: Wasser = 70:30:5), $R_f$ = 0,30 (Chloroform: Isopropanol: Eisessig = 70:8:2).

7,0 g (14,35 mmol N-Acetyl-1α-0-benzyl-4,6-0-isopropyliden-normuraminsäure-Na-Salz werden in 70 ml Dimethylformamid suspendiert, 4,7 g L-Seryl-D-isoglutamin-tert.-butylester-hydrochlorid und 4,3 g N-Aethoxycarbonyl-2-äthoxy-1,2-dihydrochinolin hinzugefügt und das Ganze während 19 Stunden bei Raumtemperatur gerührt. Man verdampft zur Trockne, gibt den Rückstand auf eine Kieselgelsäule (1:20), wäscht das entstandene Chinolin mit Chloroform herunter und eluiert das Produkt mit Chloroform/Methanol = 95:5 in 5 ml Fraktionen. Das in den Franktionen 35 bis 100 enthaltene reine Material wird gesammelt. Man erhält 6,6 g (68%) N-Acetyl-1α-C-benzyl-4,6-0-isopropyliden-normuramyl-L-seryl-D-isoglutamin-tert.-butylester als farblosen Schaum mit

$$[\alpha]_D^{20}$$

= +81 ± 1° (c=1, Methanol).

**Beispiel 2:**

2,5 g N-Acetyl-1 α-0-benzyl-4,6-0-isopropyliden-nonnuramyl-L-(O-behenoyl)-seryl-D-isoglutamin-tert.-butylester werden analog Beispiel 1 mit 40 ml 2N HCl in Essigsäureäthylester gespalten. Nach Istündigem Rühren bei Raumtemperatur wird das Produkt durch Zugabe von 50 ml abs. Aether ausgefällt, nach 15minütigem Rühren in der Kälte filtriert und der Niederschlag über Natronasbest getrocknet, $R_f$ = 0,43 (Chloroform: Methanol: Wasser = 70:30:5).

Das schwach verunreinigte Produkt wird in 25 ml Dimethoxyäthan/Wasser= 20:1 in Gegenwart

von Palladium auf Bariumsulfat (10%ig) während 65 Stunden hydriert. Der Katalysator wird abfiltriert, gewaschen und die vereinigten Filtrate zur Trockne verdampft (0,8 g). Der Rückstand wird 2mal an Kieselgel (1:50) im System Chloroform/Methanol = 7:3 (5 ml Fraktionen) chromatographiert, die entsprechenden Fraktionen vereint und zur Trockne verdampft. Der Rückstand wird in Dimethoxyäthan/Wasser 1:1 gelöst und wie üblich an 10 ml Dowex 50, H-Form, entsalzt. Das Eluat wird eingeengt, mit tert. Butanol versetzt und nach Filtration durch einen Milliporfilter (Teflon, 0,2μ) lyophilisiert. Man erhält 0,246 g N-Acetyl-normuramyl-L-(O-behenoyl)seryl-D-isoglutamin als farbloses Pulver. $R_f$ = 0,23 (Chloroform: Methanol: Wasser = 70:30:5), $R_f$ 0,59 (Acetonitril:Wasser = 3:1), $R_f$ = 0,39 (Essigsäureäthylester: n-Butanol: Pyridin:Eisessig:Wasser = 42:21:21:6:10).

Das Ausgangsmaterial erhält man wie folgt:

1,5g N-Acetyl-1α-0-benzyl-4,6-0-isopropyliden-normuramyl-L-seryl-Disoglutamin-tert. butylester werden in 15 ml Pyridin gelöst, auf 0° abgekühlt, und 1,6 g Behensäurechlorid, gelöst in 0,5 ml Dichlormethan, zugegeben. Nach 22 stündigem Rühren bei Raumtemperatur wird das Pyridin unter vermindertem Druck abdestilliert. Das gelbliche Oel wird in 200 ml Essigsäureäthylester aufgenommen und in der Kälte 2mal mit je 15 ml 1 N Zitronensäurelösung, 2mal mit je 15 ml Wasser, dann 2mal mit 5%iger Natriumhydrogencarbonatlösung und wieder mit Wasser gewaschen. Nach Trocknen über Natriumsulfat wird das Lösungsmittel entfernt. Man erhält 2,5 g N-Acetyl-1α-0-benzyl-4,6-0-isopropylidennonnuramyl-L-(O-behenoyl)-seryl-D-isoglutamin-tert. butylester mit $R_f$ = 0,85 (Chloroform: Methanol: Wasser = 70:30:5), $R_f$ = 0,43 (Chloroform: Isopropanol: Eisessig = 70:8:2).

$$[\alpha]_D^{20}$$

= + 43° (c = 0,611, Chloroform).

**Beispiel 3:**

Eine Lösung von 0,40 g N-Acetyl-1α-0-benzyl-muramyl-L-(O-[N-{DL-2-n-hexadecanoylamino-n-hexadecanoyl}-L-alanyl])-threonyl-D-isoglutamin-γ-benzylester in 30 ml Dimethylacetamid wird während 8 Tagen in Gegenwart von 2 g eines Palladium/Barium-Katalysators (10%ig) mit Wasserstoff behandelt. Der Katalysator wird abfiltriert, das Filtrat zur Trockene verdampft und der Rückstand zur Entfernung von noch vorhandenem Dicyclohexylharnstoff an Silikagel (1:100) im System Chloroform-Methanol 8:2 (2 ml Fraktionen) chromatographiert. Das in den Fraktionen 225-57 enthaltene debenzylierte Material wird zur Trockene verdampft. Der Rückstand wird in doppelt destilliertem Wasser aufgenommen, durch einen Teflonfilter (Millipor, 0,2μ) filtriert und die Lösung lyophilisiert.

Man erhält so das N-Acetyl-muramyl-L-O-[N-{DL-2-n-hexadecanoylamino-n-hexadecanoyl}-L-alanyl]-threonyl-D-isoglutamin als lockeres Pulver (0,17 g) in Form des Natriumsalzes,

$$[\alpha]_D^{20}$$

= +1 ± 1° (c=0,5; Wasser), $R_f$ = 0,33/0,30 (Diastereomerenpaar, Chloroform/MethanoL/Wasser = 70:30:5) und $R_f$ = 0,75(Essigsäureäthylester/n-ButanoL/Pyridin/Eisessig/Wasser = 42:21:21:6:10).

Das Ausgangsmaterial erhält man wie folgt:

Eine Lösung von 0,38 g DL-2-n-Hexadecanoylamino-n-hexadecansäure und 0,173 g N-Hydroxy-succinimid in einem Gemisch von 5 ml 1,2-Dimethoxy-äthan, 2 ml Dimethylformamid und 2 ml Chloroform wird mit 0,185 g Dicyclohexylcarbodiimid versetzt. Nach halbstündigem Rühren bei Raumtemperatur werden 0,622 g N-Acetyl-1α-0-benzyl-muramyl-L-(O-L-alanyl)-threonyl-D-isoglutamin-γ-benzylester-trifluoracetat und 0,09 ml N-Methylmorpholin zugegeben und mit 2 ml des obengenannten Gemisches nachgespült. Nach 23stündigem Rühren bei Raumtemperatur wird die Suspension mit 10 ml Essigsäureäthylester verdünnt, 30 Minuten im Eisbad gerührt und filtriert. Nach mehrfachem Verreiben des Filterrückstandes mit Methanol, Wasser und wieder Methanol erhält man 0,7 g N:Acetyl-1α-C-benzyl-muramyl-L-0-[N-{DL-2-n-hexadecanoylamino-n-hexadecanoyl}-L-alanyl]-threonyl.-D-isoglutamin-γ-benzylester, noch verunreinigt durch Dicyclohexylharnstoff, $R_f$ =0,35/0,31 (Diasteroisomerengemisch, Chloroform/Methanol 9:1).

1 g N-Acetyl-1α-0-benzyl-4,6-0-isopropyliden-muramyl-L-O-[tert.-butyloxycarbonyl-L-alanyl]-threonyl-D-isoglutamin-γ-benzylester, gelöst in 15 ml Trifluoressigsäure, wird während 90 Minuten bei Raumtemperatur stehen gelassen. Di-e Reaktionslösung wird bei 25° am Rotationsverdampfer stark eingeengt und das Produkt durch Zugabe von 20 ml Diäthyläther ausgefällt. Das Ueberstehende wird abdekantiert, der Rückstand mit neuem Aether verrieben und wieder abgegossen. Nach Wiederholen der gleichen Operation und Trocknen des Rückstandes über Natronasbest (Merck AG) erhält man 0,74 g N-Acetyl-1α-0-benzyl-muramyl-L-(O-L-alanyl)-threonyl-D-isoglutamin-γ-benzylester-trifluoracetat als farbloses Pulver mit $R_f$ = 0,40(Chloroform/Methanol/Wasser = 70:30:5) und $R_f$=0,52 (Essigsäureäthylester/n-Butanol/Pyridin/Eisessig/Wasser = 42:21:21:6:10).

2,2 g N-Acetyl-1α-O-benzyl-4,6-0-isopropyliden-muramyl-L-threonyl-D-isoglutamin-γ-benzylester, 0,57 g N-10C-L-alanin, 0,367 g

Dimethylaminopyridin und 0,811 g N-Hydroxybenztriazol werden nacheinander in einem Gemisch von 10 ml Dimethylformamid und 5 ml Dichlormethan gelöst. Nach Abkühlen auf 0° werden 1,24 g Dicyclohexylcarbodiimid hinzugefügt und das Ganze während 25 Stunden bei Raumtemperatur gerührt. Zur Aufarbeitung wird mit Essigsäureäthylester (250 ml) verdünnt, der unlösliche Dicyclohexylharnstoff abgesaugt und das Filtrat mit je 20 ml Portionen (3mal) mit Wasser, 0,6N-Zitronensäurelösung, gesättigter Natriumhydrogencarbonatlösung und wieder Wasser extrahiert.

Das nach dem Verdampfen des Lösungsmittels wegen partieller Razemisierung des L-Alanins als Diastereomerengemisch anfallende Produkt kann durch wiederholte Chromatographie an Silikagel (1:50) in Chloroform/Methanol = 95:5 in die Komponenten aufgetrennt werden. Das im Dünnschichtchromatogramm rascher laufende L-Alanin enthaltende Diastereomere (Hauptteil, 1 g) N-Acetyl-1α-0-benzyl-4,6-0-isopropyli-den-L-(O-[tert.-butyloxycarbonyl-L-alanyl])-threonyl-D-isoglutamin-γ-benzylester zeigt einen $R_f$ = 0,52, das D-Alanin enthaltende Isomere einen solchen von 0,48 (System: Chloroform/Methanol = 9:1).

8,9 g N-Acetyl-1α-benzyl-4,6-0-isopropyliden-muraminsäure-Na-salz, 6,8 g L-Threonyl-D-isoglutamin-γ-benzylesterhydrochlorid und 4,2 g N-Hydroxysuccinimid werden in einem Gemisch, bestehend aus 30 ml Dimethylformamid und 6 ml Dichlormethan, suspendiert. Man kühlt auf 0° ab, fügt 4,51 g Dicyclohexylcarbodiimid hinzu und rührt das Ganze während 16 Stunden bei Raumtemperatur. Die dicke Suspension wird mit 50 ml Essigsäureäthylester verdünnt, der Niederschlag abgesaugt und das Filtrat zur Trockne verdampft. Der dabei erhaltene Rückstand wird in 300 ml Essigsäureäthylester aufgenommen und in der Kälte auf die übliche Weise mit Wasser, 0,6N-Zitronensäurelösung, gesättigter Natriumhydrogencarbonatlösung und wieder Wasser gewaschen.

Das nach dem Verdampfen des Lösungsmittels verbleibende Oel wird an Silikagel (1:20) im System Chloroform(Methanol 95:5 chromatographiert. Die Fraktionen 25-47 enthalten 9,7 g (70% d. Th.) reinen N-Acetyl-1 α-O-benzyl-4,6-O-isopropyliden-muamyl-L-threonyl-D- isoglutamin- γ-benzylester, weitere 10% können durch eine wiederholte Chromatographie der Mischfraktion gewonnen werden,

$$[\alpha]_D^{20}$$

= +92 ± ° (c=1; Methanol), $R_f$ = 0,84 (Chloroform/Methanol/Wasser = 75:30:5) und $R_f$ = 0,49 (Chloroform/Methamol 9:1).

9,12 g N-BOC-L-threonyl-D-isoglutamin-γ-benzylester werden in 150 ml trockenem Essigsäureäthylester gelöst und bei Raumtemperatur unter Rühren und Ausschluss von Feuchtigkeit mit dem gleichen Volumen einer 4,5 normalen Lösung von Salzsäure in Essigsäureäthylester versetzt. Die sofort beginnende Abscheidung des Spaltproduktes wird nach 1 1/4 Stunden durch Zugabe von 200 ml Diäthyläther vervollständigt. Der Niederschlag wird abfiltriert, mit Aether gewaschen und im Vakuum über Natriumasbest (Merck AG) getrocknet. Man erhält 7,4 (95% d. Th) L-Threonyl-D-isoglutamin-γ-benzylester-hydrochlorid als farbloses Pulver, Zersetzungspunkt 196-98°,

$$[\alpha]_D^{20}$$

= +13 ± 1° (c=1,- Methanol), $R_f$ = 0,71 (Chloroform/Methanol/Wasser) und $R_f$ = 0,80 (Essigsäureäthylester/n-Butanol/Pyridin/Eisessig/Wasser = 42:21:21:6:10).

Das Ausgangsmaterial erhält man wie folgt:

5,48 g BOC-L-threonin und 6,82 g D-Isoglutamin-γ-benzylester-hydro-chlorid werden in einem Gemisch von 10 ml Dimethylfonnamid und 50 ml Essigsäureäthylester suspendiert und nach Zugabe von 2,78 ml N-Methylmorpholin und 6,18 g 1-Aethoxycarbonyl-2-äthoxy-1,2-dihydrochinolin (EEDQ) während sieben Stunden bei Raumtemperatur gerührt. Die orangegelbe Suspension wird in 300 ml Essigsäureäthylester aufgenommen und auf die übliche Weise gewaschen. Der nach dem Verdampfen des Lösungsmittels verbleibende Neutralkörper wird durch Chromatographie an Silikagel (1:25) im System Chloroform/Methanol = 95:5 gereinigt. Die reinen Fraktionen werden gesammelt. Man erhält 10,2 g N-BOC-L-threonyl-D-isoglutamin-γ-benzylester als farbloses Harz,

$$[\alpha]_{546\ nm}^{20}$$

= -13 + 1° (c=1. Methanol), $R_f$ = 0,65 (Chloroform/Methanol/Wasser = 70:30:5) und $R_f$ = 0,80 (Acetonitril/Wasser = 3:1).

**Beispiel 4:**

In analoger Weise zu den vorstehenden Beispielen lassen sich, ausgehend von entsprechenden Ausgangsstoffen, die folgenden Verbindungen erhalten:

N-Acetyl-normuramyl-L-(O-[N-{12-hydroxy-cis-9-octadecenoyl}-glycyl])-seryl-D-isoglutamin,

N-Benzoyl-normuramyl-L-(O-stearoyl)-seryl-D-isoglutamin,

N-Acetyl-muramyl-L-(O-[ω-n-stearoylamino-n-undecanoyl])-threonyl-D isoglutamin,

N-Acetyl-muramyl-L-(O-[N-{3-hydroxy-ätio-cholenoyl}-6-aminohexanoyl]) γ-hydroxyprolyl-D-

isoglutamin,
N-Acetyl-muramyl-L-(O-behenoyl)-tyrosyl-D-isoglutamin,
N-Acetyl-muramyl-L-(S-stearoyl)-cysteinyl-D-isoglutamin,
N-Acetyl-muramyl-L-[C-γ]-(tetracosylamido-glycyl)-glutamyl-Disoglutamin,
N-Acetyl-muramyl-L-[($C_γ$)-lauryl]-α-glutamyl-D-isoglutamin, und N-Acetyl-normuramyl-L-γ-stearoylamino-α-amino-butanoyl-D-isoglutamin.

**Beispiel 5:**

1.09 g (3 mMol) 2-Phenyl-4,5-(3-0-carboxymethyl-5,6-0-isopropyliden-D-glucofurano)-$\Delta^2$-oxazolin und 0,35 g N-Hydroxysuccinimid löst man in 6 ml Dimethylformamid, kühlt im Eisbad und gibt 0,74 g (3,6 mMol) Dicyclohexylcarbodiimid zu. Nach 30 Minuten versetzt man mit 0,45 ml Triäthylamin und tropft langsam unter gutem Rühren eine Lösung von 1,7 g L-(O-Stearoyl)-seryl-D-glutaminsäuredimethylester-hydrochlorid in 10 ml Dimethylformamid zu. Man rührt noch 0,5 Stunden unter Eiskühlung und 4 Stunden bei Raumtemperatur. Das Reaktionsprodukt hat Rf = 0.64 und wird auf folgende Weise gereinigt:

Nach Eindampfen der Reaktionslösung am Oelvakuum nimmt man den Rückstand mit Chlorofonn auf, schüttelt dreimal mit Wasser aus und extrahiert die wässrige Phase mit $CHCl_3$. Nach Trocknen und Eindampfen der $CHCl_3$-Phase reinigt man den Rückstand chromatographisch an Kieselgel G, Merck (Elution mit Chloroform und $CHCl_3$:Aceton = 9:1). Man erhält 1,9 g (72,5% d.Th.) reines, geschütztes Muramylpeptid als amorphes Pulver.

1,28 g dieser Verbindung hydrolysiert man während 17 Stunden bei 40° in einer Mischung aus 20 ml 0.1N Salzsäure und 40 ml Tetrahydrofuran. Dann neutralisiert man mit $NaHCO_3$-Lösung und dampft im Vakuum ein. Den so erhaltenen Rückstand chromatographiert man an Kieselgel G, Merck, durch sukzessive Elution mit $CHCl_3$, $CHCl_3$:Aceton = 9:1, $CHCl_3$: Aceton = 7:3 und $CHCl_3$:Methanol = 9:1.

Man erhält 0, 54 g.43,5% d.Th., N-Benzoyl-normuramyl-L-(O-stearoyl-L-seryl)-D-glutaminsäure-dimethylester mit $R_f$ = 0,65, ($CHCl_3$:Metha-nol = 85:15). Durch Umfällen aus Aether/Petroläther erhält man ein mikrokristallines Pulver vom Schmelzpunkt 56-61° mit

$$[\alpha]_D^{20}$$

= +25±1° (c = 1, DMSO).

Das als Ausgangsmaterial verwendete 0-Stearoyl-L-seryl-D-glutaminsäuredimethylester-hydrochlorid erhält man wie folgt:

Zu 4,1 g (2 mMol) tert.Butyloxycarbonyl-L-serin, 4,23 g (2 mMol) D-Glutaminsäure-dimethylesterhydrochlorid und 1,01 g (2 mMol) N-Methylmorpholin in 40 ml Dimethylformamid gibt man 5,4 g (2,2 mMol) 2-Aethoxy-N-carboethoxy-1,2-dihydrochinolin (EEDQ). Nach 16 stündigem Rühren bei Raumtemperatur wird die entstandene Suspension filtriert und das Filtrat zur Trockne verdampft. Der ölige Rückstand wird in 150 ml Essigsäureäthylester aufgenommen und in der Kälte 5 mal mit je 50 ml 2N-Zitronensäurelösung, dann 2 mal mit je 50 ml Wasser gewaschen. Die organische Phase wird getrocknet, auf ca. 40 ml eingeengt und durch portionenweise Zugabe von 150 ml Aether/Petroläther = 2:1 zur Kristallisation gebracht. Man erhält 4,95 g (79%) tert.-Butyloxycarbonyl-L-seryl-D-glutaminsäuredimethylester als farblose Nadeln vom Fp. 90-92°, $R_f$ = 0,69 (Acetonitril/Wasser = 3:1), $R_f$ = 0,60 (n-ButanoL/Essigsäure/Wasser = 75:75:21).

Zu einer Lösung von 3,63 g (10 mMol) tert.Butyloxycarbonyl-L-seryl-D-glutaminsäuredimethylester in 100 ml abs. Pyridin werden unter Rühren 3,03 g (10 mMol) Stearinsäurechlorid, gelöst in 30 ml abs. 1,2-Dimethoxyäthan, innerhalb von 15 Minuten eingetropft. Das nach 30stündigem Stehen bei 4°C ausgefallene Pyridinhydrochlorid wird abfiltriert, das Filtrat am Hochvakuum eingedampft und getrocknet (5,9 g).

5,5 g (8,7 mMol) der wachsartigen Masse werden in 100 ml absolutem Essigsäureäthylester gelöst und unter Rühren und Ausschluss von Feuchtigkeit bei 0° mit 40 ml kalter 4,5N HCl in Essigsäureäthylester versetzt. Nach 45minütigem Rühren wird die Essigesterphase 4 mal mit je 50 ml kaltem Wasser extrahiert, getrocknet und nach dem Verdampfen des Lösungsmittels aus 100 ml tert. Butanol lyophilisiert. Man erhält 4,8 g 0-Stearoyl-L-seryl-D-glutaminsäure-dimethylesterhydrochlorid als stark hygroskopisches Pulver,

$$[\alpha]_D^{20}$$

= +11±1° (c = 1,2; Methanol);

$R_f$ = 0,46 (n-Butanol/Essigsäure/Wasser = 75:7,5:21),

$R_f$ = 0,83 (Essigsäureäthylester/n-ButanoL/Pyridin/Essigsäure/Wasser = 42:21:21:6-10).

**Beispiel 6:**

1,45 g (4 mMol) 2-Phenyl-4,5-(3-0-carboxymethyl-5,6-iso-propyliden-D-glucofurano)-$\Delta^2$-oxazolin, 0,78 g Hydroxybenztriazol, 1 07 g N-Aethyl-N'-(3-dimethylamino-propyl)-carbodiimid-hydrochlorid und 3 g L-0-Behenoyl-seryl-D-

isoglutamin-γ-benzylester-hydrochlorid (4,4 mMol) löst man in 40 ml abs. Dimethylformamid und stellt den pH-Wert mit 2,2 ml Triäthylamin auf 7 ein. Nach 18 Stunden bei Raumtemperatur dampft man im Vakuum zum Sirup ein, nimmt mit Essigester auf und schüttelt mit Wasser, $NaHCO_3$-Lösung und nochmal mit Wasser aus. Die über $Na_2SO_4$ getrocknete organische Phase gibt nach Eindampfen 2,5 g eines Rohproduktes, das an 80 g Kieselgel Merck mit CHCl3/Methanol 95:5 chromatographisch gereinigt wird. Man erhält 1,2 g des geschützten Muramylpeptides in reiner Form als amorphe Substanz mit $R_f$ = 0,55.

Zur Oeffnung des Oxazolinringes und zur Abspaltung der Isopropylidengruppe hydrolysiert man 1,1 g des geschützten Muramylpeptidbenzylesters in einer Mischung aus 23 ml Tetrahydrofuran und 17 ml 0.1N Salzsäure während 17 Stunden bei 45°. Danach stellt man die Reaktionslösung mit $NaHCO_3$-Lösung auf pH = 6 und dampft im Vakuum zur Trockne ein. Den Rückstand löst man in $CHCl_3$:Aethanol = 9:1, filtriert von anorganischen Salzen über Millipore-Filter und erhält 660 mg, 58% d.Th., N-Benzoyl-normuramyl-L-(O-behenoyl)seryl-D-isoglutamin-γ-benzylester als farblose Kristalle mit Zersetzungsbereich 170-180°, $R_f$ = 0,21 ($CHCl_3$:Methanol = 9:1),

$$[\alpha]_D^{20}$$

= +18 ± 1° (c = 1, Dimethylsulfoxid).

500 mg des erhaltenen Benzylesters hydriert man mit 100 mg 10%igem $Pd/BaSO_4$ in 10 ml Dimethoxyäthan/$H_2O$ = 9:1. Nach Abtrennen des Katalysators und Eindampfen der Reaktionslösung erhält man durch Verreiben des Rückstandes mit warmem Aceton 350 mg (77% d.Th.) N-Benzoyl-normuramyl-L-(O-behenoyl)-seryl-D-isoglutamin als farblose Kristalle mit Zersetzungsbereich 166-172°, $R_f$ = 0.18 ($GHCl_3$:Methanol = 7:1),

$$[\alpha]_D^{20}$$

= +18 ± 1° (c = 1, DMSO).

Das als Ausgangsstoff verwendete L-(O-Behenoyl)-seryl-D-isoglutamin-γ-benzylester-hydrochlorid wird wie folgt hergestellt:

16,4 g (80 mMol) N-tert.Butyloxycarbonyl-L-serin, 21,81 g (80 mMol) D-Isoglutamin-γ-benzylester-hydrochlorid, 8,9 ml (80 mMol) N-Methylmorpholin und 21,7 g EEDQ werden in 250 ml Dimethylformamid gelöst und über Nacht bei Raumtemperatur gerührt. Die rötliche Suspension wird bei 30° am Rotationsverdampfer eingedampft, der Rückstand in 1 1 Essigsäureäthylester aufgenommen und 4 mal mit je 200 ml Wasser extrahiert. Die getrocknete Essigesterphase wird auf ca. 100 ml eingeengt und das Produkt durch Zugabe von 1 1 Aether-Petroläther (1:1) ausgefällt. Die überstehende Lösung wird abdekantiert, das ölige Material mehrfach mit Aether verrieben, wobei es fest wird. Nach Kristallisation aus Essigsäureäthylester/Petroläther (1:10) erhält man 27 g (79%) N-tert.Butyloxycarbonyl-L-seryl-D-isoglutamin-γ-benzylester, Fp. 83-85°,

$$[\alpha]_D^{20}$$

= −9 ± 1° (c = 1; Methanol), Rf = 0,14 (Chloroform/ IsopropanoL/Essigsäure = 70:8:2), Rf = 0,90 (Essigsäureäthylester/ n-Butanol/Pyridin/Essigsäure/Wasser = 42:21:21:6:10).

Auf analoge Weise erhält man aus Carbobenzoxy-D-serin und D-Iso-glutamin-γ-tert.butylester den Carbobenzoxy-D-seryl-D-isoglutamin-γ-tert.butylester in 70%iger Ausbeute, Fp. 126-128°,

$$[\alpha]_D^{20}$$

= −7 ± 1° (c = 2; Essigsäure),
$R_f$ = 0,59 (Chloroform/Methanol/Wasser = 70:30:5),
$R_f$ = 0,17 (Chloroform/Isopropanol/Essigsäure = 70:8:2).

10,0 g (23,6 mMol) t-Butyloxycarbonyl-L-seryl-D-isoglutamin-γ-benzyl-ester werden in 60 ml abs. Pyridin gelöst und bei Raumtemperatur mit 11,0 g (30,7 mMol) Behensäurechlorid in 50 ml abs. 1,2-Dimethoxyäthan versetzt. Nach erfolgter Reaktion (1 Stunde) wird die Reaktionslösung mit 50 ml Methanol versetzt, nach 3 stündigem Stehen eingedampft und nach Aufnahme in 400 ml Essigsäureäthylester 4 mal mit je 50 ml Wasser extrahiert. Die Essigesterphase wird getrocknet, etwas eingeengt und über Nacht in der Kälte auskristallieren gelassen. Man erhält 16,5 g (94%) 0-Behenoyl-N-tert.-butyloxycarbonyl-L-seryl-D-isoglutamin-γ-benzylester, Fp. 56-57°,

$$[\alpha]_D^{20}$$

= +3 ± 1° (c = 0,25. Methanol), $R_f$ = 0,54 (Chloroform/Isopropanol/Essigsäure = 70:8:2), $R_f$ = 0,73 (n-Butanol/Essigsäure/Wasser = 75:75:21).

11,0 g (14,7 mMol) O-Behenoyl-N-tert.-butyloxycarbonyl-L-seryl-D-isoglutamin-γ-benzylester werden in 50 ml abs. Essigsäureäthylester suspendiert und bei 0° und Ausschluss von Feuchtigkeit mit 100 ml 2N HCl in Essigester versetzt. Man rührt 1 8tunde bei 0° und

dampft das Lösungsmittel bei 25° ab. Der ölige Rückstand wird mehnnals mit Petroläther verrieben, die überstehende Lösung abdekantiert und der pulverige Rückstand über Natronasbest (Merck) scharf getrocknet. Man erhält 9,3 g (93%) L-(O-Behenoyl)-seryl-D-isoglutamin-γ-benzylesterhydrochlorid als starkhygroskopisches Pulver,

$[a]_D^{20}$

= + 3 ± 1° (c = 1,1; Methanol),
$R_f$ = 0,43 (n-Butanol/Essigsäure/Wasser = 75:7,5:21), $R_f$ = 0,68 (Essigsäureäthylester/n-Butanol/Pyridin/Essigsäure/Wasser = 42:21:21:6:10).

**Beispiel 7:**

1,922 g (2 mMol) N-Acetyl-4,6-0-isopropyliden-isomuramyl-L-(O-behenoyl)-seryl-D-isoglutamin-γ-benzylester werden in 40 ml 60%iger Essigsäure gelöst und während 16 Stunden bei Raumtemperatur stehen gelassen. Man fügt 2,0 g eines Palladium auf Bariumsulfat-Ratalysators (10%ig) zu und behandelt das Ganze mit Wasserstoff. Nach 1 Stunde wird der Ratalysator abfiltriert, die Reaktionslösung mit Essigsäure verdünnt und lyophilisiert. Der Rückstand wird in 60 ml tert.-Butanol/Wasser = 95:5 gelöst, die Lösung durch ein PTFE-Milliporfilter (0,2 μ) filtriert und lyophilisiert. Man erhält das N-Acetyl-isomuramyl-L-(O-behenoyl)-seryl-D-isoglutamin als lockeres Pulver,

$R_f$ = 0,53 (Chloroform/Methanol/Wasser = 70:30:5),

$R_f$ = 0,64 (Essigsäureäthylester/n-Butanol/Pyridin/Essigsäure/Wasser = 42:21:21:6:10).

Das Ausgangsmaterial erhält man wie folgt:

Zu 1,05 g (2,6 mMol) N-Acetyl-4,6-isopropyliden-isomuraminsäure-natriumsalz, 1,80 g (2,6 mMol) L-(O-Behenoyl)-seryl-D-isoglutamin-γ-benzyl-ester-hydrochlorid und 0,60 g (5,2 mMol) N-Hydroxysuccinimid in 22 ml Dimethylformsmid werden 0,65 g (3,17 mMol) Dicyclohexylcarbodiimid gegeben und das Ganze während 16 Stunden bei Raumtemperatur gerührt. Die Suspension wird mit 80 ml Essigsäureäthylester verdünnt und das Filtrat bei 30° zur Trockne verdampft. Der ölige Rückstamd wird an Kieselgel (1:30) i-n Chloroform/Methanol = 9:1 chromatographiert. Die nach Dünnschichtchromatogramm einheitlichen Fraktionen werden gesammelt. Man erhält N-Acetyl-4,6-0-isopropyliden-isomuramyl-L-(O-behenoyl)-seryl-D-isoglutamin-γ-benzylester, $R_f$ = 0,46 (Chloroform/ Methanol = 9:1).

Auf analoge Weise erhält man N-Acetyl-muramyl-L-(O-behenoyl)-seryl-D-isoglutamin,

$[\alpha]_D^{20}$

= +29 ± 1° (c = 0,115; Wasser),
$R_f$ = 0,82 (Essigsäureäthylester/n-ButanoL/Pyridin/Essigsäure/Wasser = 42:21:21:6:10),
$R_f$ = 0,23 (Chloroform/Methanol/Wasser = 70:30:5).

Die Ausgangsmaterialien erhält man wie folgt:

10 g (21 mMol) N-Acetyl-1α-O-benzyl-4,6-0-isopropyliden-isomuraminsäure-natriumsalz (NaCl-haltig) werden in 100 ml eines Gemisches von 1,2-Dimethoxyäthan/Wasser = 2:1 gelöst und in Gegenwart von 5 g Palladium auf Kohle (10%ig) während 50 Stunden mit Wasserstoff behandelt. Der Katalysator wird abfiltriert, das Filtrat eingedampft und der Rückstand über Phosphorpentoxid getrocknet. Man erhält 8,1 g eines farblosen Pulvers mit einem Gehalt von 2,09 mMol N-Acetyl-4,6-0-isopropyliden-isomuraminsäure-natriumsalz pro Gramm (NaCl-haltig),

$R_f$ = 0,67(Essigsäureäthylester/n-Butanol/Pyridin/Essigsäure/Wasser = 42:21:21:6:10),

$R_f$ = 0,25 (Chloroform/Methanol/Wasser = 70:30:5).

Analog erhält man aus N-Acetyl-1α-0-benzyl-4,6-0-isopropyliden-mura-minsäure-natriumsalz das N-Acetyl-4,6-0-isopropyliden-muraminsäure-natriumsalz,

$R_f$ = 0,60 (Essigsäureäthylester/n-Butanol/Pyridin/Essigsäure/Wasser = 42:21:21:6:10),

Rf = 0,32 (Chloroform/Methanol/Wasser = 70:30:5), und schliesslich aus N-Acetyl-1α-0-benzyl-4,6-0-isopropyliden-nonnuraminsäure-natriumsalz das N-Acetyl-4,6-0-isopropyliden-normuraminsäure-natriumsalz (NaCl-haltig),

$R_f$ = 0,48 (Essigsäureäthylester/n-Butanol/Pyridin/Essigsäure/Wasser = 42:21:21:6:10),

$R_f$ = 0,22 (Chloroform/Methanol/Wasser = 70:30:5).

Das N-Acetyl-1α-0-benzyl-4,6-0-isopropyliden-isomuraminsäure-natriumsalz erhält man folgendermassen:

14.6 g (32,4 mMol) N-Acetyl-1α-0-benzyl-4,6-0-isopropyliden-isonnraminsäureäthylester werden in 150 ml Methanol gelöst, mit 24,3 ml 2N Natronlauge versetzt und während einer Stunde bei Raumtemperatur stehen gelassen. Die Reaktionslösung wird nach Zugabe von 8,1 ml 2N Salzsäure eingedampft und der Rückstand über Phosphorpentoxid getrocknet. Man erhält 15,53 g eines farblosen Pulvers mit einem Gehalt von 2,09 mMol N-Acetyl-1α-0-benzyl-4,6-0-isopropyliden-isomuraminsäure-natriumsalz pro Gramm (NaCl-haltig)

$R_f$ = 0,54 (Chloroform/Methanol/Essigsäure =

85:13:1,5:0,5), $R_f$ = 0,6 (Chloroform/Methanol = 85:15).

**Beispiel 8:**

3,02 g (5 mMol) N-Acetyl-4,6-0-isopropyliden-muramyl-D-seryl-D-isoglutamin-γ-tert.-butylester werden auf die übliche Weise mit 1,25 Aequivalenten Oelsäurechlorid (Fluka) in Pyridin umgesetzt. Nach 30stündigem Stehen bei Raumtemperatur werden 50 ml Methanol zugefügt, das Ganze während 2 Stunden gerührt und dann bei 30° eingedampft. Der Rückstand wird auf einer Säule, enthaltend 100 g $UPC_{12}$-Kieselgel (4 mal belegt; ANTEC) mit Chloroform von Nebenprodukten befreit und das Produkt mit Chloroform/Methanol = 95:5 eluiert (2,2 g).

1,1 g des in den Fraktionen 22-50 enthaltenen Materials werden in der Kälte in 8 ml 95%iger Trifluoressigsäure gelöst, die Lösung nach 2 stündigem Stehen bei 0° eingedampft, der Rückstand in 20 ml tert.-Butanol aufgenommen und lyophilisiert. Das Rohprodukt wird wie vordem an 100 g $UPC_{12}$-Kieselgel im System Chloroform/Isopropanol/Essigsäure = 70:8:2 (10 ml Fraktionen) gereinigt. Das in den Fraktionen 21-60 enthaltene N-Acetyl-muramyl-D-(O-oleoyl)-seryl-D-isoglutamin wird in 95%igem tert.Butanol aufgenommen, durch ein PTFE-Milliporfilter (0,2 μ) filtriert und lyophilisiert,

$$[\alpha]_D^{20}$$

= +26 ± 1° (c = 0,395, Methanol),
$R_f$ = 0,22 (Chloroform/Methanol/Wasser = 70:30:5),
$R_f$ = 0,62 (Acetonitril/Wasser = 3:1).

Das Ausgangsmaterial erhält man wie folgt:

5,08 g (12,3 mMol) Carbobenzoxy-D-seryl-D-isoglutamin-γ-tert.-butyl-ester, gelöst in 300 ml Methanol werden in Gegenwart von 1 g eines Palladium auf Kohle Katalysators (10%ig) während 30 Minuten mit Wasserstoff behandelt, wobei der pH-Wert durch Zugabe von methanolischer Salzsäure (0,7N) auf 3,5 gehalten wird. Der nach üblicher Aufarbeitung anfallende und über Natronasbest (Merck) getrocknete Schaum wird in 40 ml Dimethylformamid gelöst, 4,24 g (2,3 mMol) N-Acetyl-4,6-0-isopropyliden-muraminsäure-natriumsalz (2,9 mMol/g) und 3,34 g (13,5 mMol) EEDQ zugefügt und über Nacht bei Raumtemperatur gerührt. Die Suspension wird bei 30° eingedampft, der Rückstand zwischen 300 ml Essigsäureäthylester und 50 ml gesättigter Kaliumchloridlösung verteilt. Die wässrige Phase wird abgelassen und die organische Phase noch 3 mal mit je 50 ml gesättigter Kaliumchlorid-Lösung extrahiert. Die Essigesterphase wird getrocknet, auf ca. 30 ml eingeengt und durch portionemweise Zugabe von 150 ml Aether zur Kristallisation gebracht. Man erhält 5,4 g (73%) N-Acetyl-4,6-0-isopropyliden-muramyl-D-seryl-D-isoglutamin-γ-tert.-butylester,

$$[\alpha]_D^{20}$$

= +29° (c = 0,782, Methanol)
$R_f$ = 0,40 (Chloroform/Methanol/Wasser = 70:30:5),
$R_f$ = 0,60 (Acetonitril/Wasser = 3:1).

**Beispiel 9:**

Zu einer Lösung von 1,4 g (1,43 mMol) N-Acetyl-normuramyl-L-(O-behenoyl)-seryl-D-isoglutamin-γ-diphenylmethylester im 15 ml abs. Pyridin werden 0,48 g (4,72 mMol) Essigsäureanhydrid, gelöst in 15 ml Pyridin, eingetropft und das Ganze während einer Stunde bei Raumtemperatur stehen gelassen. Man gibt 1 ml Wasser zu und dampft nach 15 Minuten das Lösungsmittel ab. Der Rückstand wird in 60 ml Essigsäureäthylester aufgenonnen, die Essigesterphase 5 mal mit je 20 ml Wasser extrahiert und getrocknet. Der nach dem Verdampfen des Lösungsmittels verbleibende Rückstand (1,4 g) wird in 20 ml 1,2-Dimethoxyäthan/Wasser = 95:5 gelöst und während 30 Minuten in Gegenwart von Palladium auf Bariumsulfat (10%ig) mit Wasserstoff behandelt. Der Ratalysator wird abfiltriert, das Filtrat eingedampft und der Rückstamd aus tert.-Butanol/Wasser = 9:1 lyophilisiert. Das feste Material wird mehrfach mit Aether verrieben, wieder in 20 ml tert.-Butanol/ Wasser = 9:1 gelöst, durch ein PTFE-Milliporfilter (0,2 μ) filtriert und lyophilisiert. Es verbleiben 1,1 g N-Acetyl-1,4,6-0-triacetyl-normuramyl-L-(O-behenoyl)-seryl-D-isoglutamin

$$[\alpha]_D^{20}$$

= +33 ± 1°
(c = 0,53; Methanol),
$R_f$ = 0,38 (Chloroform/Methanol/Wasser = 70:30:5),
$R_f$ = 0,77 (Essigsäureäthylester/n-Butanol/Pyridim/Essigsäure/Wasser = 42:21:21:6:10).

Das Ausgangsmaterial erhält man wie folgt:

1,5 g (1,84 mMol) N-Acetyl-normuramyl-L-(O-behenoyl)-seryl-D-isoglutamin werden in 40 ml Methanol/1,2-Dimethoxyäthan = 2:1 gelöst, mit 0,7 g (3,7 mMol) Diphenyldiazomethan versetzt und während 3 Stunden bei Raumtemperatur gerührt. Die rote Suspension wird bei 30° eingedampft, der ölige Rückstand mit 200 ml Aether/Petroläther = 1:2 verrieben und das

Unlösliche abfiltriert. Der Niederschlag wird farblos gewaschen, in 50 ml Methanol gelöst und durch Zugabe von Aether zur Kristallisation gebracht. Nach Abfiltrieren, Waschen und Trocknen verbleiben 1,6 g (89%) N-Acetyl-nonnuramyl-L-(O-behenoyl)-seryl-D-isoglutamin-γ-diphenylmethylester als farblose Kristalle, Fp. 165° Zers., $R_f$= 0,5 (Chloroform/Methanol/Wasser = 70:30:5).

Analog erhält man aus N-Acetyl-normuramyl-L-seryl-D-isoglutamin-γ-diphenylmethylester das N-Acetyl-1,4,6-0-triacetyl-nonnuramyl-L-(O-acetyl)-seryl-D-isoglutamin.

**Beispiel 10:**

0,89 g (1 mMol) N-Acetyl-4,6-0-isopropyliden-muramyl-L(S-stearoyl)-cysteinyl-D-isoglutamin-tert.butylester werden in der Kälte in 10 ml 95%iger Trifluoressigsäure gelöst. Nach zweistündigem Stehen bei 0° wird vorsichtig eingeengt, der Rückstand mit tert.-Butanol versetzt und letzterer abgedampft (2 mal). Man nimmt in tert.-ButanoL/Wasser = 95:5 auf, filtriert durch ein Teflon-Millipor-Filter (0,2 μ) und lyophilisiert. Man erhält N-Acetyl-muramyl-L-(S-stearoyl)cysteinyl-D-isoglutamin als farbloses Pulver.

Das Ausgangsmaterial erhält man wie folgt:

Zu 1,29 g (1,5 mMol) N-Acetyl-4,6-0-isopropyliden-muramyl-L-(S-trityl)-cysteinyl-D-isoglutamin-γ-tert.butylester, gelöst in einer Mischung aus 10 ml Essigsäureäthylester und 5 ml Methanol, gibt man 0,526 g (1,65 mMol) Quecksilber-11-acetat (Merck). Nach vierstündigem Rühren bei Raumtemperatur wird das Mercaptid durch 10 minütiges Einleiten von Schwefelwasserstoff zersetzt. Das flockige, gut filtrierbare Quecksilbersulfid wird abgesaugt, das Filtrat eingedampft und die Mercaptogruppe nach Lösen in abs. Pyridin wie üblich mit 1,1 Aequivalenten Stearinsäurechlorid acyliert. Das Rohprodukt wird durch Chromatographie am Kieselgel gereinigt. Man erhält N-Acetyl-4,6-0-isopropylidenmuramyl-L-(S-stearoyl)-cysteinyl-D-isoglutamin-tert.butylester.

Das vollgeschützte Produkt erhält man auf folgende Weise:

1,74 g (2,2 mMol) N,S-Ditrityl-L-cysteinyl-D-isoglutamin-γ-tert.-butylester werden in 35 ml 90%igem Trifluoräthanol gelöst und bei Raumtemperatur mit 0,1 N HCl in 90%igem Trifluoräthanol auf pH = 3,5 (pH-stat) titriert (Verbrauch: 19,8 ml). Die dünne Suspension wird mit Essigsäureäthylester und tert.Butanol verdünnt und bei 30° im Vakuum stark eingeengt. Nach zweimaliger Zugabe von tert.Butanol und Verdampfen desselben wird wieder in tert.Butanol aufgenommen und lyophilisiert, $R_f$ = 0,66 (AcetonitriL/Wasser = 3:1).

Das oben erhaltene Rohprodukt wird in 6 ml Dimethylfonnamid aufgenommen, 0,78 g (2,2 mMol) N-Acetyl-4,6- 0-isopropylidenmuraminsäurenatriumsalz, 0,544 g (2,64 mMol) Dicyclohexylcarbodiimid und 0,506 g (4,4 mMol) N-Hydroxysuccinimid zugegeben. Man spült mit 2 ml Acetonitril nach und rührt das Ganze während 20 Stunden bei Raumtemperatur. Die Suspension wird mit dem vierfachen Volumen an Essigsäureäthylester verdünnt, das Unlösliche abfiltriert und das Filtrat bei 30° zur Trockne verdampft. Das Rohprodukt wird an Kieselgel (1:50) im System Chloroform/Isopropanol = 95:5 (10 ml Fraktionen) von den Hauptverunreinigungen befreit und mit Chloroform/Isopropanol = 85:15 eluiert. Man erhält 1,35 g 4,6-0-Isopropyliden-N-acetyl-muramyl-L-(S-trityl)-cysteinyl-D-isoglutamin-γ-tert.butylester,

$R_f$ = 0,57 (Acetonitril/Wasser = 3:1),

$R_f$ = 0,69 (Chloroform/Methanol/Wasser = 70:30:5).

Das geschützte Dipeptidderivat erhält man folgendermassen:

Zu 3,03 g (15 mMol) D-Isoglutami-n-γ-tert.butylester, gelöst in einer Mischumg aus 27 ml Dimethylformamid und 3 ml Acetonitril, werden 10 54 g (15 mMol) N,S-Ditrityl-L-cystein-succinimidester gegeben und das Ganze während 29 Stunden bei Raumtemperatur stehen gelassen. Die gelbliche Lösung wird mit 300 ml Essigsäureäthylester verdünnt und 10 mal mit je 30 ml Wasser extrahiert. Die organische Phase wird getrocknet und das Lösungsmittel verdampft. Der farblose Rückstand wird in 35 ml heissem Aceton gelöst, 1 ml Methanol und unter Rühren 5 ml Wasser zugegeben. Das Produkt fällt beim Abkühlen in farblosen Nadelbüscheln aus. Man erhält 9,7 g (82%) N,S-Ditrityl-L-cysteinyl-D-isoglutamin-γ-tert.-butylester, Zersetzungsbereich 206-207°,

$$[\alpha]_D^{20}$$

= +58 ± 1° (c = 1,275; Chloroform),

$R_f$ = 0,78 (Acetonitril/Wasser = 3:1),

$R_f$ = 0,65 (Chloroform/Isopropanol/Essigsäure = 70:8:2).

**Beispiel 11:**

1,09 g (1 mMol) N-Benzyloxycarbonyl-1α-0-benzyl-4,6-isopropyliden-normuramyl-L-(O-behenoyl)-seryl-D-isoglutamin-tert.butylester hydriert man mit 0,5 g Palladi-um-auf-Bariumsulfat (10%ig) in 10 ml 5%igem wässrigem Dioxan unter pH-Kontrolle bei pH = 7, Raumtemperatur und Normaldruck etwa 1 Stunde. Man filtriert den Katalysator ab und acetyliert die freie Aminogruppe der Normuraminsäure unter Zugabe von 0,3 ml Acetanhydrid und 6 ml 5%iger $NaHCO_3$-Lösung. Nach 2 Stunden bei Raumtemperatur engt man

die Lösung im Vakuum ein und versetzt den Rückstand mit 20 ml 2N HCl in Essigester. Nach einstündigem Rühren bei Raumtemperatur fällt man das entstandene amorphe N-Acetyl-1α-0-benzyl-normuramyl-L-(O-behenoyl)-seryl-D-isoglutamin mit 50 ml Aether aus, $R_f$ = 0,43 ($CHCl_3$/Methanol/$H_2O$ = 70:30:5). Man hydriert diese Verbindung miit 0,5 g Pd/$BaSO_4$ in 15 ml 5%igem wässrigem Dioxan während 60 Stunden.

Nach Abfiltration des Katalysators dampft man im Vakuum zur Trockne, chromatographiert an Kieselgel (Merck) in $CHCl_3$/Methanol = 7:3 und entsalzt die reinen Fraktionen mit Dowex-50 H+ in Dimethoxyäthan/$H_2O$ = 1:1. Nach Eindampfen, Lösen in tert.Butanol, Filtration durch Milliporefilter (0,2μ) und Gefriertrocknen des Filtrats erhält man N-Acetyl-normuramyl-L-(O-behenoyl)-seryl-D-isoglutamin als farbloses, amorphes Pulver, $R_f$ = 0,23 (Chloroform/Methanol/Wasser = 70:30:5), $R_f$ = 0.59 (Acetonitril/Wasser = 3:1), $R_f$ = 0,39 (Essigsäureäthylester/ n-Butanol/Pyridin/Eisessig/Wasser = 42:21:21:6:10).

Das Ausgangsmaterial erhält man wie folgt:

Analog Beispiel 7 kondensiert man 1,0 g (2 mMol) N-Benzyloxycarbonyl-1α -O-benzyl-4,6-0-isopropyliden-normuraminsäure-Na-Salz mit 1,3 g (2 mMol) L-(O-Behenoyl)-seryl-D-isoglutamin-γ-tert.-butylester-hydrochlorid, 0,6 g N-Hydroxysuccinimid und 0,65 g Dicyclohexylcarbodiimid in 25 ml abs. Dimethylformamid. Nach 24 Stunden bei Raumtemperatur saugt man vom Dicyclohexylharnstoff ab, dampft am Oelvakuum zur Trockne und chromatographiert den Rückstand an Kieselgel Merck mit $CHCl_3$:Methanol = 9:1. Man erhält so N-Benzyloxycarbonyl-1α-O-benzyl-4,6-0-isopropyliden-normuramyl-L-(O-behenoyl)-seryl-D-isoglutamin-γ-tert.-butylester mit

$[\alpha]_D^{20}$

= +75° (c = 1,Dimethylformamid), $R_f$ = 0,55 ($CHCl_3$:Methanol = 9:1).

**Beispiel 12:**

3,5 g (3,85 mMol) N-Acetyl-1α-O-benzyl-4,6-0-isopropyliden-normuramyl-L-(O-behenoyl)-serinbenzylester, gelöst in 50 ml 1,2-Dimethoxyäthan/Wasser = 95:5, werden nach Zugabe von 2 g Pd auf Bariumsulfat-Ratalysator während 3 Stunden mit Wasserstoff behandelt. Der nach dem Eindampfen und Trocknen verbleibende Rückstand wird in 12 ml Dimethylformamid/Chloroform = 3:1 gelöst. Man fügt in der Kälte 0,78 g (3,85 mMol) D-Isoglutamin-γ-tert.butylester, 0,531 g (4,63 mMol) N-Hydroxysuccinimid und schliesslich 0,952 g (4,62 mMol) Dicyclohexylcarbodiimid zu und rührt das Ganze während 24 Stunden bei Raumtemperatur. Die Suspension wird mit 0,5 ml Essigsäure versetzt, nach 30 Minuten mit 60 ml Essigsäureäthylester verdünnt und der Niederschlag abgesaugt. Das Filtrat wird analog Beispiel 2 aufgearbeitet und das Rohprodukt zusätzlich an Kieselgel (1:30) im System Chloroform/Isopropanol = 95:5 gereinigt. Man erhält 2,6 g N-Acetyl-1α-O-benzyl-4,6-0-isopropyliden-normuramyl-L-(O-behenoyl)-seryl-D-isoglutamin-γ-tert.butylester

$R_f$ = 0,85 (Chloroform/Methanol/Wasser = 70:30:5),

$R_f$ = 0,43 (Chloroform/Isopropanol/Essigsäure = 70:8:2).

Das Ausgangsmaterial erhält man wie folgt:

3,52 g (6 mMol) N-Acetyl-1α-0-benzyl-4,6-0-isopropyliden-normuramyl-L-serinbenzylester werden mit Behensäurechlorid in abs. Pyridin analog Beispiel 2 acyliert. Man erhält 3,93 g (72%) N-Acetyl-1α-0-benzyl-4,6-O-isopropyliden-normuramyl-L-(O-behenoyl)-serinbenzylester,

$R_f$ = 0,85 (Acetonitril/Wasser = 3:1)

$R_f$ = 0,35 (Essigsäureäthylester.

Die Ausgangsverbindung erhält man wie folgt:

4,24 g (12,3 mMol) N-Acetyl-1α-O-benzyl-4,6-0-isopropyliden-normuraminsäure-natriumsalz, 2,85 g (12,3 mMol) L-Serinbenzylester-hydrochlorid und 3,34 g (13,5 mMol) EEDQ in Dimethylformamid werden analog Beispiel 8 umgesetzt. Das Lösungsmittel wird im Vakuum entfernt, der Rückstand in 150 ml Essigsäureäthylester aufgenommen und in der Kälte erst mit je 15 ml Wasser (2 mal), dann mit kalter IN HCl (4 mal) gesättigter Natriumhydrogencarbonatlösung und wieder Wasser (2 mal) extrahiert. Nach dem Verdampfen des Lösungsmittels erhält man 5 g (70%) N-Acetyl-1α-0-benzyl-4,6-0-isopropyliden-normuramyl-L-serinbenzylester als farblosen Schaum,

$[\alpha]_D^{20}$ = +75° (c = 1; Chloroform),

$R_f$ = 0,6 (Chloroform/Methanol = 15:1),

$R_f$ = 0,5 (Essigsäureäthylester).

**Beispiel 13:**

Ausgehend vom bekannten 2-Acetamino-1α-0-benzyl-2-deoxy-5,6-0-isopropyliden-3-0-(D-1-carboxy-äthyl)-D-mannofuranosid [Agric. Biol. Chem. 42, 2187 (1978)] erhält man durch Kupplung mit Peptid analog Beispiel 6 und übliche Abspaltung der Schutzgruppen 2-(2-Acetami-no-2-deoxy-D-mannos-3-0-yl)-D=propionyl-L-[O-behenoyl]-seryl-D-isoglutamin.

**Beispiel 14:**

Man hydriert analog Beispiel 7 N-Benzyloxycarbonyl-1α-O-benzyl-muramyl-L-O-benzoyl-seryl-D-isoglutamin-benzylester bei pH = 7 und erhält so Muramyl-L-O-benzoyl-seryl-D-isoglutamin als inneres Salz.
Diese Verbindung kann durch Umsetzen mit Hydroxy-succinimidester von Alkyl- und Arylcarbonsäuren unter Zusatz von Triäthylamin oderNa$HCO_3$-Lösung in entsprechende N-acylierte Muramylpeptide verwandelt werden (vgl. Beispiel 11): Man erhält so N-(p-Chlorbenzoyl)-muramyl-L-(O-behenoyl)-seryl-D-isoglutamin, N-Pivaloyl-muramyl-L-(O-behenoyl)seryl-D-isoglutamin und N-Octanoyl-muramyl-L-(O-behenoyl)-seryl-D-isoglutamin.

**Beispiel 15:**

Analog Beispiel 5 kuppelt man 2-(1,2;5,6-Diisopropyliden-glucofuranos-3-0-yl)-D-propionsäure (Carbohydrate Research 79, C 17 [1980]) mit Peptid, spaltet mit verdünnter Säure und durch katalytische Hydrierung die Schutzgruppen ab und erhält 2-(Glucopyranos-3-0-yl)-D-propionyl-L-(O-dodecyloxycarbonyl)-seryl-D-isoglutamin.
Analog erhält man aus 2-(1,2;5,6-Diisopropyliden-galaktofuranos-3-0-yl)-D-propionsäure 2-(Galaktopyranos-3-0-yl)-D-propionyl-L-(O-dodecyloxycarbonyl)-seryl-D-isoglutamin.

**Beispiel 16:**

Aus N-Acetyl-normuramyl-L-(O-behenoyl)-seryl-D-isoglutamin erhält man mit N,O-bis-Trimethylsilyl-carbamat in absolutem Dimethylformamid bei Raumtemperatur N-Acetyl-1,4,6-0-tris-trimethyl-silyl-normuramyl-L-(O-behenoyl)-seryl-D-isoglutamin-trimethylsilylester.

**Beispiel 17:**

Analog Beispiel 9 erhält man N-Acetyl-1,4,6-0-triacetyl-muramyl-L-(N-methyl-O-behenoyl)-seryl-D-isoglutamin.

**Beispiel 18:**

Die Azidogruppe in 1-O-Benzyl-2-acetylamino-6-azido-2,6-didesoxy-3-0-(D-1-methoxycarbonyl-äthyl)-α-D-glucopyranosid

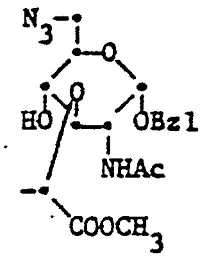

wird zu Aminogruppe reduziert, acetyliert und nach Verseifung des Methylesters analog Beispiel 7 mit L-Seryl-D-isoglutamin-γ-tert.-butyl-ester verknüpft. Nach Acylieren der Hydroxygruppe des Serins und Entfernen der Schutzgruppen erhält man N-Acetyl-6-acetylamino-6-deoxy-muramyl-L-(O-dodecanoyl)-seryl-D-isoglutamin.

**Beispiel 19:**

2-Acetylamino-1α-0-benzyl-2-deoxy-4,6-0-isopropylidenD-glucose wird mit α-Brom-isobuttersäuremethylester veräthert. Nach Verseifen des Esters, Kupplung mit L-Seryl-D-isoglutamin-γ-benzylester-hydrochlorid und Acylieren mit Stearinsäurechlorid und Abspaltung der Schutzgruppen erhält man 2-(2-Acetylamino-2-deoxy-D-glucos-3-0-yl)-2,2-di-methylacetyl-L-O-stearoyl-seryl:D-isoglutamin.

**Beispiel 20:**

N-Acetyl-4,6-0-isopropyliden-muraminsäure-natriumsalz wird analog Beispiel 7 mitL-(O-Behenoyl)-seryl-D-γ-carboxyglutamin-säure-γ,γ'-di-tert.-butylester-α-glycinamid gekuppelt. Nach Entfernung der Schutzgruppen erhält man N-Acetyl-muramyl-L-(O-behenoyl)-seryl-D-γ-carboxy-α-glutamyl-glycinamid.

**Beispiel 21:**

Analog Beispiel 7 erhält man aus N-Acetyl-1α-O-benzyl-4,6-0-isopropyliden-normuraminsäure-natriumsalz und L-(O-Palmitoyl)-α-methyl-seryl-D-isoglutamin-γ-tert.-butylester nach Abspalten der Schutzgruppen N-Acetyl-normuramyl-L-(O-palmitoyl)-α-methyl-seryl-D-iso-glutamin.

**Beispiel 22:**

Der in Beispiel 12 beschriebene N-Acetyl-1α-O-benzyl-4,6-0-isopropyliden-normuramyl-L-serinbenzylester wird hydriert und amalog Beispiel 12 mit den folgenden Derivaten

verknüpft: D-γ-Carboxy-glutaminsäure-γ,γ'-dimethylester-α-amid beziehungsweise D-γ-Carboxy-glutaminsäure-γ'-methylester-α,γ-diamid.

Nach Acylierung der β-Hydroxygruppe des Serins und Entfernung der Schutzgruppen erhält man N-Acetyl-normuramyl-L-(O-behenoyl)-seryl-D-γ-carboxy-glutaminsäure-γ,γ'-dimethylester-α-amid beziehungsweise N-Acetyl-normuramyl-L-(O-behenoyl)-seryl-D-γ-carboxy-glutaminsäure-γ-methylester-α,γ-diamid.

**Beispiel 23:**

Herstellung von 1000 Kapseln mit 260 mg der aktiven Ingredienzien pro Kapsel:

**Zusammensetzung:**

Rifampicin 250 g
N-Acetyl-normuramyl-L-O-behenoyl-seryl-D-isoglutamin 10 g
Talk 36 g
Weizenstärke 24 g
Magnesiumstearat 16 g
Laktose 4 g
340 g

**Zubereitung:**

Die pulverförmigen Substanzen werden durch ein Sieb mit einer Maschenweite von 0,6 mm geschlagen und gründlich gemischt. Mit je 340 g dieser Mischung werden mit einer Kapselfüllmaschine Gelatine-Kapseln bereitet.

**Beispiel 24:**

Analog Beispiel 7 erhält man aus N-Acetyl-4,6-0-isopropyli-den-muramimsäure-natriumsalz und L-O-Behenoyl-4-hydroxyprolyl-D-iso-glutaminyl-L-alaninbenzylester-hydrochlorid nach üblichem Abspalten der Schutzgruppen N-Acetyl-muramyl-L-(O-behenoyl)-4-hydroxyprolyl-D-isoglutaminyl-L-alanin.

**Beispiel 25:**

N-Acetyl-4,6-0-isopropyliden-muraminsäure-matriumsalz und N$^{\varepsilon}$-tert.Butyloxycarbonyl-L-lysyl-D-glutaminsäuredimethylesterhydrochlorid werden analog Beispiel 7 verknüpft. Nach acidolytischer Abspaltung der Schutzgruppen und Acylierung mit Palmitinsäure-N-hydroxysuccinimidester erhält man: N-Acetyl-muramyl-L-(N$^{\varepsilon}$-palmitoyl)-lysyl-D-glutaminsäure-dimethylester.

**Patentansprüche** für die Vertragsstaaten BE, CH, DE, FR, IT, LI, LU, NL, SE

1. Derivate von Pyranosen der Formel 1,

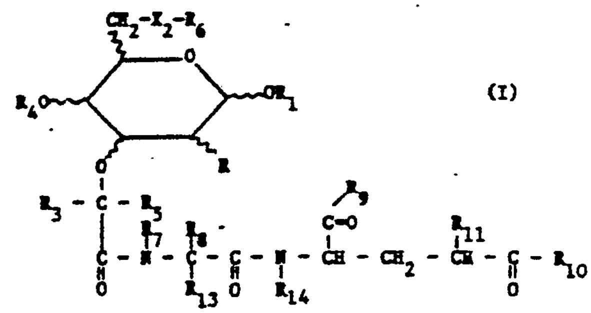

(I)

worin R für Hydroxy, Amino oder einen Rest der Formel

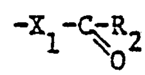

steht, $X_1$ und $X_2$ unabhängig voneinander für $NR_{15}$ oder ein Sauerstoffatom stehen, wobei $R_{15}$ Wasserstoff oder Niederalkyl bedeutet, $R_1$ und $R_4$ unabhängig voneinander für Wasserstoff, Acyl oder eine Hydroxyschutzgruppe, $R_6$ für Wasserstoff, Acyl oder, falls $X_2$ ein Sauerstoffatom bedeutet, für eine Hydroxyschutzgruppe stehen, $R_2$ jeweils gegebenenfalls substituiertes Alkyl, Aryl oder Alkoxy darstellt, $R_3$, $R_5$, $R_7$, $R_{13}$ und $R_{14}$ unabhängig voneinander für Wasserstoffoder Niederalkyl stehen, und $R_8$ einen Niederalkyl- oder Phenylniederalkylrest darstellt, der auch mit $R_7$ verbunden sein kann, und der eine Oxycarbonyl-, Mercaptocarbonyl- oder Aminocarbonylgruppe trägt, welche mit einem gegebenenfalls substituierten aliphatischen, cycloaliphatisch-aliphatischen, cycloaliphatischen oder aromatischen Kohlenwasserstoffrest $R_o$ verbunden ist, welch letzterer durch Oxycarbonyl, Mercaptocarbomyl und/oder Iminocarbonyl unterbrochen sein kann und der mehr als 5 C-Atome aufweisen muss, wenn $R_1$, $R_4$ und $R_6$ Wasserstoff bedeuten, worin die Reste $R_9$ und $R_{10}$ für gegebenenfalls veräthertes Hydroxy oder gegebenenfalls substituiertes Amino stehen, und $R_{11}$ Wasserstoff oder einen Rest der Formel -C(=O)-$R_{12}$ (Ia) bedeutet, worin $R_{12}$ für gegebenenfalls veräthertes Hydroxy oder gegebenenfalls substituiertes Amino steht, wobei

mit "nieder" bezeichnete Reste bis und mit 7 Kohlenstoffatom enthalten, und Salze von Verbindungen der Formel I mit mindestens einer salzbildenden Gruppe.

2. Verbindungen gemäss Anspruch 1, deren Pyranoseteil sich von der D-Glucose ableitet.

3. Verbindungen gemäss Anspruch 1, deren Pyranoseteil sich von der D-Mannose oder der D-Calaktose ableitet.

4. Verbindungen der Formel 1 nach einem der Ansprüche 1-3, die in Fällen asymmetrischer Substitution am C-$NR_{14}$ die (D)-Konfiguration aufweisen.

5. Verbindungen der Formel 1 nach Anspruch 4, die in Fällen asymmetrischer Substitution am C-$R_3$ die (D)- und am C-$R_8$ die (L)-Konfiguration aufweisen.

6. Verbindungen der Formel 1 nach einem der Ansprüche 1-5, worin R für einen Rest der Formel

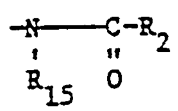

und $X_2$ für ein Sauerstoffatom stehen.

7. Verbindungen der Formel I nach einem der Ansprüche 1-6, worin $R_5$, $R_{13}$, $R_{14}$ und $R_{15}$ für Wasserstoff stehen.

8. Verbindungen der Formel I nach einem der Ansprüche 1-7, worin $R_1$, $R_4$ und $R_6$ für Wasserstoff, gegebenenfalls ungesättigtes Alkanoyl mit 2-25 C-Atomen oder für gegebenenfalls substituiertes Benzoyl stehen.

9. Verbindungen der Formel I nach einem der Ansprüche 1-7, worin $R_6$ für den Rest

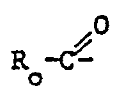

steht.

10. Peptidderivate gemäss Anspruch 1 von Glucosaminverbindungen der Formel

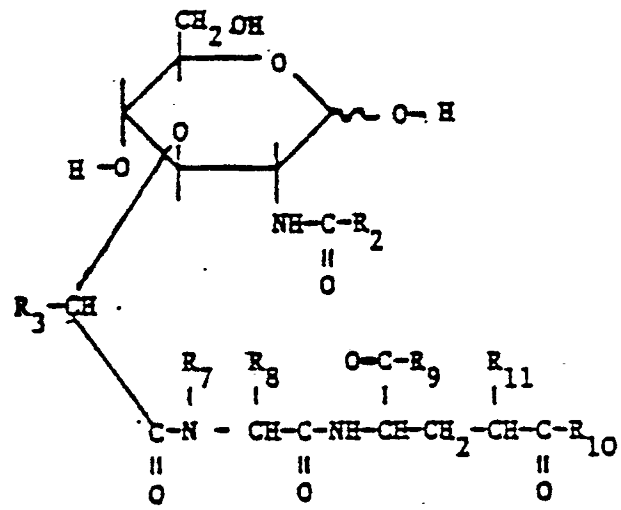

(Ic)

worin $R_2$ gegebenenfalls substituiertes Niederalkyl, Phenyl, Niederalkoxy oder Phenylniederalkoxy darstellt, $R_3$ für Wasserstoff oder Niederalkyl steht, $R_7$ Wasserstoff oder Niederalkyl bedeutet, und $R_8$ einen Niederalkyl- oder Phenylniederalkylrest darstellt, der auch mit $R_7$ verbunden sein kann, und der eine Oxycarbonyl-, Mercaptocarbonyloder Aminocarbonylgruppe trägt, welche mit einem gegebenenfalls substituierten, langkettigen aliphatischen oder cycloaliphatisch-aliphatischen Kohlenwasserstoffrest verbunden ist, welch letzterer durch Oxycarbonyl, Mercaptocarbonyl und/oder Iminocarbonyl unterbrochen sein kann, worin $R_9$ und $R_{10}$ für gegebenenfalls veräthertes Hydroxy oder gegebenenfalls substituiertes Amino stehen, und $R_{11}$ Wasserstoff oder einen Rest der Formel -C(=O)-$R_{12}$ (Ia) bedeutet, worin $R_{12}$ für gegebenenfalls veräthertes Hydroxy oder gegebenenfalls substituiertes Amino steht, und Salze von Verbindungen der Formel Ic mit mindestens einer salzbildenden Gruppe.

11. Verbindungen der Formel Ic gemäss Patentanspruch 10, dadurch gekennzeichnet, dass der Rest der Hydroxyessigsäure mit der Gruppierung der Formel -CH($R_3$)-, worin $R_3$ Niederalkyl darstellt, in der D-Form, der Rest der Aminoessigsäure mit der Gruppierung der Formel -CH($R_8$)-, in der L-Form, und der Rest der terminalen α-Amino-glutarsäureverbindung in der D-Form vorliegen, oder Salze von solchen Verbindungen mit salzbildenden Gruppen.

12. Verbindungen der Formel Ic, gemäss Patentanspruch 10, worin $R_2$ für gegebenenfalls durch Hydroxy, Niederalkoxy, Niederalkanoyloxy oder Halogen substituiertes Niederalkyl oder Niederalkoxy, oder gegebenenfalls durch Niederalkyl, Hydroxy, Niederalkoxy, Niederalkanoyloxy oder Halogen substituiertes Phenyl oder Phenylniederalkoxy steht, $R_3$ Wasserstoff oder Niederalkyl bedeutet, $R_7$ Wasserstoff oder Niederalkyl bedeutet, und $R_8$ einen Niederalkyl- oder Phenylniederalkylrest

darstellt, der als Substituenten eine Oxycarbonyl-, Mercaptocarbonyl- oder Aminocarbonylgruppe trägt, welche ihrerseits wiederum mit einem gegebenenfalls durch Hydroxy, Niederalkoxy, Niederalkanoyloxy, Oxo oder Halogen substituierten Alkyl- oder Alkenylrest mit mehr als 6 und bis zu 90 Kohlenstoffatomen oder einem cycloaliphatisch substituierten Alkyl- oder Alkenylrest mit z.B. bis zu 30 Kohlenstoffatomen verbunden ist, und worin die Alkyl-, Alkenyl- und cycloaliphatisch substituierten Alkyl- bzw. Alkenylreste auch durch eine oder zwei Oxycarbonyl- oder Iminocarbonyl-Gruppen unterbrochen sein können, $R_{11}$ Wasserstoff oder den Rest der Formel Ia bedeutet, und jeder der Reste $R_9$, $R_{10}$ und $R_{12}$ Hydroxy, Niederalkoxy, Amino oder durch, gegebenenfalls Carboxy, Niederalkoxycarbonyl oder Carbamoylenthaltendes Niederalkyl substituiertes Amino darstellt, wobei in solchen Verbindungen z.B. der Rest der Hydroxyessigsäure mit der Gruppierung der Formel -CH($R_3$)-, worin $R_3$ Niederalkyl darstellt, in der D-Form, der Rest der Aminoessigsäure mit der Gruppierung der Formel -CH($R_8$)-, in der L-Form, und der Rest der terminalen α-Amino-glutarsäureverbindung in der D-Form vorliegen, sowie Salze von solchen Verbindungen mit salzbildenden Gruppen.

13. Verbindungen der Formel Ic gemäss Patentanspruch 10, worin $R_2$ Niederalkyl mit bis zu 4 Kohlenstoffatomen oder Phenyl darstellt, $R_3$ Wasserstoff oder Niederalkyl mit bis zu 4 Kohlenstoffatomen bedeutet, $R_7$ Wasserstoff darstellt und $R_8$ einen Niederalkylrest mit 1-4 Kohlenstoffatomen oder einen Phenylniederalkylrest darstellt, der als Substituenten eine Oxycarbonyl-, Mercaptocarbonyl- oder Aminocarbonylgruppe trägt, welche ihrerseits mit einem gegebenenfalls durch Hydroxy, Niederalkoxy, Oxo oder Halogen substituierten Alkyl oder Alkenyl mit mehr als 10 und bis zu 50 Kohlenstoffatomen oder einem gegebenenfalls durch Hydroxy, Niederalkoxy, Oxo oder Halogen substituierten tetracyclischen Cycloalkyl-alkyl oder -alkenyl mit mehr als 20 und bis zu 50 Kohlenstoffatomen verbunden ist, wobei solche Reste auch durch 1 oder 2 Oxycarbonyl oder Iminocarbonylgruppen unterbrochen sein können, $R_9$ für Amino oder gegebenenfalls Carboxy oder Carbamoyl enthaltendes Niederalkylamino steht, $R_{10}$ Hydroxy bedeutet, und $R_{11}$ Wasserstoff darstellt, wobei in solchen Verbindungen z.B. der Rest der Hydroxyessigsäure mit der Gruppierung der Formel -CH($R_3$)-, worin $R_3$ Niederalkyl darstellt, in der D-Form, der Rest der Aminoessigsäure mit der Gruppierung der Formel -CH($R_8$)- in der L-Form, und der Rest der terminalen α-Amino-glutarsäureverbindung in der D-Form vorliegen, und pharmazeutisch verwendbare Salze von solchen Verbindungen mit salzbildenden Gruppen.

14. Verbindungen der Formel Ic gemäss Patentanspruch 10, worin $R_2$ Niederalkyl mit bis zu 4 Kohlenstoffatomen, oder Phenyl bedeutet, $R_3$ insbesondere Wasserstoff, ferner Niederalkyl mit bis zu 4 Kohlenstoffatomen, bedeutet, $R_7$ Wasserstoff darstellt, $R_8$ Niederalkyl mit 1-4 Kohlenstoffatomen oder Benzyl darstellt, die als Substituenten eine Oxycarbonyl-, Mercaptocarbonyl- oder Aminocarbonylgruppe tragen, welche vorzugsweise über das Heteroatom mit dem Niederalkyl- bzw. Benzylrest verbunden ist, und die ihrerseits mit einem gegebenenfalls durch Hydroxy, Niederalkoxy oder Oxo substituierten Alkyl oder Alkenyl mit mehr als 10 und bis zu 50 Kohlenstoffatomen oder einem gegebenenfalls durch Hydroxy oder Niederalkoxy substituierten tetracyclischen Cycloalkyl-alkyl- oder Cycloalkenyl-alkylrest mit mehr als 20 und bis zu 50 Kohlenstoffatomen verbunden ist, welche Reste auch durch eine Oxycarbonyl- oder Iminocarbonylgruppe unterbrochen sein können, $R_9$ Amino bedeutet, $R_{10}$ für Hydroxy steht, und $R_{11}$ Wasserstoff darstellt, wobei in solchen Verbindungen der Rest der Hydroxyessigsäure mit der Gruppierung der Formel -CH($R_3$)- worin $R_3$ Niederalkyl darstellt, in der D-Form, der Rest der Aminoessigsäure mit der Gruppierung der Formel -CH($R_8$)- in der L-Form, und dei Rest der terminalen α-Amino-glutarsäureverbindung in der D-Form vorliegen, und pharmazeutisch verwendbare Salze von solchen Verbindungen mit salzbildenden Gruppen.

15. Verbindungen der Formel Ic gemäss Patentanspruch 14, worin $R_2$ Methyl oder Phenyl, $R_3$ Wasserstoff oder Methyl bedeuten und die übrigen Reste die in Patentanspruch 14 gegebenen Bedeutungen haben.

16. Verbindungen der Formel Ic gemäss Patentanspruch 10, worin $R_2$ Niederalkyl mit bis zu 3 Kohlenstoffatomen oder Phenyl, $R_3$ Wasserstoff oder Methyl, $R_7$ Wasserstoff, $R_8$ Niederalkyl mit 1-3 Kohlenstoffatomen, das als Substituenten eine Oxycarbonyl-, Mercaptocarbonyl-oder Aminocarbonylgruppe trägt, welche über das Heteroatom mit dem Niederalkylrest verbunden ist und die ihrerseits mit einem gegebenenfalls durch Hydroxy, Niederalkoxy oder Oxo substituierten Alkyl oder Alkenyl mit mehr als 10 und bis zu 50 Kohlenstoffatomen oder einem gegebenenfalls durch Hydroxy oder Niederalkoxy substituierten tetracyclischen Cycloalkyl-alkyl oder Cycloalkenyl-alkyl-rest mit mehr als 20 und bis zu 50 Kohlenstoffatomen verbunden ist, welche Reste auch durch Oxycarbonyl- oder Iminocarbonyl unterbrochen sein können, $R_9$ Amino, Niederalkylamino oder Carbamoylniederalkylamino oder Hydroxy, $R_{10}$ Hydroxy, Niederalkoxy, Amino oder Carbamoylniederalkylamino, und $R_{11}$ Wasserstoff bedeuten, wobei in solchen Verbindungen der Rest der Hydroxyessigsäure mit der Gruppierung -CH($R_3$)-, worin $R_3$ Methyl darstellt, in der D-Form, der Rest der Aminosäure mit der Gruppierung -CH($R_8$)-in der L-Form, und der Rest der terminalen α-Amino-glutarsäureverbindung in der D-Form

vorliegen, und pharmazeutisch verwendbare Salze von solchen Verbindungen mit salzbildenden Gruppen.

17. Verbindungen der Formel Ic gemäss Patentanspruch 11, worin $R_2$ Niederalkyl mit bis zu 3 Kohlenstoffatomen, $R_3$ Wasserstoff oder Methyl, $R_7$ Wasserstoff, $R_8$ Niederalkyl mit 1-4 C-Atomen, das als Substituenten eine Oxycarbonyl-, Mercaptocarbonyl- oder Aminocarbonylgruppe trägt, welche über das Heteroatom mit dem Niederalkylrest verbunden ist und die ihrerseits mit Alkyl mit mehr als 10 und bis zu 50 Kohlenstoffatomen verbunden ist, das auch durch Iminocarbonyl unterbrochen sein kann, $R_9$ Amino, Hydroxy oder Niederalkoxy mit 1-3 C-Atomen, $R_{10}$ Hydroxy, Niederalkoxy mit 1-3 C-Atomen oder Amino und $R_{11}$ Wasserstoff oder Carboxy bedeuten, und pharmazeutisch verwendbare Salze von solchen Verbindungen mit salzbildenden Gruppen.

18. Verbindungen der Formel Ic gemäss Anspruch 17, worin $R_8$ Alkanoyloxymethyl oder Alkanoyloxyäthyl mit mehr als 10 und bis zu 30 C-Atomen im Alkylteil des Alkanoylrestes, $R_9$ Amino und $R_{10}$ Hydroxy bedeuten, und ihre pharmazeutisch verwendbaren Salze.

19. N-Acetyl-normuramyl-L-O-(N-behenoyl-glycyl)-seryl-D-isoglutamin oder ein pharmazeutisch verwendbares Salz davon nach Anspruch 1.

20. N-Acetyl-normuramyl-L-O-behenoyl-seryl-D-isoglutamin oder ein pharmazeutisch verwendbares Salz davon nach Anspruch 1.

21. N-Acetyl-muramyl-L-O-[N-(D,L-2-n-hexadecanoyl-amino-n-hexadecanoyl) -L-alanyl]-threonyl-D-isoglutamin oder ein pharmazeutisch verwendbares Salz davon nach Anspruch 1.

22. N-Acetyl-muramyl-L-S-stearoyl-cysteinyl-D-isoglutamin oder ein pharmazeutisch verwendbares Salz davon nach Anspruch 1.

23. Eine Verbindung nach Anspruch 1, ausgewählt aus der Gruppe der folgenden Verbindungen und ihrer Salze:

N-Acetyl-normuramyl-L-O-[N-(12-hydroxy-cis-9-octadecenoyl)-glycyl]-seryl-D-isoglutamin,

N-Benzoyl-normuramyl-L-O-stearoyl-seryl-D-isoglutamin,

N-Acetyl-muramyl-L-O-(ω-n-stearoylamino-n-undecanoyl)-threonyl-D-isoglutamin

N-Acetyl-muramyl-L-O-[N-(3-hydroxy-ätio-cholenoyl)-6-amino-hexanoyl]-γ-hydroxyprolyl-D-isoglutamin,

N-Acetyl-muramyl-L-O-behenoyl-tyrosyl-D-isoglutamin,

N-Acetyl-muramyl-L-($C_\gamma$)-[tetracosylamido-glycyl]-glutamyl-D -isoglutamin,

N-Acetyl-muramyl-L-[($C_\gamma$)-lauryl]-α-glutamyl-D-isoglutamin,

N-Acetyl-normuramyl-L-γ-stearoylamino-α-amino-butanoyl-D-isoglutamin,

N-Acetyl-muramyl-L-(O-behenoyl-N-methyl)-seryl-D-isoglutamin,

N-Acetyl-muramyl-L-(Nε -palmitoyl)-lysyl-D-glutaminsäure-dimethylester.

24. N-Benzoyl-normuramyl-L-(O-stearoyl)-seryl-D-glutaminsäuredimethylester nach Anspruch 1.

25. N-Benzoyl-normuramyl-L-(O-behenoyl)-seryl-D-isoglutamin oder ein pharmazeutisch verwendbares Salz davon nach Anspruch 1.

26. N-Acetyl-isomuramyl-L-(O-behenoyl)-seryl-D-isoglutamin oder ein pharmazeutisch verwendbares Salz davon nach Anspruch 1.

27. N-Acetyl-muramyl-L-(O-behenoyl)-seryl-D-isoglutamin oder ein pharmazeutisch verwendbares Salz davon nach Anspruch 1.

28. Eine Verbindung nach Anspruch 1 ausgewählt aus der Gruppe der folgenden Verbindungen und ihrer Salze:

N-Acetyl-muramyl-D-(O-oleoyl)-seryl-D-isoglutamin,

N-Acetyl-1,4,6-0-triacetyl-normuramyl-L-(O-behenoyl)-seryl-D-isoglutamin,

N-Acetyl-muramyl-L-(4-behenoyloxy)-prolyl-D-isoglutaminyl-γ-L-alanin und

N-Acetyl-muramyl-L-(O-behenoyl)-seryl-D-γ-carboxy-α-glutamyl-glycinamid.

29. Verbindungen der Formel I und ihre Salze gemäss einem der Ansprüche 1-28 zur Anwendung in einem Verfahren zur therapeutischen Behandlung des menschlichen oder tierischen Korpers.

30. Die immunpotenzierenden Verbindungen der Formel Ic gemäss einem der Ansprüche 10-22 und Salze von solchen Verbindungen mit salzbildenden Gruppen.

31. Verbindungen der Formel I gemäss einem der Ansprüche 1-28 als immunomodulierende Mittel.

32. Pharmazeutisch verwendbare Präparate enthaltend Verbindungen der Formel Ic gemäss einem der Ansprüche 10-22 oder pharmazeutisch verwendbare Salze von solchen Verbindungen mit salzbildenden Gruppen.

33. Pharmazeutische Präparate, die pharmakologisch wirksame Verbindungen der Formel I gemäss einem der Ansprüche 1-9 und 23-28 oder pharmazeutisch verwendbare Salze von solchen Verbindungen mit salzbildenden Gruppen enthalten.

34. Verwendung der pharmakologisch verwendbaren Verbindungen nach einem der Ansprüche 1-28 und ihrer Salze zur Herstellung von pharmazeutischen Präparaten.

35. Verwendung von Verbindungen der Formel Ic gemäss einem der Ansprüche 10-22 und von pharmazeutisch verwendbaren Salzen von solchen Verbindungen mit salzbildenden Gruppen als immunpotenzierend wirksame Verbindungen.

36. Pharmazeutische Präparate, die mindestens ein Antibiotikum und mindestens ein Pyranosederivat der Formel 1 gemäss einem der Ansprüche 1-28 und/oder ein Salz desselben enthalten.

37. Futtermittel und Futtermittelzusätze, die mindestens ein Antibiotikum und mindestens ein Muramylpeptid gemäss einem der Ansprüche 1-28 und/oder eim Salz desselben enthalten.

38. Verfahren zur Herstellung von Verbindungen der Formel 1 gemäss einem der Ansprüche 1-28 und Salzen von solchen

Verbindungen mit salzbildenden Gruppen, dadurch gekennzeichnet, dass man

a) in einer Verbindung der Formel I, worin funktionelle Gruppen gegebenenfalls in geschützter Form vorliegen, mindestens eine leicht durch Wasserstoff ersetzbare Gruppe abspaltet, oder mindestens eine funktionelle Gruppe in die den Radstoffem entsprechenden Derivate überführt oder

b) im einer Verbindung der Formel I, worin funktionelle Gruppen gegebenenfalls in geschützter Form vorhanden sind und worin $R_8$ einen Niederalkyl- oder Phenylniederalkylrest darstellt, der auch mit $R_7$ verbunden sein kann und der eine Hydroxy-, Mercapto-, Amino- oder Carboxygruppe trägt, die gegebenenfalls in reaktionsfähiger Form vorliegt,

und worin die übrigen Substituenten die in dem Anspruch, auf den man diesen Anspruch rückbezieht, genannte Bedeutung haben, gegebenenfalls stufenweise, über eine Oxycarbonyl-, Mercaptocarbonyl- und/oder Iminocarbonylgruppe den Rest $R_0$ einführt, oder

c) in einer Verbindung der Formel

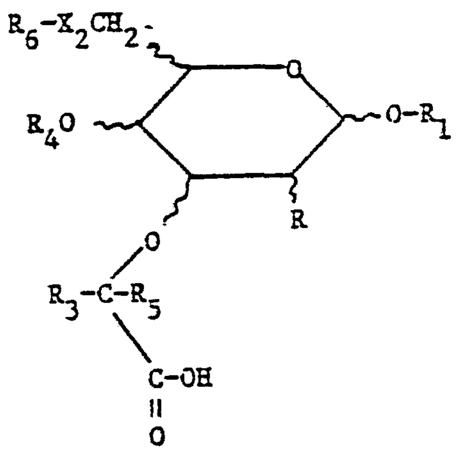

(IV)

worin die Substituenten die in dem Anspruch, auf den man diesen Anspruch rückbezieht, genannte Bedeutung haben, wobei freie funktionelle Gruppen gegebenenfalls in geschützter Form vorhanden sind, und worin die Carboxygruppe in derivatisierter Form vorliegen kann, die Carboxygruppe, gegebenenfalls stufenweise, mit einem den Rest der Formel

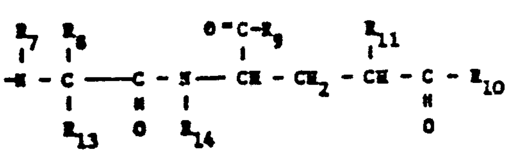

(IVa)

worin die Substituenten die in dem Anspruch, auf den man diesen Anspruch rückbezieht, genannte Bedeutung haben, wobei funktionelle Gruppen gegebenenfalls in geschützter Form vorhanden sind, übertragenden Mitteln amidiert,

und, wenn notwendig, in einer erhaltenen Verbindung geschützte funktionelle Gruppen in die freien funktionellen Gruppen überführt,

oder

d) eine Verbindung der Formel

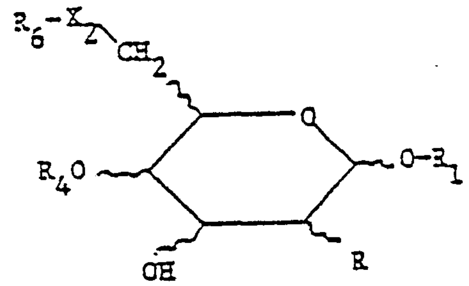

(VII),

worin die Substituenten die in dem Anspruch, auf den man diesen Anspruch rückbezieht, genannte Bedeutung haben, wobei funktionelle Gruppen vorzugsweise in geschützter Form vorliegen, oder ein reaktionsfähiges Derivat davon mit einer Verbindung der Formel

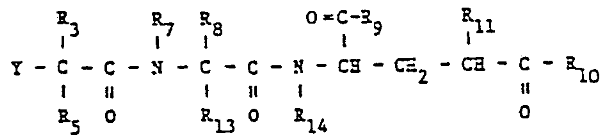

(VIII),

worin Y eine reaktionsfähige veresterte Hydroxygruppe darstellt und die übrigen Substituenten die in dem Anspruch, auf den man diesen Anspruch rückbezieht, genannte Bedeutung haben, wobei funktionelle Gruppen vorzugsweise in geschützter Form vorliegen, umsetzt, und, wenn notwendig, in einer erhaltenen Verbindung geschützte funktionelle Gruppen in die freien funktionellen Gruppen überführt, und, wenn erwünscht, eine verfahrensgemäss erhältliche Verbindung der Formel I in eine andere Verbindung der Formel I überführt, und/oder, wenn erwünscht, ein verfahrensgemäss erhältliches Salz einer Verbindung der Formel I mit einer salzbildenden Gruppe in die entsprechende freie Verbindung der Formel I oder in ein anderes Salz umwandelt, und/oder, wenn erwünscht, eine verfahrensgemäss erhältliche Verbindung der Formel I mit einer salzbildenden Gruppe in ein Salz überführt, und/oder, wenn erwünscht, ein verfahrensgemäss erhältliches Isomerengemisch von Verbindungen der Formel I in die isomeren Verbindungen der Formel I auftrennt.

39. Verfahren zur Herstellung von Verbindungen der im Anspruch 1 abgebildeten Formel I gemäss einem der Ansprüche 2 und 10-22 oder von Salzen solcher Verbindungen mit mindestens einer salzbildenden Gruppe, worin die Substituenten die in dem betreffenden Anspruch

genannten Bedeutungen haben, mit der Massgabe, dass R einen Rest der Formel

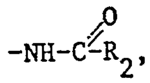

$R_1$, $R_4$ und $R_6$ Wasserstoff und $X_2$ Sauerstoff bedeuten und dass sich der Pyranoseteil von D-Glucose ableitet, dadurch gekennzeichnet, dass man in einer Verbindung der Formel

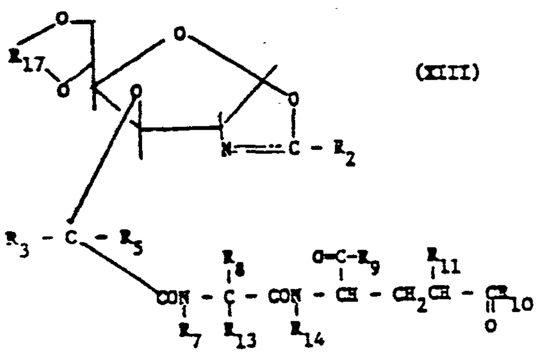

(XIII)

worin $R_{17}$ eine Alkyliden- oder Cycloalkylidengruppe ist und die anderen Substituenten die obengenannten Bedeutungen haben, den Oxazolinund den Dioxolanring sauer aufspaltet, und gegebenenfalls vorhandene Schutzgruppen abspaltet, und, wenn erwünscht, eine verfahrensgemäss erhältliche Verbindung der Formel Ic in eine andere Verbindung der Formel Ic überführt, und/oder, wenn erwünscht, ein verfahrensgemäss erhältliches Salz einer Verbindung der Formel Ic mit einer salzbildenden Gruppe in die entsprechende freie Verbindung der Formel Ic oder in ein anderes Salz umwandelt, und/oder, wenn erwünscht, eine verfahrensgemäss erhältliche Verbindung der Formel Ic mit einer salzbildenden Gruppe in ein Salz überführt, und/oder, wenn erwünscht, ein verfahrensgemäss erhältliches Isomerengemisch von Verbindungen der Formel Ic in die isomeren Verbindungen der Formel Ic auftrennt.

40. Die nach den Verfahren der Ansprüche 38 und 39 erhältlichen Verbindungen.

41. Peptide der Formel II

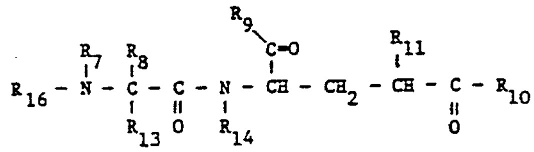

(II)

worin $R_{16}$ Wasserstoff oder eine Aminoschutzgruppe oder der Rest $R_{16}$-$\overset{R_7}{N}$ eine aktivierte Aminogruppe bedeuten und die übrigen Substituenten die in einem der Ansprüche 1, 7, 10, 12-14 und 16 genannte Bedeutung haben, und Salze von solchen Verbindungen mit salzbildenden Gruppen.

42. Verfahren zur Herstellung von Verbindungen der Formel (II) gemäss Anspruch 41 und ihrer Salze, dadurch gekennzeichnet, dass man

a) eine Aminosäure der Formel

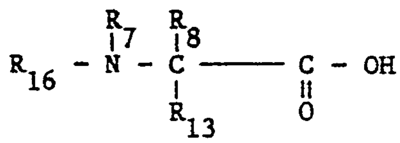

worin $R_{16}$ eine Aminoschutzgruppe bedeutet, während die übrigen Substituenten die in Anspruch 41 genannte Bedeutung haben, und die Carboxylgruppe gegebenenfalls in aktivierter Form vorliegt, wobei andere funktionelle Gruppen gegebenenfalls in geschützter Form vorliegen, mit einer Aminosäure der Formel

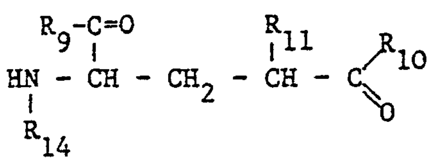

worin die

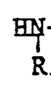–Gruppe

gegebenenfalls in aktivierter Form vorliegt, und die übrigen Substituenten die in Anspruch 41 genannte Bedeutung haben, wobei darin vorhandene funktionelle Gruppen gegebenenfalls in geschützter Form vorliegen, umsetzt, und, wenn erwünscht, mindestens eine gegebenenfalls vorhandene Schutzgruppe abspaltet, und/oder, wenn erwünscht, eine erhaltene Verbindung mit mindestens einer salzbildenden Gruppe in ihr Salz überführt, oder

b) in einer Verbindung der Formel II, worin funktionelle Gruppen gegebenenfalls in geschützter Form vorliegen und worin $R_8$ einen

Niederalkyl- oder Phenylniederalkylrest darstellt, der auch mit $R_7$ verbunden sein kann und der eine gegebenenfalls in reaktionsfähiger Form vorliegende Hydroxy-, Mercapto-, Amino- oder Carboxygruppe trägt, gegebenenfalls stufenweise, über die Oxycarbonyl-, Mercaptocarbonyl- oder Iminocarbonylgruppe den Rest $R_o$, der gemäss einem der Ansprüche 1, 7, 10, 12-14 und 16 definiert ist, einführt, und, wenn erwünscht, mindestens eine gegebenenfalls vorhandene Schutzgruppe abspaltet, und/oder, wenn erwünscht, eine erhaltene Verbindung mit mindestens einer salzbildenden Gruppe in ein Salz überführt, oder

c) in einer Verbindung der Formel II, worin funktionelle Gruppen gegebenenfalls in geschützter Form vorliegen, mindestens eine leicht durch Wasserstoff ersetzbare Gruppe abspaltet, oder mindestens eine funktionelle Gruppe in die den Endstoffen entsprechenden Derivate überführt.

43. Die nach dem Verfahren des Anspruchs 42 erhältlichen Verbindungen und ihre Salze.

44. Verwendung eZ/M Peptids der Formel II gemäss Anspruch 41 als Zwischenprodukt zur Herstellung von Verbindungen der Formel I gemäss einem der Ansprüche 1-16.

**Patentansprüche** für den Vertragsstaat AT

1. Verfahren zur Herstellung von Pyranosederivaten der Formel I,

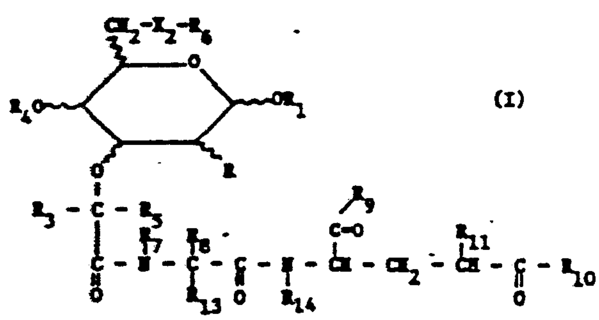

(I)

worin R für Hydroxy, Amino oder einen Rest der Formel $-X_1$

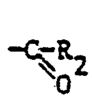

steht,

X und $X_2$ unabhängig voneinander für $NR_{15}$ oder ein Sauerstoffatom stehen, wobei $R_{15}$ Wasserstoff oder Niederalkyl bedeutet, $R_1$ und $R_4$ unabhängig voneinander für Wasserstoff, Acyl oder eine Hydroxyschutzgruppe, $R_6$ für Wasserstoff, Acyl oder, falls $X_2$ ein Sauerstoffatom bedeutet, für eine Hydroxyschutzgruppe stehen, $R_2$ jeweils gegebenenfalls substituiertes Alkyl, Aryl oder Alkoxy darstellt, $R_3$, $R_5$, $R_7$, $R_{13}$ und $R_{14}$ unabhängig voneinander für Wasserstoff oder Niederalkyl stehen, und $R_8$ einen Niederalkyl- oder Phenylniederalkylrest darstellt, der auch mit $R_7$ verbunden sein kann, und der eine Oxycarbonyl-, Mercaptocarbonyl- oder Aminocarbonylgruppe trägt, welche mit einem gegebenenfalls substituierten aliphatischen, cycloaliphatisch-aliphatischen, cycloaliphatischen oder aromatischen Kohlenwasserstoffrest $R_o$ verbunden ist, welch letzterer durch Oxycarbonyl, Mercaptocarbonyl und/oder Iminocarbonyl unterbrochen sein kann und der mehr als 5 C-Atoms aufweisen muss, wenn $R_1$, $R_4$ und $R_6$ Wasserstoff bedeuten, worin die Reste $R_9$ und $R_{10}$ für gegebenenfalls veräthertes Hydroxy oder gegebenenfalls substituiertes Amino stehen, und $R_{11}$ Wasserstoff oder einen Rest der Formel $-C(=O)-R_{12}$ (Ia) bedeutet, worin $R_{12}$ für gegebenenfalls veräthertes Hydroxy oder gegebenenfalls substituiertes Amino steht, wobei mit "nieder" bezeichnete Reste bis und mit 7 Kohlenstoffatome enthalten, und von Salzen von Verbindungen der Formel I mit mindestens einer salzbildenden Gruppe, dadurch gekennzeichnet, dass man

a) in einer Verbindung der Formel I, worin funktionelle Gruppen gegebenenfalls in geschützter Form vorliegen, mindestens eine leicht durch Wasserstoff ersetzbare Gruppe abspaltet oder mindestens eine funktionelle Gruppe in die den Endstoffen entsprechenden Derivate überführt oder

b) in einer Verbindung der Formel 1, worin funktionelle Gruppen gegebenenfalls in geschützter Form vorhanden sind und worin $R_8$ einen Niederalkyl- oder Phenylniederalkylrest darstellt, der auch mit $R_7$ verbunden sein kann und der eine Hydroxy-, Mercapto-, Amino- oder Carboxygruppe trägt, die gegebenenfalls in reaktionsfähiger Form vorliegen, und worin die übrigen Substituenten die obengenannte Bedeutung haben, gegebenenfalls stufenweise, über eine Oxycarbonyl-, Mercaptocarbonyl- und/oder Iminocarbonylgruppe den Rest $R_o$ einführt, und, wenn notwendig, in einer erhaltenen Verbindung geschützte funktionelle Gruppen in die freien funktionellen Gruppen überführt,

oder

c) in einer Verbindung der Formel

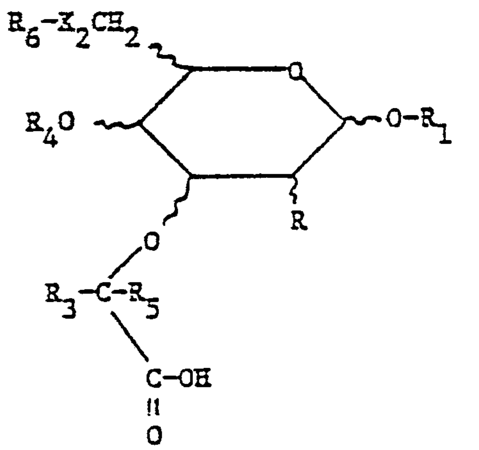

(IV)

worin die Substituenten die obengenannte Bedeutung haben, wobei freie funktionelle Gruppen gegebenenfalls in geschützer Form vorhanden sind, und worin die Carboxygruppe in derivatisierter Form vorliegen kann, die Carboxygruppe, gegebenenfalls stufenweise, mit einem den Rest der Formel

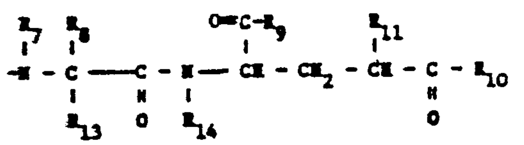

(IVa)

worin die Substituenten die obengenannte Bedeutung haben, wobei funktionelle Gruppem gegebenenfalls in geschützter Form vorhanden sind, übertragenden Mittel amidiert, und, wenn notwendig, in einer erhaltenen Verbindung geschützte funktionelle Gruppen in die freien funktionellen Gruppen überführt,

oder

d) eine Verbindung der Formel

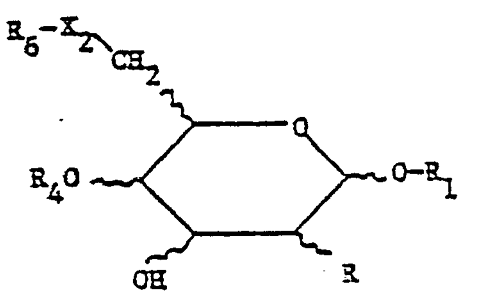

(VII),

worin die Substituenten die obengenannte Bedeutung haben, wobei funktionelle Gruppen vorzugsweise in geschützter Form vorliegen, oder ein reaktionsfähiges Derivat davon mit einer Verbindung der Formel

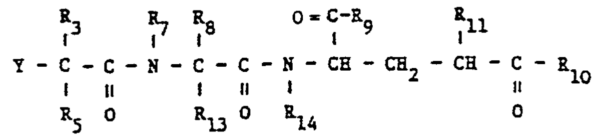

(VIII),

worin Y eine reaktiomsfähige veresterte Hydroxygruppe darstellt und die übrigen Substituenten die obengenannte Bedeutung haben, wobei funktionelle Gruppen vorzugsweise in geschützter Form vorliegen, umsetzt, und, wenn notwendig, in einer erhaltenen Verbindung geschützte funktionelle Gruppen in die freien funktionellen Gruppen überführt, oder

e) zur Herstellung einer Verbindung der Formel I, worin R einen Rest der Formel

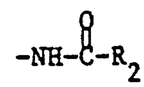 ,

$R_1$, $R_4$ und $R_6$ Wasserstoff und $X_2$ Sauerstoff bedeuten und worin sich der Pyranoseteil von D-Glucose ableitet und die übrigen Substituenten die obengenannten Bedeutungen haben, oder eines Salzes einer solchen Verbindung mit mindestens einer salzbildenden Gruppe in einer Verbindung der Formel

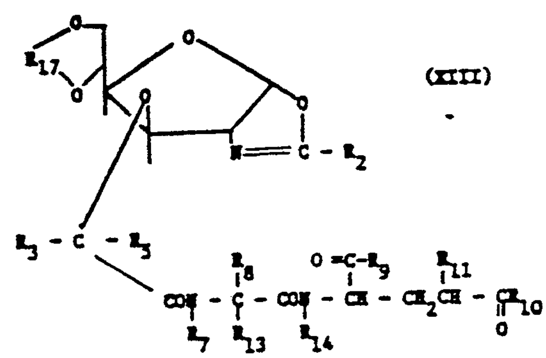

(XIII)

worin $R_{17}$ eine Alkyliden- oder Cycloalkylidengruppe ist und die anderen Substituenten die obengenannten Bedeutungen haben, den Oxazolinund den Dioxolanring sauer aufspaltet, und gegebenenfalls vorhandene Schutzgruppen abspaltet, und, wenn erwünscht, eine verfahrensgemäss erhältliche Verbindung der Formel I in eine andere Verbindung der Formel I überführt, und/oder, wenn erwünscht, ein verfahrensgemäss erhältliches Salz einer Verbindung der Formel I mit einer salzbildenden Gruppe in die entsprechende freie Verbindung

der Formel I oder in ein anderes Salz umwandelt, und/oder, wenn erwünscht, eine verfahrensgemäss erhältliche Verbindung der Formel I mit einer salzbildenden Gruppe in ein salz überführt, und/oder, wenn erwünscht, ein verfahrensgemäss erhältliches Isomerengemisch von Verbindungen der Formel I in die isomeren Verbindungen der Formel I auftrennt.

2. Verfahren gemäss Anspruch 1, dadurch gekennzeichnet, dass man Verbindungen der Formel I herstellt, deren Pyransoseteil sich von der D-Glucose ableitet.

3. Verfahren gemäss Anspruch 1, dadurch gekennzeichent, dass man Verbindungen der Formel I herstellt, deren Pyranoseteil sich von der D-Mannose oder der D-Galaktose ableitet.

4. Verfahren nach einem der Ansprüche 1-3, dadurch gekennzeichnet, dass man Verbindungen der Formel I herstellt, die in Fällen asymmetrischer Substitution am C-$NR_{14}$ die (D)-Konfiguration aufweisen.

5. Verfahren nach Anspruch 4, dadurch gekennzeichnet, dass man Verbindungen der Formel I herstellt, die in Fällen asymmetrischer Substitution am C-$R_3$ die (D)- und am C-$R_8$ die (L)-Konfiguration aufweisen.

6. Verfahren nach einem der Ansprüche 1-5, dadurch gekennzeichnet, dass man Verbindungen der Formel I herstellt, worin R für einen Rest der Formel

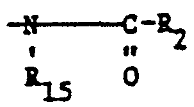

und $X_2$ für ein Sauerstoffatom stehen.

7. Verfahren nach einem der Ansprüche 1-6, dadurch gekennzeichnet, dass man Verbindungen der Formel I herstellt, worin $R_5$, $R_{13}$, $R_{14}$ und $R_{15}$ für Wasserstoff stehen.

8. Verfahren nach einem der Ansprüche 1-7, dadurch gekennzeichnet, dass man Verbindungen der Formel I herstellt, worin $R_1$, $R_4$ und $R_6$für Wasserstoff, gegebenenfalls ungesättigtes Alkanoyl mit 2-25 C Atomen oder für gegebenenfalls substituiertes Benzoyl stehen.

9. Verfahren nach einem der Ansprüche 1-7, dadurch gekennzeichnet, dass man Verbindungen der Formel I herstellt, worin $R_6$ für den Rest

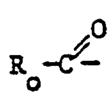

steht.

10. Verfahren gemäss Anspruch 1, dadurch gekennzeichnet, dass man Peptidderivate von Glucosaminverbindungen der Formel Ic herstellt,

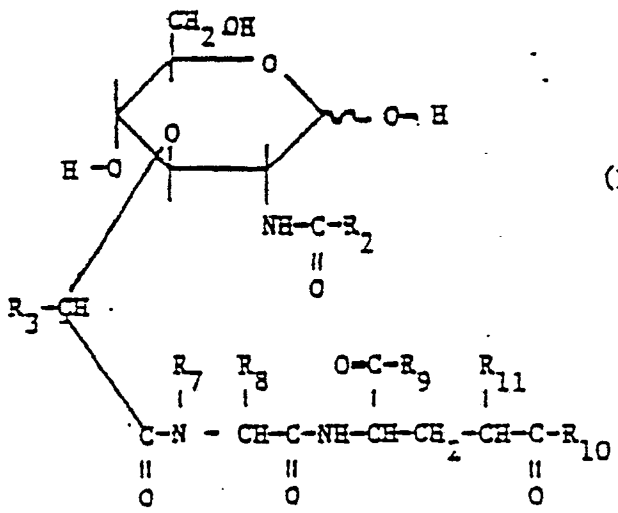

(Ic)

worin $R_2$ gegebenenfalls substituiertes Niederalkyl Phenyl, Niederalkoxy oder Phenylniederalkoxy darstellt, $R_3$ für Wasserstoff oder Niederalkyl steht, $R_7$ Wasserstoff oder Niederalkyl bedeutet, und $R_8$ einen Niederalkyl- oder Phenylniederalkylrest darstellt, der auch mit $R_7$ verbunden sein kann, und der eine Oxycarbonyl-, Mercaptocarbonyl- oder Aminocarbonylgruppe trägt, welche mit einem gegebenenfalls substituierten, langkettigen aliphatischenoder cycloaliphatisch-aliphatischen Kohlenwasserstoffrest verbunden ist, welch letzterer durch Oxycarbonyl, Mercaptocarbonyl und/oder Iminocarbonyl unterbrochen sein kann, worin $R_9$ und $R_{10}$ für gegebenenfalls veräthertes Hydroxy oder gegebenenfalls substituiertes Amino stehen, und $R_{11}$ Wasserstoff oder einen Rest der Formel -C(=O)-$R_{12}$ (Ia) bedeutet, worin $R_{12}$ für gegebenenfalls veräthertes Hydroxy oder gegebenenfalls substituiertes Amino steht, oder Salze von Verbindungen der Formel Ic mit mindestens einer salzbildenden Gruppe herstellt.

11. Verfahren gemäss Anspruch 10, dadurch gekennzeichnet, dass man Verbindungen der Formel Ic, worin der Rest der Hydroxyessigsäure mit der Gruppierung der Formel -CH($R_3$)-, worin $R_3$ Niederalkyl darstellt, in der D-Form, der Rest der Aminoessigsäure mit der Gruppierung der Formel -CH($R_8$)- in der L-Form, und der Rest der terminalen α-Amino-glutarsäureverbiudung in der D-Form vorliegen, oder Salze von solchen Verbindungen mit mindestens einer salzbildenden Gruppe herstellt.

12. Verfahren gemäss Anspruch 10, dadurch gekennzeichnet, dass man Verbindungen der Formel Ic, worin $R_2$ für gegebenenfalls durch Hydroxy, Niederalkoxy, Niederalkanoyloxy oder Halogen substituiertes Niederalkyl oder Niederalkoxy, oder gegebenenfalls durch Niederalkyl, Hydroxy, Niederalkoxy, Niederalkanoyloxy oder Halogen substituiertes Phenyl oder Phenylniederalkoxy steht, $R_3$ Wasserstoff oder Niederalkyl bedeutet, $R_7$

Wasserstoff oder Niederalkyl bedeutet, und $R_8$ einen Niederalkyl- oder Phenylniederalkylrest darstellt, der als Substituenten eine Oxycarbonyl-, Mercaptocarbonyl- oder Aminocarbonylgruppe trägt, welche ihrerseits wiederum mit einem gegebenenfalls durch Hydroxy, Niederalkoxy, Niederalkanoyloxy, Oxo oder Halogen substituierten Alkyloder Alkenylrest mit mehr als 6 und bis zu 90 Kohlenstoffatomen oder einem cycloaliphatisch substituierten Alkyl- oder Alkenylrest mit z.B. bis zu 30 Kohlenstoffatomen verbunden ist, und worin die Alkyl-, Alkenyl- und cycloaliphatisch substituierten Alkyl- bzw. Alkenylreste auch durch eine oder zwei Oxycarbonyl- oder Iminocarbonyl-Gruppen unterbrochen sein können, $R_{11}$ Wasserstoff oder den Rest der Formel Ia bedeutet, und jeder der Reste $R_9$, $R_{10}$ und $R_{12}$ Hydroxy, Niederalkoxy, Amino oder durch, gegebenenfalls Carboxy, Niederalkoxycarbonyl oder Carbamoylenthaltendes Niederalkyl substituiertes Amino darstellt, wobei in solchen Verbindungen z.B. der Rest der Hydroxyessigsäure mit der Gruppierung der Formel -CH($R_3$)-, worin $R_3$ Niederalkyl darstellt, in der D-Form, der Rest der Aminoessigsäure mit der Gruppierung der Formel -CH($R_8$)-, in der L-Form, und der Rest der terminalen α-Aminoglutarsäureverbindung in der D-Form vorliegen, sowie Salze von solchen Verbindungen mit mindestens einer salzbildenden Gruppe herstellt.

13. Verfahren gemäss Anspruch 10, dadurch gekennzeichnet, dass man Verbindungen der Formel Ic, worin $R_2$ Niederalkyl mit bis zu 4 Kohlenstoffatomen oder Phenyl darstellt, $R_3$ Wasserstoff oder Niederalkyl mit bis zu 4 Kohlenstoffatomen bedeutet, $R_7$ Wasserstoff darstellt und $R_8$ einen Niederalkylrest mit 1-4 Kohlenstoffatomen oder einen Phenylniederalkylrest darstellt, der als Substituenten eine Oxycarbonyl-, Mercaptocarbonyl- oder Aminocarbonylgruppe trägt, welche ihrerseits mit einem gegebenenfalls durch Hydroxy, Niederalkoxy, Oxo oder Halogen substituierten Alkyl oder Alkenyl mit mehr als 10 und bis zu 50 Kohlenstoffatomen oder einem gegebenenfalls durch Hydroxy, Niederalkoxy, Oxo oder Halogen substituierten tetracyclischen Cycloalkyl-alkyl oder -alkenyl mit mehr als 20 und bis zu 50 Kohlenstoffatomen verbunden ist, wobei solche Reste auch durch 1 oder 2 Oxycarbonyl- oder Iminocarbonylgruppen unterbrochen sein können, $R_9$ für Amino oder gegebenenfalls Carboxy oder Carbamoyl enthaltendes Niederalkylamino steht, $R_{10}$ Hydroxy bedeutet und $R_{11}$ Wasserstoff darstellt wobei in solchen Verbindungen z.B. der Rest der Hydroxyessigsäure mit der Gruppierung der Formel -CH($R_3$)-, worin $R_3$ Niederalkyl darstellt, im der D-Form, der Rest der Aminoessigsäure mit der Gruppierung der Formel -CH($R_8$)- in der L-Form, und der Rest der terminalen α-Aminoglutarsäureverbindung in der D-Form vorliegen, und pharmazeutisch verwendbare Salze von solchen Verbindungen mit mindestens einer salzbildenden Gruppe herstellt.

14. Verfahren gemäss Anspruch 10, dadurch gekennzeichnet, dass msn Verbindungen der Formel Ic, worin $R_2$ Niederalkyl mit bis zu 4 Kohlenstoffatomen oder Phenyl bedeutet, $R_3$ insbesondere Wasserstoff, ferner Niederalkyl mit bis zu 4 Kohlenstoffatomen, bedeutet, $R_7$ Wasserstoff darstellt, $R_8$ Niederalkyl mit 1-4 Kohlenstoffatomen oder Benzyl darstellt, die als Substituenten eine Oxycarbonyl-, Mercaptocarbonyloder Aminocarbonylgruppe tragen, welche vorzugsweise über das Heteroatom mit dem Niederalkyl- bzw. Benzylrest verbunden ist, und die ihrerseits mit einem gegebenenfalls durch Hydroxy, Niederalkoxy oder Oxo substituierten Alkyl oder Alkenyl mit mehr als 10 und bis zu 50 Kohlenstoffatomen oder einem gegebenenfalls durch Hydroxy oder Niederalkoxy substituierten tetracyclischen Cycloalkyl-alkyl- oder Cycloalkenyl-alkylrest mit mehr als 20 und bis zu 50 Kohlenstoffatomen verbunden ist, welche Reste auch durch eine Oxycarbonyl- oder Iminocarbonylgruppe unterbrochen sein können, $R_9$ Amino bedeutet, $R_{10}$ für Hydroxy steht, und $R_{11}$ Wasserstoff darstellt, wobei in solchen Verbindungen der Rest der Hydroxyessigsäure mit der Gruppierung der Formel -CH($R_3$)-, worin $R_3$ Niederalkyl darstellt, in der D-Form, der Rest der Aminoessigsäure mit der Gruppierung der Formel -CH($R_8$)- in der L-Form und der Rest der terminalen α-Aminoglutarsäureverbindung in der D-Form vorliegen, oder pharmazeutisch verwendbare Salze von solchen Verbindungen mit mindestens einer salzbildenden Gruppe herstellt.

15. Verfahren gemäss Anspruch 14, dadurch gekennzeichnet, dass man Verbindungen der Formel Ic, worin $R_2$ Methyl oder Phenyl, $R_3$ Wasserstoff oder Methyl bedeuten und die übrigen Reste die in Anspruch 14 gegebenen Bedeutungen haben, herstellt.

16. Verfahren gemäss Anspruch 10, dadurch gekennzeichnet, dass man Verbindungen der Formel Ic, worin $R_2$ Niederalkyl mit bis zu 3 Kohlenstoffatomen oder Phenyl, $R_3$ Wasserstoff oder Methyl, $R_7$ Wasserstoff, $R_8$ Niederalkyl mit 1-3 Kohlenstoffatomen, das als Substituenten eine Oxycarbonyl-, Mercaptocarbonyl- oder Aminocarbonylgruppe trägt, welche über das Heteroatom mit dem Niederalkylrest verbunden ist und die ihrerseits mit einem gegebenenfalls durch Hydroxy, Niederalkoxy oder Oxo substituierten Alkyl oder Alkenyl mit mehr als 10 und bis zu 50 Kohlenstoffatomen oder einem gegebenenfalls durch Hydroxy oder Niederalkoxy substituierten tetracyclischen Cycloalkyl-alkyl- oder Cycloalkenyl-alkylrest mit mehr als 20 und bis zu 50 Kohlenstoffatomen verbunden ist, welche Reste auch durch Oxycarbonyl oder Iminocarbonyl unterbrochen sein können, $R_9$ Amino, Niederalkylamino oder Carbamoylniederalkylamino oder Hydroxy, $R_{10}$ Hydroxy, Niederalkoxy, Amino oder Carbamoylniederalkylamino, und $R_{11}$ Wasserstoff bedeuten, wobei in solchen Verbindungen der

Rest der Hydroxyessigsäure mit der Gruppierung -$CH(R_3)$-, worin $R_3$ Methyl darstellt, in der D-Form, der Rest der Aminosäure mit der Gruppierung -$CH(R_8)$- in der L-Form und der Rest der terminalen α-Amino-glutarsäureverbindung in der D-Form vorliegen, und pharmazeutisch verwendbare Salze von solchen Verbindungen mit mindestens einer salzbildenden Gruppe herstellt.

17. Verfahren gemäss Anspruch 11, dadurch gekennzeichnet, dass man Verbindungen der Formel Ic, worin $R_2$ Niederalkyl mit bis zu 3 Kohlenstoffatomen, $R_3$ Wasserstoff oder Methyl, $R_7$ Wasserstoff, $R_8$ Niederalkyl mit 1-4 C-Atomen, das als Substituenten eine Oxycarbonyl-, Mercaptocarbonyl- oder Aminocarbonylgruppe trägt, welche über das Heteroatom mit dem Niederalkylrest verbunden ist und die ihrerseits mit Alkyl mit mehr als 10 und bis zu 50 Kohlenstoffatomen verbunden ist, das auch durch Iminocarbonyl unterbrochen sein kann, $R_9$ Amino, Hydroxy oder Niederalkoxy mit 1-3 C-Atomen, $R_{10}$ Hydroxy, Niederalkoxy mit 1-3 C-Atomen oder Amino und $R_{11}$ Wasserstoff oder Carboxy bedeuten, oder pharmazeutisch verwendbare Salze von solchen Verbindungen mit mindestens einer salzbildenden Gruppe herstellt.

18. Verfahren gemäss Anspruch 17, dadurch gekennzeichnet, dass man Verbindungen der Formel Ic, worin $R_8$ Alkanoyloxymethyl oder Alkanoyloxyäthyl mit mehr als 10 und bis zu 30 C-Atomen in Alkylteil des Alkanoylrestes, $R_9$ Amino und $R_{10}$ Hydroxy bedeuten, oder ihre pharmazeutisch verwendbaren Salze herstellt.

19. Verfahren nach Anspruch 10, dadurch gekennzeichnet, dass man N-Acetyl-normuramyl-L-O-(N-behenoyl-glycyl)-seryl-D-isoglutamin oder ein pharmazeutisch verwendbares Salz davon, herstellt.

20. Verfahren nach Anspruch 10, dadurch gekennzeichnet, dass man N-Acetyl-normuramyl-L-O-behenoyl-seryl-D-isoglutamin oder ein pharmazeutisch verwendbares Salz davon herstellt.

21. Verfahren nach Anspruch 10, dadurch gekennzeichnet, dass man N-Acetyl-muramyl-L-O-[N-(D,L-2-n-hexadecanoyl-amino-n-hexadecanoyl)-L-alanyl]-threonyl-D-isoglutamin oder ein pharmazeutisch verwendbares Salz davon herstellt.

22. Verfahren nach Anspruch 10, dadurch gekennzeichnet, dass man N-Acetyl-muramyl-L-S-stearoyl-cysteinyl-D-isoglutamin oder ein pharmazeutisch verwendbares Salz davon herstellt.

23. Verfahren nach Anspruch 10, dadurch gekennzeichnet, dass man eine Verbindung ausgewählt aus der Gruppe der folgenden Verbindungen und ihrer Salze herstellt: N-Acetyl-normuramyl-L-O-[N-(12-hydroxy-cis-9-octadecenoyl)-glycyl]seryl-D-isoglutamin,

N-Benzoyl-normuramyl-L-O-stearoyl-seryl-D-isoglutamin,

N-Acetyl-muramyl-L-O(ω-n-stearoylamino-n-undecanoyl)-threonyl-D-isoglutamin,

N-Acetyl-muramyl-L-O-[N-(3-hydroxy-ätio-cholenoyl)-6-amino-hexanoyl]γ hydroxyprolyl-D-isoglutamin,

N-Acetyl-muramyl-L-O-behenoyl-tyrosyl-D-isoglutamin,

N-Acetyl-muramyl-L-(C)-[tetracosylamido-glycyl]-glutamyl-D-isoglutamin,

N-Acctyl-muramyl-L-[($C_\gamma$)-lauryl]-α-glutamyl-D-isoglutamin,

N-Acetyl-normuramyl-L-γ-stearoylamino-α-amino-butanoyl-D-isoglutamin,

N-Acetyl-muramyl-L-(O-behenoyl-N-methyl)-seryl-D-isoglutamin,

N-Acetyl-muramyl-L-(Nε-palmitoyl)-lysyl-D-glutaminsäure-dimethylester.

24. Verfahren nach Anspruch 10, dadurch gekennzeichnet, dass man N-Benzoyl-normuramyl-L-(O-stearoyl)-seryl-D-glutaminsäure-dimethylester herstellt.

25. Verfahren nach Anspruch 10, dadurch gekennzeichnet, dass man N-Benzoyl-normuramyl-L-(O-behenoyl)-seryl-D-isoglutamin oder ein pharmazeutisch verwendbares Salz davon herstellt.

26. Verfahren nach Anspruch 10, dadurch gekennzeichnet, dass man N-Acetyl-isomuramyl-L-(O-behenoyl)-seryl-D-isoglutamin oder ein pharmazeutisch verwendbares Salz davon herstellt.

27. Verfahren nach Anspruch 10, dadurch gekennzeichnet, dass man N-Acetyl-muramyl-L-(O-behenoyl)-seryl-D-isoglutamin oder ein pharmazeutisch verwendbares Salz davon herstellt.

28. Verfahren nach Anspruch 10, dadurch gekennzeichnet, dass man eine Verbindung ausgewählt aus der Gruppe der folgenden Verbindungen und ihrer Salze herstellt:

N-Acetyl-muramyl-D-(O-oleoyl)-seryl-D-isoglutamin,

N-Acetyl-1,4,6-0-triacetyl-normuramyl-L-(O-behenoyl)-seryl-D-isoglutamin,

N-Acetyl-nuramyl-L-(4-behenoyloxy)-prolyl-D-isoglutaminyl-γ-L-alanin und

N-Acetyl-muramyl-L-(O-behenoyl)-seryl-D-γ-carboxy-α-glutamyl-glycinamid.

29. Verfahren zur Herstellung von Peptiden der Formel II,

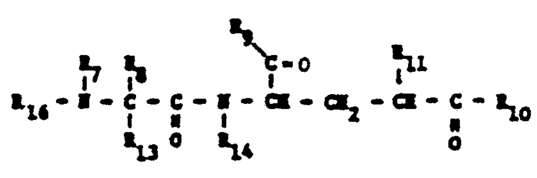 (II)

worin $R_{16}$ Wasserstoff oder eine Aminoschutzgruppe bedeutet und die übrigen Substituenten die in einem der Ansprüche 1, 7, 10, 12-14 und 16-18 genannte Bedeutung haben, und von Salzen von solchen Verbindungen mit salzbildenden Gruppen, dadurch gekennzeichnet, dass man

a) eine Aminosäure der Formel

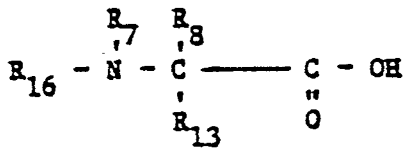

worin $R_{16}$ eine Aminoschutzgruppe bedeutet, während die übrigen Substituenten die obengenannte Bedeutung haben, und die Carboxylgruppe gegebenenfalls in aktivierter Form vorliegt, wobei andere funktionelle Gruppen gegebenenfalls in geschützer Form vorliegen, mit einer Aminosäure der Formel

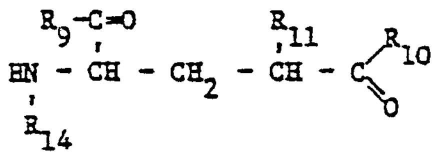

worin die

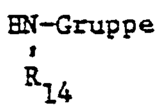

gegebenenfalls in aktivierter Form vorliegt, und die übrigen Substituenten die obengenannte Bedeutung haben, wobei darin vorhandene funktionelle Gruppen gegebenenfalls in geschützter Form vorliegen, umsetzt, und, wenn erwünscht, mindestens eine gegebenenfalls vorhandene Schutzgruppe abspaltet, und/oder, wenn erwünscht, eine erhaltene Verbindung mit mindestens einer salzbildenden Gruppe in ihr Salz überführt, oder

b) in einer Verbindung der Formel II, worin funktionelle Gruppen gegebenenfalls in geschützter Form vorliegen und worin $R_8$ einen Niederalkyl- oder Phenylniederalkylrest darstellt, der auch mit $R_7$ verbunden sein kann und der eine gegebenenfalls in reaktionsfähiger Form vorliegende Hydroxy-, Mercapto-, Amino- oder Carboxygruppe trägt, gegebenenfalls stufenweise, über die Oxycarbonyl-, Mercaptocarbonyl- oder Iminocarbonylgruppe den Rest $R_0$, der gemäss einem der Ansprüche 1, 10, 12-14 und 16-18 definiert ist, einführt, und, wenn erwünscht, mindestens eine gegebenenfalls vorhandene Schutzgruppe abspaltet, und/oder, wenn erwünscht, eine erhaltene Verbindung mit mindestens einer salzbildenden Gruppe in ein Salz überführt, oder

c) in einer Verbindung der Formel 11, worin funktionelle Gruppen gegebenenfalls in geschützter Form vorliegen, mindestens eine leicht durch Wasserstoff ersetzbare Gruppe abspaltet, oder mindestens eine funktionelle Gruppe in die den Endstoffen entsprechenden Derivate überführt.

30. Verfahren nach Anspruch 29, dadurch gekennzeichnet, dass man die Ausgangsstoffe so wählt, dass man eine Verbindung der Formel II, die am C-$R_8$ die (L)- und am C-$NR_{14}$ die (D)-Konfiguration aufweist, oder ein Salz einer solchen Verbindung mit einer salzbildenden Gruppe herstellt.

**Claims** for the contracting states BE, CH, DE, FR, IT, LI, LU, NL, SE

1. Derivatives of pyranoses of the formula (I),

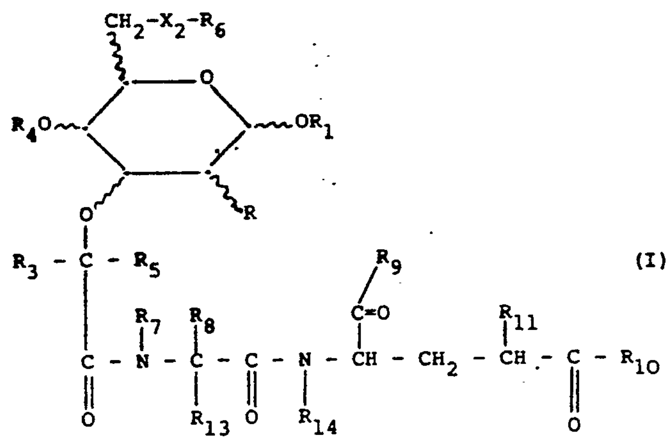

(I)

in which

R represents hydroxy, amino or a radical of the formula

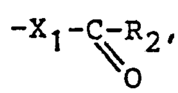

$X_1$ and $X_2$, independently of one another, each represents $NR_{15}$ or an oxygen atom, wherein $R_{15}$ represents hydrogen or lower alkyl,

$R_1$ and $R_4$, independently of one another, each represents hydrogen, acyl, or a hydroxyprotecting group,

$R_6$ represents hydrogen or acyl or, if $X_2$ represents an oxygen atom, a hydroxy-protecting group,

$R_2$ represents in each case optionally substituted alkyl, aryl or alkoxy,

$R_3$, $R_5$, $R_7$, $R_{13}$ and $R_{14}$, independently of one another, each represents hydrogen or lower alkyl, and

$R_8$ represents a lower alkyl or phenyl-lower alkyl

radical, which may also be bonded to $R_7$ and which carries an oxycarbonyl, mercaptocarbonyl or aminocarbonyl group which is bonded to an optionally substituted aliphatic, cycloaliphaticaliphatic, cycloaliphatic or aromatic, hydrocarbon radical $R_o$, which latter may be interrupted by oxycarbonyl, mercaptocarbonyl and/or iminocarbonyl, and which must have more than 5 carbon atoms when $R_1$, $R_4$ and $R_6$ each represents hydrogen,

$R_9$ and $R_{10}$ each represents optionally etherified hydroxy or optionally substituted amino, and

$R_{11}$ represents hydrogen or a radical of the formula $-C(=O)-R_{12}$ (Ia), in which $R_{12}$ represents optionally etherified hydroxy or optionally substituted amino,

wherein radicals designated "lower" contain up to and including 7 carbon atoms,

and salts of compounds of the formula I having at least one salt-forming group.

2. Compounds according to claim 1, the pyranose moiety of which is derived from D-glucose.

3. Compounds according to claim 1, the pyranose moiety of which is derived from D-mannose or D-galactose.

4. Compounds of the formula I, according to any one of claims 1 to 3, which, in cases of asymmetrical substitution, have the (D)-configuration at the C-$NR_{14}$.

5. Compounds of the formula I, according to claim 4, which, in cases of asymmetrical substitution, have the (D)-configuration at the C-$R_3$ and the (L)configuration at the C-$R_8$.

6. Compounds of the formula I, according to any one of claims 1 to 5, in which R represents a radical of the formula

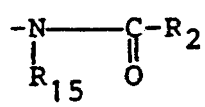

and $X_2$ represents an oxygen atom.

7. Compounds of the formula I, according to any one of claims 1 to 6, in which $R_5$, $R_{13}$, $R_{14}$ and $R_{15}$ each represents hydrogen.

8. Compounds of the formula I, according to any one of claims 1 to 7, in which $R_1$, $R_4$ and $R_6$ each represents hydrogen, optionally unsaturated alkanoyl having from 2 to 25 carbon atoms, or optionally substituted benzoyl.

9. Compounds of the formula I, according to any one of claims 1 to 7, in which $R_6$ represents the radical

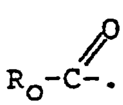

10.

10. Peptide derivatives, according to claim 1, of glucosamine compounds of the formula

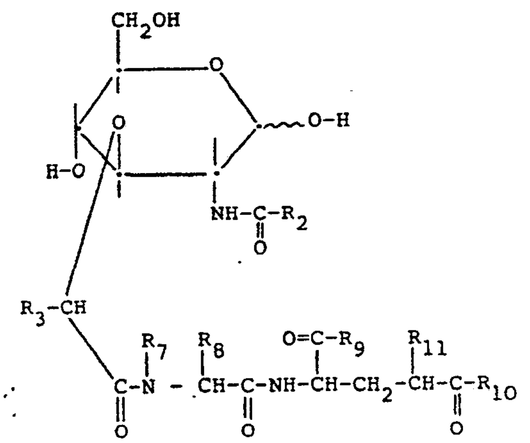

(Ic)

in which

$R_2$ represents optionally substituted lower alkyl, phenyl, lower alkoxy or phenyl-lower alkoxy,

$R_3$ represents hydrogen or lower alkyl,

$R_7$ represents hydrogen or lower alkyl, and

$R_8$ represents a lower alkyl or phenyl-lower alkyl radical, which may also be bonded to $R_7$ and which carries an oxycarbonyl, mercaptocarbonyl or aminocarbonyl group which is bonded to an optionally substituted long-chain aliphatic or cycloaliphatic-aliphatic hydrocarbon radical, which latter may be interrupted by oxycarbonyl, mercaptocarbonyl and/or iminocarbonyl,

$R_9$ and $R_{10}$ each represents optionally etherified hydroxy or optionally substituted amino, and

$R_{11}$ represents hydrogen or a radical of the formula $-C(=O)-R_{12}$ (Ia), in which $R_{12}$ represents optionally etherified hydroxy or optionally substituted amino,

and salts of compounds of the formula Ic having at least one salt-forming group,

11. Compounds of the formula Ic, according to patent claim 10, characterised in that the radical of the hydroxyacetic acid with the grouping of the formula $-CH(R_3)-$ in which $R_3$ represents lower alkyl is in the D-form, the radical of the aminoacetic acid with the grouping of the formula $-CH(R_8)-$ is in the L-form, and the radical of the terminal α-aminoglutaric acid compound is in the D-form, or salts of such compounds having salt-forming groups.

12. Compounds of the formula Ic, according to patent claim 10, in which $R_2$ represents lower alkyl or lower alkoxy each optionally substituted by hydroxy, lower alkoxy, lower alkanoyloxy or halogen, or phenyl or phenyl-lower alkoxy each optionally substituted by lower alkyl, hydroxy, lower alkoxy, lower alkanoyloxy or halogen, $R_3$ represents hydrogen or lower alkyl, $R_7$ represents hydrogen or lower alkyl, and $R_8$ represents a lower alkyl or phenyl-lower alkyl radical which

carries as substituent an oxycarbonyl, mercaptocarbonyl or aminocarbonyl group, which is itself in turn bonded to an alkyl or alkenyl radical having more than 6 and up to 90 carbon atoms optionally substituted by hydroxy, lower alkoxy, lower alkanoyloxy, oxo or halogen, or to a cycloaliphatically substituted alkyl or alkenyl radical having, for example, up to 30 carbon atoms, and in which the alkyl, alkenyl and cycloaliphatically substituted alkyl or alkenyl radicals may also be interrupted by one or two oxycarbonyl or iminocarbonyl groups, $R_{11}$ represents hydrogen or a radical of the formula Ia, and each of the radicals $R_9$, $R_{10}$ and $R_{12}$ represents hydroxy, lower alkoxy, amino, or amino which is substituted by lower alkyl optionally containing carboxy, lower alkoxycarbonyl or carbamoyl, wherein in such compounds, for example the radical of the hydroxyacetic acid with the grouping of the formula -CH($R_3$)- in which $R_3$ represents lower alkyl is in the D-form, the radical of the aminoacetic acid with the grouping of the formula -CH($R_8$)- is in the L-form, and the radical of the terminal α-aminoglutaric acid compound is in the D-form, and salts of such compounds having salt-forming groups.

13. Compounds of the formula Ic, according to patent claim 10, in which $R_2$ represents lower alkyl having up to 4 carbon atoms, or phenyl, $R_3$ represents hydrogen or lower alkyl having up to 4 carbon atoms, $R_7$ represents hydrogen and $R_8$ represents a lower alkyl radical having from 1 to 4 carbon atoms or a phenyl-lower alkyl radical, which carries as substituent an oxycarbonyl, mercaptocarbonyl or aminocarbonyl group which is itself bonded to an alkyl or alkenyl radical having more than 10 and up to 50 carbon atoms optionally substituted by hydroxy, lower alkoxy, oxo or halogen, or to a tetracyclic cycloalkylalkyl or cycloalkylalkenyl radical having more than 20 and up to 50 carbon atoms optionally substituted by hydroxy, lower alkoxy, oxo or halogen, wherein such radicals may also be interrupted by 1 or 2 oxycarbonyl or iminocarbonyl groups, $R_9$ represents amino, or lower alkylamino optionally containing carboxy or carbamoyl, $R_{10}$ represents hydroxy and $R_{11}$ represents hydrogen, wherein in such compounds, for example the radical of the hydroxyacetic acid with the grouping of the formula -CH($R_3$)- in which $R_3$ represents lower alkyl is in the D-form, the radical of the aminoacetic acid with the grouping of the formula -CH($R_8$)- is in the L-form and the radical of the terminal α-aminoglutaric acid compound is in the D-form, and pharmaceutically acceptable salts of such compounds having salt-forming groups.

14. Compounds of the formula Ic, according to patent claim 10, in which $R_2$ represents lower alkyl having up to 4 carbon atoms, or phenyl, $R_3$ represents especially hydrogen, and also lower alkyl having up to 4 carbon atoms, $R_7$ represents hydrogen, $R_8$ represents lower alkyl having from 1 to 4 carbon atoms or benzyl, which carries as substituent an oxycarbonyl, mercaptocarbonyl or aminocarbonyl group which is preferably bonded via the hetero atom to the lower alkyl or benzyl radical respectively, and which is itself bonded to alkyl or alkenyl having more than 10 and up to 50 carbon atoms optionally substituted by hydroxy, lower alkoxy or oxo, or to a tetracylic cycloalkylalkyl or cycloalkenylalkyl radical having more than 20 and up to 50 carbon atoms optionally substituted by hydroxy or lower alkoxy, which radicals may also be interrupted by an oxycarbonyl or iminocarbonyl group, $R_9$ represents amino, $R_{10}$ represents hydroxy and $R_{11}$ represents hydrogen, wherein in such compounds the radical of the hydroxyacetic acid with the grouping of the formula -CH($R_3$)- in which $R_3$ represents lower alkyl is in the D-form, the radical of the aminoacetic acid with the grouping of the formula -CH($R_8$)- is in the L-form and the radical of the terminal α-aminoglutaric acid compound is in the D-form, and pharmaceutically acceptable salts of such compounds having salt-forming groups.

15. Compounds of the formula Ic, according to patent claim 14, in which $R_2$ represents methyl or phenyl, $R_3$ represents hydrogen or methyl and the remaining radicals have the meanings given in patent claim 14.

16. Compounds of the formula Ic, according to patent claim 10, in which $R_2$ represents lower alkyl having up to 3 carbon atoms, or phenyl, $R_3$ represents hydrogen or methyl, $R_7$ represents hydrogen, $R_8$ represents lower alkyl having from 1 to 3 carbon atoms, which carries as substituent an oxycarbonyl, mercaptocarbonyl or aminocarbonyl group which is bonded via the hetero atom to the lower alkyl radical and which is itself bonded to alkyl or alkenyl having more than 10 and up to 50 carbon atoms optionally substituted by hydroxy, lower alkoxy or oxo, or to a tetracyclic cycloalkylalkyl or cycloalkenylalkyl radical having more than 20 and up to 50 carbon atoms optionally substituted by hydroxy or lower alkoxy, which radicals may also be interrupted by oxycarbonyl or iminocarbonyl, $R_9$ represents amino, lower alkylamino or carbamoyl-lower alkylamino or hydroxy, $R_{10}$ represents hydroxy, lower alkoxy, amino or carbamoyl-lower alkylamino, and $R_{11}$ represents hydrogen, wherein in such compounds the radical of the hydroxyacetic acid with the grouping -CH($R_3$)- in which $R_3$ represents methyl is in the D-form, the radical of the amino acid with the grouping -CH($R_8$)- is in the L-form and the radical of the terminal α-aminoglutaric acid compound is in the D-form, and pharmaceutically acceptable salts of such compounds having salt-forming groups.

17. Compounds of the formula Ic, according to patent claim 11, in which $R_2$ represents lower alkyl having up to 3 carbon atoms, $R_3$ represents hydrogen or methyl, $R_7$ represents hydrogen, $R_8$ represents lower alkyl having from 1 to 4 carbon atoms, which carries as substituent an oxycarbonyl, mercaptocarbonyl or aminocarbonyl group which is bonded via the hetero atom to the lower alkyl radical and which is itself bonded to

alkyl having more than 10 and up to 50 carbon atoms which may also be interrupted by iminocarbonyl, $R_9$ represents amino, hydroxy or lower alkoxy having from 1 to 3 carbon atoms, $R_{10}$ represents hydroxy, lower alkoxy having from 1 to 3 carbon atoms, or amino and $R_{11}$ represents hydrogen or carboxy, and pharmaceutically acceptable salts of such compounds having salt-forming groups.

18. Compounds of the formula Ic, according to claim 17, in which $R_8$ represents alkanoyloxymethyl or alkanoyloxyethyl having more than 10 and up to 30 carbon atoms in the alkyl moiety of the alkanoyl radical, $R_9$ represents amino and $R_{10}$ represents hydroxy, and pharmaceutically acceptable salts thereof.

19. N-acetyl-normuramyl-L-O-(N-behenoyl-glycyl)-seryl-D-isoglutamine or a pharmaceutically acceptable salt thereof according to claim 1.

20. N-acetyl-normuramyl-L-O-behenoyl-seryl-D-isoglutamine or a pharmaceutically acceptable salt thereof according to claim 1.

21. N-acetyl-muramyl-L-O-[N-(D,L-2-n-hexadecanoylamino-n-hexadecanoyl)-L-alanyl]-threonyl-D-isoglutamine or a pharmaceutically acceptable salt thereof according to claim 1.

22. N-acetyl-muramyl-L-S-stearoyl-cysteinyl-Disoglutamine or a pharmaceutically acceptable salt thereof according to claim 1.

23. A compound according to claim 1 selected from the group of the following compounds and their salts:

N-acetyl-normuramyl-L-O-[N-(12-hydroxy-cis-9-octadecenoyl)-glycyl]-seryl-D-isoglutamine,

N-benzoyl-normuramyl-L-O-stearoyl-seryl-D-isoglutamine,

N-acetyl-muramyl-L-O-(ω-n-stearoylamino-n-undecanoyl)threonyl-D-isoglutamine,

N-acetyl-muramyl-L-O-[N-(3-hydroxy-etio-cholenoyl)-6-amino-hexanoyl]-$_\gamma$-hydroxyprolyl-D-isoglutamine,

N-acetyl-muramyl-L-O-behenoyl-tyrosyl-D-isoglutamine,

N-acetyl-muramyl-L-($C_\gamma$)-[tetracosylamido-glycyl]glutamyl-D-isoglutamine,

N-acetyl-muramyl-L-[($C_\gamma$)-lauryl]-α-glutamyl-Disoglutamine,

N-acetyl-normuramyl-L-γ-stearoylamino-α-amino-butanoyl-D-isoglutamine,

N-acetyl-muramyl-L-(O-behenoyl-N-methyl)-seryl-Disoglutamine,

N-acetyl-muramyl-L-($N^\varepsilon$-palmitoyl)-lysyl-D-glutamic acid dimethyl ester.

24. N-benzoyl-normuramyl-L-(O-stearoyl)-seryl-Dglutamic acid dimethyl ester according to claim 1.

25. N-benzoyl-normuramyl-L-(O-behenoyl)-seryl-Disoglutamine or a pharmaceutically acceptable salt thereof according to claim 1.

26. N-acetyl-isomuramyl-L-(O-behenoyl)-seryl-Disoglutamine or a pharmaceutically acceptable salt thereof according to claim 1.

27. N-acetyl-muramyl-L-(O-behenoyl)-seryl-D-isoglutamine or a pharmaceutically acceptable salt thereof according to claim 1.

28. A compound according to claim 1 selected from the group of the following compounds and their salts:

N-acetyl-muramyl-D-(O-oleoyl)-seryl-D-isoglutamine,

N-acetyl-1,4,6-0-triacetyl-normuramyl-L-(O-behenoyl)seryl-D-isoglutamine,

N-acetyl-muramyl-L-(4-behenoyloxy)-prolyl-D-isoglut-aminyl-γ-L-alanine

and

N-acetyl-muramyl-L-(O-behenoyl)-seryl-D-γ-carboxy-α-glutamyl-glycinamide.

29. Compounds of the formula I and their salts according to any one of claims 1 to 28 for use in a method for the therapeutic treatment of the human or animal body.

30. The immunopotentiating compounds of the formula Ic according to any one of claims 10 to 22 and salts of such compounds having salt-forming groups.

31. Compounds of the formula I according to any one of claims 1 to 28 as immunomodulating agents.

32. Pharmaceutically acceptable preparations containing compounds of the formula Ic according to any one of claims 10 to 22 or pharmaceutically acceptable salts of such compounds having salt-forming groups.

33. Pharmaceutical preparations that contain pharmacologically active compounds of the formula I according to any one of claims 1 to 9 and 23 to 28 or pharmaceutically acceptable salts of such compounds having salt-forming groups.

34. Use of pharmacologically acceptable compounds according to any one of claims 1 to 28 and the salts thereof for the manufacture of pharmaceutical preparations.

35. Use of compounds of the formula Ic according to any one of claims 10 to 22 and pharmaceutically acceptable salts of such compounds having salt-forming groups as compounds having an immunopotentiating action.

36. Pharmaceutical preparations that contain at least one antibiotic and at least one pyranose derivative of the formula I according to any one of claims 1 to 28 and/or a salt of the same.

37. Feedstuffs and feedstuffs additives that contain at least one antibiotic and at least one muramyl peptide according to any one of claims 1 to 28 and/or a salt of the same.

38. Process for the manufacture of compounds of the formula I according to any one of claims 1 to 28 and salts of such compounds having salt-forming groups, characterised in that

a) in a compound of the formula I in which functional groups are optionally in protected form, at least one group that is readily replaceable by hydrogen is removed, or at least one functional group is converted to form the derivatives corresponding to the end products, or

b) in a compound of the formula I in which functional groups are optionally in protected form, in which $R_8$ represents a lower alkyl or phenyl-lower alkyl radical which may also be

bonded to $R_7$ and which carries a hydroxy-, mercapto-, amino- or carboxy-group optionally present in reactive form, and in which the remaining substituents have the meanings given in the claim to which this claim is appended, the radical $R_0$ is introduced, optionally in stages, by way of an oxycarbonyl, mercaptocarbonyl and/or iminocarbonyl group, or

c) in a compound of the formula

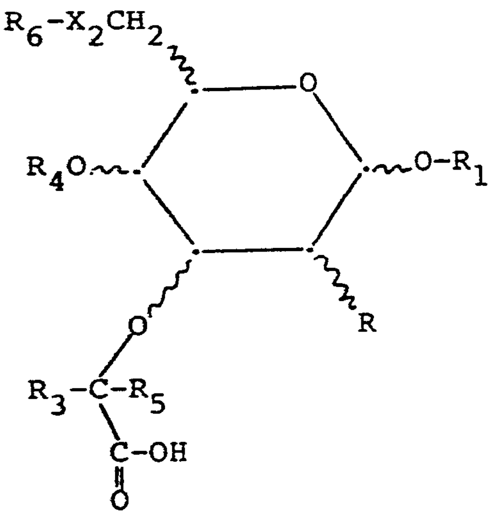

(IV)

in which the substituents have the meanings given in the claim to which this claim is appended, free functional groups optionally being in protected form, and in which the carboxy group may be in derivatised form, the carboxy group is amidated, optionally in stages, with an agent that transfers the radical of the formula

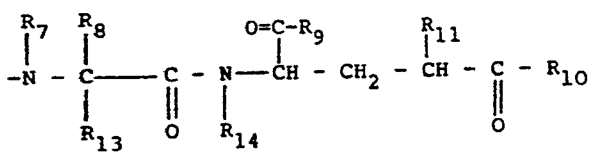

(IVa)

in which the substituents have the meanings given in the claim to which this claim is appended, functional groups optionally being in protected form, and, if necessary, protected functional groups in a resulting compound are converted into free functional groups, or

d) a compound of the formula

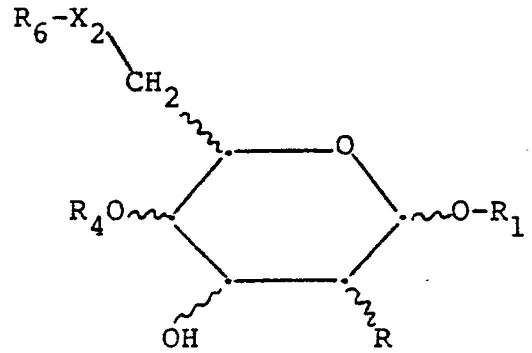

(VII),

in which the substituents have the meanings given in the claim to which this claim is appended and functional groups are preferably in protected form, or a reactive derivative thereof, is reacted with a compound of the formula

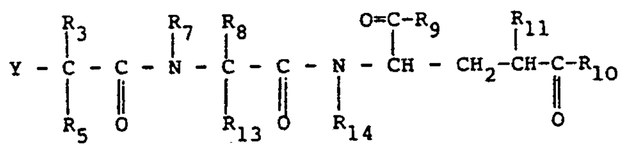

(VIII),

in which Y represents a reactive esterified hydroxy group and the remaining substituents have the meanings given in the claim to which this claim is appended, functional groups preferably being present in protected form, and, if necessary, protected functional groups in a resulting compound are converted into free functional groups and, if desired, a compound of the formula I obtainable in accordance with the process is converted into a different compound of the formula I and/or, if desired, a salt, obtainable in accordance with the process, of a compound of the formula I having a saltforming group is converted into the corresponding free compound of the formula I or into a different salt and/or, if desired, a compound of the formula I having a salt-forming group obtainable in accordance with the process is converted into a salt and/or, if desired, an isomeric mixture of compounds of the formula I obtainable in accordance with the process is separated into the isomeric compounds of the formula I. 39. Process for the manufacture of compounds of the formula I given in claim 1 according to any one of claims 2 and 10 to 22 or salts of such compounds having at least one salt-forming group, the substituents having the meanings given in the relevant claim, with the proviso that R represents a radical of the formula

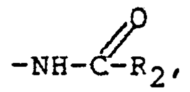

$R_1$, $R_4$ and $R_6$ each represents hydrogen and $X_2$ represents oxygen and that the pyranose moiety is derived from D-glucose, characterised in that, in a compound of the formula

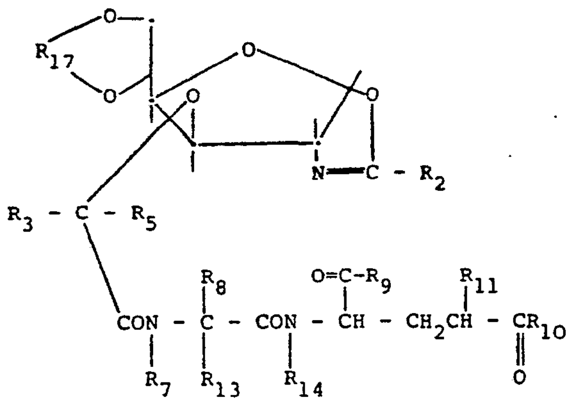

(XIII)

in which $R_{17}$ is an alkylidene or cycloalkylidene group and the other substituents have the meanings given above, the oxazoline ring and the dioxolane ring are cleaved by means of acid, and protecting groups that may be present are removed, and, if desired, a compound of the formula Ic obtainable in accordance with the process is converted into a different compound of the formula Ic and/or, if desired, a salt, obtainable in accordance with the process, of a compound of the formula Ic having a salt-forming group is converted into the corresponding free compound of the formula Ic or into a different salt and/or, if desired, a compound of the formula Ic having a saltforming group obtainable in accordance with the process is converted into a salt and/or, if desired, an isomeric mixture of compounds of the formula Ic obtainable in accordance with the process is separated into the isomeric compounds of the formula Ic.

40. The compounds obtainable by the processes of claims 38 and 39.

41. Peptides of the formula II

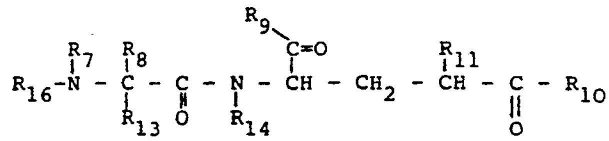

(II)

in which $R_{16}$ represents hydrogen or an aminoprotecting group, or the radical $R_{16}$

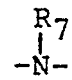

represents an activated amino group and the remaining substituents have the meanings given in any one of claims 1, 7, 10, 12 to 14 and 16, and salts of such compounds having salt-forming groups.

42. Process for the manufacture of compounds of the formula (II) according to claim 41 and their salts, characterised in that

a) an amino acid of the formula

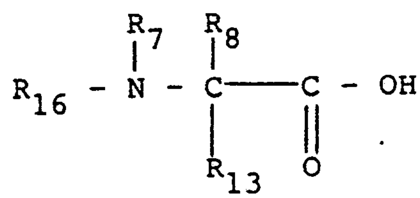

in which $R_{16}$ represents an amino-protecting group whilst the remaining substituents have the meanings given in claim 41, and the carboxyl group is optionally in activated form, other functional groups optionally being in protected form, is reacted with an amino acid of the formula

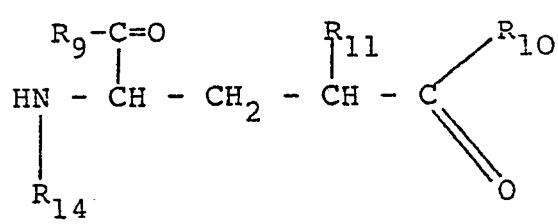

in which the

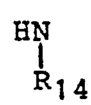

group is optionally in activated form and the remaining substituents have the meanings given in claim 41, functional groups present therein optionally being in protected form, and, if desired, at least one protecting group that may be

present is removed and/or, if desired, a resulting compound having at least one salt-forming group is converted into its salt, or

b) in a compound of the formula II, in which functional groups are optionally in protected form and in which $R_8$ represents a lower alkyl or phenyl-lower alkyl radical which may also be bonded to $R_7$ and which carries a hydroxy, mercapto, amino or carboxy group optionally present in reactive form, the radical $R_0$ defined according to any one of claims 1, 7, 10, 12 to 14 and 16 is introduced, optionally in stages, by way of the oxycarbonyl, mercaptocarbonyl or iminocarbonyl group, and, if desired, at least one protecting group that may be present is removed and/or, if desired, a resulting compound having at least one salt-forming group is converted into a salt, or

c) in a compound of the formula II in which functional groups are optionally in protected form, at least one group that is readily replaceable by hydrogen is removed, or at least one functional group is converted to form the derivatives corresponding to the end products.

43. The compounds and their salts obtainable by the process of claim 42.

44. Use of a peptide of the formula II according to claim 41 as an intermediate for the manufacture of compounds of the formula I according to any one of claims 1 to 16.

**Claims** for the contracting state: AT

1. Process for the manufacture of pyranose derivatives of the formula (I),

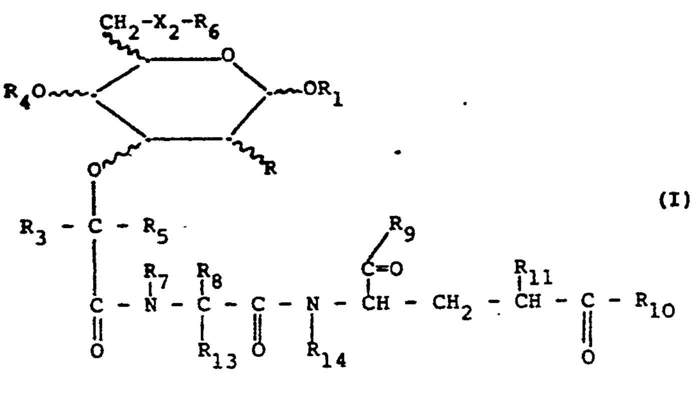

(I)

in which

R represents hydroxy, amino or a radical of the formula

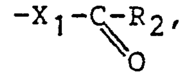

$X_1$ and $X_2$, independently of one another, each represents $NR_{15}$ or an oxygen atom, wherein $R_{15}$ represents hydrogen or lower alkyl,

$P_1$ and $R_4$, independently of one another, each represents hydrogen, acyl, or a hydroxyprotecting group,

$R_6$ represents hydrogen or acyl or, if $X_2$ represents an oxygen atom, a hydroxy-protecting group,

$R_2$ represents in each case optionally substituted alkyl, aryl or alkoxy,

$R_3$, $R_5$, $R_7$, $R_{13}$ and $P_{14}$, independently of one another, each represents hydrogen or lower alkyl, and

$R_8$ represents a lower alkyl or phenyl-lower alkyl radical, which may also be bonded to $R_7$ and which carries an oxycarbonyl, mercaptocarbonyl or aminocarbonyl group which is bonded to an optionally substituted aliphatic, cycloaliphaticaliphatic, cycloaliphatic or aromatic, hydrocarbon radical $R_0$, which latter may be interrupted by oxycarbonyl, mercaptocarbonyl and/or iminocarbonyl, and which must have more than 5 carbon atoms when $R_1$, $R_4$ and $R_6$ each represents hydrogen,

$R_9$ and $R_{10}$ each represents optionally etherified hydroxy or optionally substituted amino, and

$R_{11}$ represents hydrogen or a radical of the formula $-C(=O)-R_{12}$ (Ia), in which $R_{12}$ represents optionally etherified hydroxy or optionally substituted amino,

wherein radicals designated "lower" contain up to and including 7 carbon atoms,

and salts of compounds of the formula I having at least one salt-forming group, characterised in that,

a) in a compound of the formula I in which functional groups are optionally in protected form, at least one group that is readily replaceable by hydrogen is removed, or at least one functional group is converted to form the derivatives corresponding to the end products, or

b) in a compound of the formula I in which functional groups are optionally in protected form, in which $R_8$ represents a lower alkyl or phenyl-lower alkyl radical which may also be bonded to $R_7$ and which carries a hydroxy, mercapto, amino or carboxy group optionally present in reactive form, and in which the remaining substituents have the meanings given above, the radical $R_0$ is introduced, optionally in stages, by way of an oxycarbonyl, mercaptocarbonyl and/or iminocarbonyl group, and, if necessary, protected functional groups in a resulting compound are converted into the free functional groups, or

c) in a compound of the formula

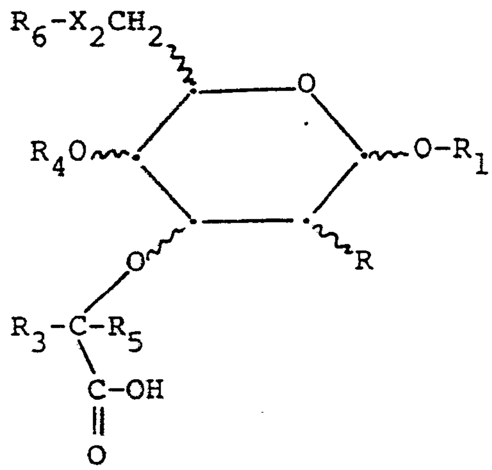

(IV)

in which the substituents have the meanings given above, free functional groups optionally being in protected form, and in which the carboxy group may be in derivatised form, the carboxy group is amidated, optionally in stages, with an agent that transfers the radical of the formula

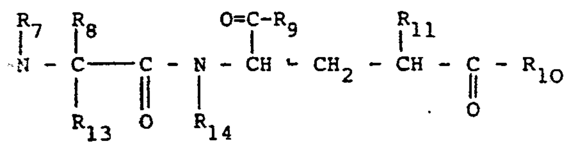

(IVa)

in which the substituents have the meanings given above, functional groups optionally being in protected form, and, if necessary, protected functional groups in a resulting compound are converted into free functional groups,

or

d) a compound of the formula

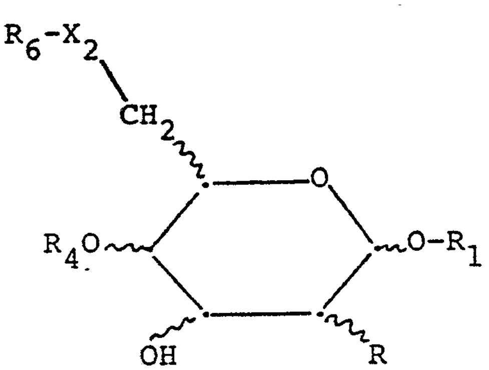

(VII),

in which the substituents have the meanings given above and functional groups are preferably in protected form, or a reactive derivative thereof, is reacted with a compound of the formula

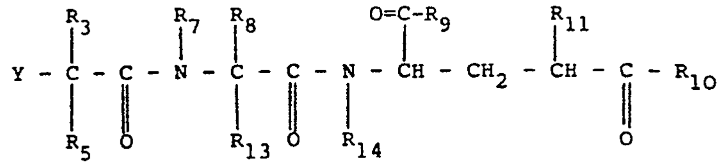

(VIII),

in which Y represents a reactive esterified hydroxy group and the remaining substituents have the meanings given above, functional groups preferably being present in protected form, and, if necessary, protected functional groups in a resulting compound are converted into free functional groups or,

e) for the manufacture of a compound of the formula I in which R represents a radical of the formula

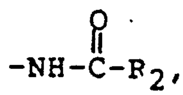

$R_1$, $R_4$ and $R_6$ each represents hydrogen and $X_2$ represents oxygen, and in which the pyranose moiety is derived from D-glucose and the remaining substituents have the meanings given above, or a salt of such a compound having at least one saltforming group, in a compound of the formula

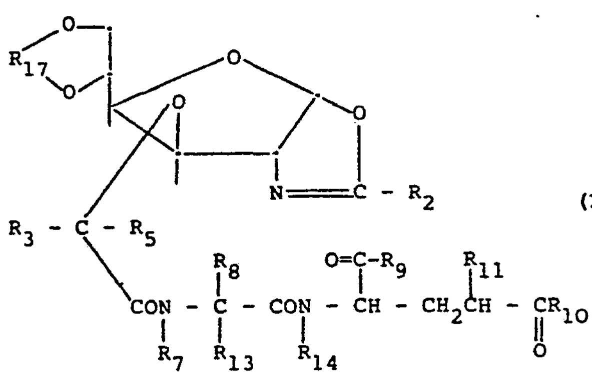

(XIII)

in which $R_{17}$ is an alkylidene or cycloalkylidene group and the other substituents have the meanings given above, the oxazoline ring and the dioxolane ring are cleaved by means of acid, and protecting groups that may be present are removed, and, if desired, a compound of the formula I obtainable in accordance with the process is converted into a different compound of

the formula I and/or, if desired, a salt, obtainable in accordance with the process, of a compound of the formula I having a salt-forming group is converted into the corresponding free compound of the formula I or into a different salt and/or, if desired, a compound of the formula I having a salt-forming group obtainable in accordance with the process is converted into a salt and/or, if desired, an isomeric mixture of compounds of the formula I obtainable in accordance with the process is separated into the isomeric compounds of the formula I.

2. Process according to claim 1, characterised in that compounds of the formula I are manufactured, the pyranose moiety of which is derived from D-glucose.

3. Process according to claim 1, characterised in that compounds of the formula I are manufactured, the pyranose moiety of which is derived from D-mannose or D-galactose.

4. Process according to any one of claims 1 to 3, characterised in that compounds of the formula I are manufactured which, in cases of asymmetrical substitution, have the (D)-configuration at the C-$NR_{14}$.

5. Process according to claim 4, characterised in that compounds of the formula I are manufactured which, in cases of asymmetrical substitution, have the (D)configuration at the C-$R_3$ and the (L)-configuration at the C-$R_8$.

6. Process according to any one of claims 1 to 5, characterised in that compounds of the formula I are manufactured in which R represents a radical of the formula

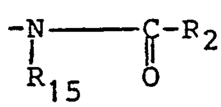

and $X_2$ represents an oxygen atom.

7. Process according to any one of claims 1 to 6, characterised in that compounds of the formula I are manufactured in which $R_5$, $R_{13}$, $R_{14}$ and $R_{15}$ each represents hydrogen.

8. Process according to any one of claims 1 to 7, characterised in that compounds of the formula I are manufactured in which $R_1$, $R_4$ and $R_6$ each represents hydrogen, optionally unsaturated alkanoyl having from 2 to 25 carbon atoms, or optionally substituted benzoyl.

9. Process according to any one of claims 1 to 7, characterised in that compounds of the formula I are manufactured in which $R_6$ represents the radical

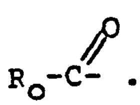

10. Process according to claim 1, characterised in that peptide derivatives of glucosamine compounds of the formula Ic

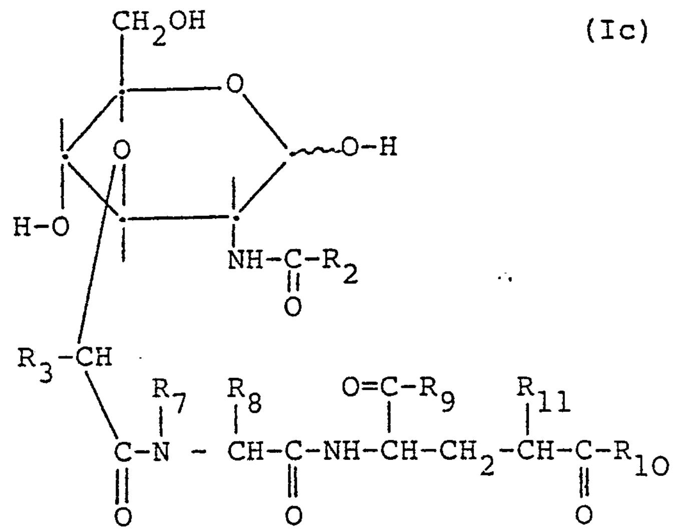

(Ic)

in which

$R_2$ represents optionally substituted lower alkyl, phenyl, lower alkoxy or phenyl-lower alkoxy,

$R_3$ represents hydrogen or lower alkyl,

$R_7$ represents hydrogen or lower alkyl, and

$R_8$ represents a lower alkyl or phenyl-lower alkyl radical, which may also be bonded to $R_7$ and which carries an oxycarbonyl, mercaptocarbonyl or aminocarbonyl group which is bonded to an optionally substituted long-chain aliphatic or cycloaliphatic-aliphatic hydrocarbon radical, which latter may be interrupted by oxycarbonyl, mercaptocarbonyl and/or iminocarbonyl,

$R_9$ and $R_{10}$ each represents optionally etherified hydroxy or optionally substituted amino, and

$R_{11}$ represents hydrogen or a radical of the formula -C(=0)-$R_{12}$ (Ia), in which $R_{12}$ represents optionally etherified hydroxy or optionally substituted amino,

or salts of compounds of the formula Ic having at least one salt-forming group, are manufactured.

11. Process according to claim 10, characterised in that compounds of the formula Ic in which the radical of the hydroxyacetic acid with the grouping of the formula -CH($R_3$)- in which $R_3$ represents lower alkyl is in the D-form, the radical of the aminoacetic acid with the grouping of the formula -CH($R_8$)- is in the Lform, and the radical of the terminal α-aminoglutaric acid compound is in the D-form, or salts of such compounds having at least one salt-forming group, are manufactured.

12. Process according to claim 10, characterised in that compounds of the formula Ic in which $R_2$ represents lower alkyl or lower alkoxy each optionally substituted by hydroxy, lower alkoxy, lower alkanoyloxy or halogen, or phenyl or phenyl-lower alkoxy each optionally substituted by lower alkyl, hydroxy, lower alkoxy, lower alkanoyloxy or halogen, $R_3$ represents hydrogen or lower alkyl, $R_7$ represents hydrogen

or lower alkyl, and $R_8$ represents a lower alkyl or phenyl-lower alkyl radical which carries as substituent an oxycarbonyl, mercaptocarbonyl or aminocarbonyl group, which is itself in turn bonded to an alkyl or alkenyl radical having more than 6 and up to 90 carbon atoms optionally substituted by hydroxy, lower alkoxy, lower alkanoyloxy, oxo or halogen, or to a cycloaliphatically substituted alkyl or alkenyl radical having, for example, up to 30 carbon atoms, and in which the alkyl, alkenyl and cycloaliphatically substituted alkyl or alkenyl radicals may also be interrupted by one or two oxycarbonyl or iminocarbonyl groups, $R_{11}$ represents hydrogen or a radical of the formula Ia, and each of the radicals $R_9$, $R_{10}$ and $R_{12}$ represents hydroxy, lower alkoxy, amino, or amino which is substituted by lower alkyl optionally containing carboxy, lower alkoxycarbonyl or carbamoyl, wherein in such compounds, for example the radical of the hydroxyacetic acid with the grouping of the formula -CH($R_3$)- in which $R_3$ represents lower alkyl is in the D-form, the radical of the aminoacetic acid with the grouping of the formula -CH($R_8$)- is in the Lform, and the radical of the terminal α-aminoglutaric acid compound is in the D-form, and salts of such compounds having at least one salt-forming group, are manufactured.

13. Process according to claim 10, characterised in that compounds of the formula Ic in which $R_2$ represents lower alkyl having up to 4 carbon atoms, or phenyl, $R_3$ represents hydrogen or lower alkyl having up to 4 carbon atoms, $R_7$ represents hydrogen and $R_8$ represents a lower alkyl radical having from 1 to 4 carbon atoms or a phenyl-lower alkyl radical, which carries as substituent an oxycarbonyl, mercaptocarbonyl or aminocarbonyl group which is itself bonded to an alkyl or alkenyl radical having more than 10 and up to 50 carbon atoms optionally substituted by hydroxy, lower alkoxy, oxo or halogen, or to a tetracyclic cycloalkylalkyl or cycloalkylalkenyl radical having more than 20 and up to 50 carbon atoms optionally substituted by hydroxy, lower alkoxy, oxo or halogen, wherein such radicals may also be interrupted by 1 or 2 oxycarbonyl or iminocarbonyl groups, $R_9$ represents amino, or lower alkylamino optionally containing carboxy or carbamoyl, $R_{10}$ represents hydroxy and $R_{11}$ represents hydrogen, wherein in such compounds, for example the radical of the hydroxyacetic acid with the grouping of the formula -CH($R_3$)- in which $R_3$ represents lower alkyl is in the D-form, the radical of the aminoacetic acid with the grouping of the formula -CH($R_8$)- is in the L-form and the radical of the terminal α-aminoglutaric acid compound is in the D-form, and pharmaceutically acceptable salts of such compounds having at least one salt-forming group, are manufactured.

14. Process according to claim 10, characterised in that compounds of the formula Ic in which $R_2$ represents lower alkyl having up to 4 carbon atoms, or phenyl, $R_3$ represents especially hydrogen, and also lower alkyl having up to 4 carbon atoms, $R_7$ represents hydrogen, $R_8$ represents lower alkyl having from 1 to 4 carbon atoms or benzyl, which carries as substituent an oxycarbonyl, mercaptocarbonyl or aminocarbonyl group which is preferably bonded via the hetero atom to the lower alkyl or benzyl radical respectively, and which is itself bonded to alkyl or alkenyl having more than 10 and up to 50 carbon atoms optionally substituted by hydroxy, lower alkoxy or oxo, or to a tetracylic cycloalkylalkyl or cycloalkenylalkyl radical having more than 20 and up to 50 carbon atoms optionally substituted by hydroxy or lower alkoxy, which radicals may also be interrupted by an oxycarbonyl or iminocarbonyl group, $R_9$ represents amino, $R_{10}$ represents hydroxy and $R_{11}$ represents hydrogen, wherein in such compounds the radical of the hydroxyacetic acid with the grouping of the formula -CH($R_3$)- in which $R_3$ represents lower alkyl is in the D-form, the radical of the aminoacetic acid with the grouping of the formula -CH($R_8$)- is in the L-form and the radical of the terminal α-aminoglutaric acid compound is in the D-form, or pharmaceutically acceptable salts of such compounds having at least one salt-forming group, are manufactured.

15. Process according to claim 14, characterised in that compounds of the formula Ic in which $R_2$ represents methyl or phenyl, $R_3$ represents hydrogen or methyl and the remaining radicals have the meanings given in claim 14 are manufactured.

16. Process according to claim 10, characterised in that compounds of the formula Ic in which $R_2$ represents lower alkyl having up to 3 carbon atoms, or phenyl, $R_3$ represents hydrogen or methyl, $R_7$ represents hydrogen, $R_8$ represents lower alkyl having from 1 to 3 carbon atoms, which carries as substituent an oxycarbonyl, mercaptocarbonyl or aminocarbonyl group which is bonded via the hetero atom to the lower alkyl radical and which is itself bonded to alkyl or alkenyl having more than 10 and up to 50 carbon atoms optionally substituted by hydroxy, lower alkoxy or oxo, or to a tetracyclic cycloalkylalkyl or cycloalkenylalkyl radical having more than 20 and up to 50 carbon atoms optionally substituted by hydroxy or lower alkoxy, which radicals may also be interrupted by oxycarbonyl or iminocarbonyl, $R_9$ represents amino, lower alkylamino or carbamoyl-lower alkylamino or hydroxy, $R_{10}$ represents hydroxy, lower alkoxy, amino or carbamoyl-lower alkylamino, and $R_{11}$ represents hydrogen, wherein in such compounds the radical of the hydroxyacetic acid with the grouping -CH($R_3$)- in which $R_3$ represents methyl is in the D-form, the radical of the amino acid with the grouping -CH($R_8$)-is in the L-form and the radical of the terminal α-aminoglutaric acid compound is in the D-form, and pharmaceutically acceptable salts of such compounds having at least one salt-forming group, are manufactured.

17. Process according to claim 11,

characterised in that compounds of the formula Ic in which $R_2$ represents lower alkyl having up to 3 carbon atoms, $R_3$ represents hydrogen or methyl, $R_7$ represents hydrogen, $R_8$ represents lower alkyl having from 1 to 4 carbon atoms, which carries as substituent an oxycarbonyl, mercaptocarbonyl or aminocarbonyl group which is bonded via the hetero atom to the lower alkyl radical and which is itself bonded to alkyl having more than 10 and up to 50 carbon atoms which may also be interrupted by iminocarbonyl, $R_9$ represents amino, hydroxy or lower alkoxy having from 1 to 3 carbon atoms, $R_{10}$ represents hydroxy, lower alkoxy having from 1 to 3 carbon atoms, or amino and $R_{11}$ represents hydrogen or carboxy, or pharmaceutically acceptable salts of such compounds having at least one salt-forming group, are manufactured.

18. Process according to claim 17, characterised in that compounds of the formula Ic in which $R_8$ represents alkanoyloxymethyl or alkanoyloxyethyl having more than 10 and up to 30 carbon atoms in the alkyl moiety of the alkanoyl radical, $R_9$ represents amino and $R_{10}$ represents hydroxy, or pharmaceutically acceptable salts thereof, are manufactured.

19. Process according to claim 10, characterised in that N-acetyl-normuramyl-L-O-(N-behenoyl-glycyl)-seryl-D-isoglutamine, or a pharmaceutically acceptable salt thereof, is manufactured.

20. Process according to claim 10, characterised in that N-acetyl-normuramyl-L-O-behenoyl-seryl-Disoglutamine, or a pharmaceutically acceptable salt thereof, is manufactured.

21. Process according to claim 10, characterised in that N-acetyl-muramyl-L-O-[N-(D,L-2-n-hexadecanoyl-amino-n-hexadecanoyl)-L-alanyl]-threonyl-Disoglutamine, or a pharmaceutically acceptable salt thereof, is manufactured.

22. Process according to claim 10, characterised in that N-acetyl-muramyl-L-S-stearoyl-cysteinyl-Disoglutamine, or a pharmaceutically acceptable salt thereof, is manufactured.

23. Process according to claim 10, characterised in that a compound selected from the group of the following compounds and their salts is manufactured:

N-acetyl-normuramyl-L-O-[N-(12-hydroxy-cis-9-octadecenoyl)-glycyl]-seryl-D-isoglutamine,

N-benzoyl-norm-uramyl-L-O-stearoyl-seryl-D-isoglutamine,

N-acetyl-muramyl-L-O-(ω-n-stearoylamino-n-undecanoyl)threonyl-D-isoglutamine,

N-acetyl-muramyl-L-O-[N-(3-hydroxy-etio-cholenoyl)-6-amino-hexanoyl]-γ-hydroxyprolyl-D-isoglutamine,

N-acetyl-muramyl-L-O-behenoyl-tyrosyl-D-isoglutamine,

N-acetyl-muramyl-L-($C_\gamma$)-[tetracosylamido-glycyl]glutamyl-D-isoglutamine,

N-acetyl-muramyl-L-[($C_\gamma$)-lauryl]-α-glutamyl-Disoglutamine,

N-acetyl-normuramyl-L-γ-stearoylamino-α-amino-butanoyl-D-isoglutamine,

N-acetyl-muramyl-L-(O-behenoyl-N-methyl)-seryl-Disoglutamine,

N-acetyl-muramyl-L-($N^\varepsilon$-palmitoyl)-lysyl-D-glutamic acid dimethyl ester.

24. Process according to claim 10, characterised in that N-benzoyl-normuramyl-L-(O-stearoyl)-seryl-Dglutamic acid dimethyl ester is manufactured.

25. Process according to claim 10, characterised in that N-benzoyl-normuramyl-L-(O-behenoyl)-seryl-Disoglutamine, or a pharmaceutically acceptable salt thereof, is manufactured.

26. Process according to claim 10, characterised in that N-acetyl-isomuramyl-L-(O-behenoyl)-seryl-Disoglutamine, or a pharmaceutically acceptable salt thereof, is manufactured.

27. Process according to claim 10, characterised in that N-acetyl-muramyl-L-(O-behenoyl)-seryl-D-isoglutamine, or a pharmaceutically acceptable salt thereof, is manufactured.

28. Process according to claim 10, characterised in that a compound selected from the group of the following compounds and their salts is manufactured:

N-acetyl-muramyl-D-(O-oleoyl)-seryl-D-isoglutamine,

N-acetyl-1,4,6-0-triacetyl-normuramyl-L-(O-behenoyl)seryl-D-isoglutamine,

N-acetyl-muramyl-L-(4-behenoyloxy)-prolyl-D-isoglut-aminyl-γ-L-alanine and

N-acetyl-muramyl-L-(O-behenoyl)-seryl-D-γ-carboxy-α-glutamyl-glycinamide.

29. Process for the manufacture of peptides of the formula II

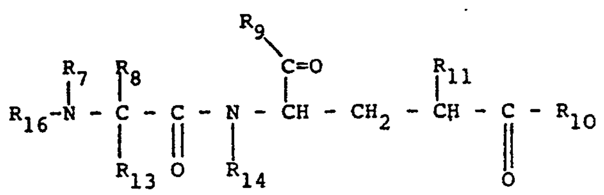

(II)

in which $R_{16}$ represents hydrogen or an aminoprotecting group and the remaining substituents have the meanings given in any one of claims 1, 7, 10, 12 to 14 and 16 to 18, and salts of such compounds having salt-forming groups, characterised in that

a) an amino acid of the formula

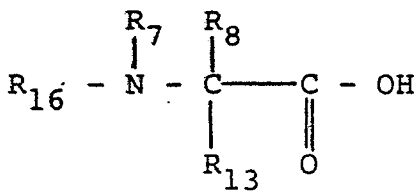

in which $R_{16}$ represents an amino-protecting group whilst the remaining substituents have the meanings given above, and the carboxyl group is optionally in activated form, other functional groups optionally being in protected form, is reacted with an amino acid of the formula

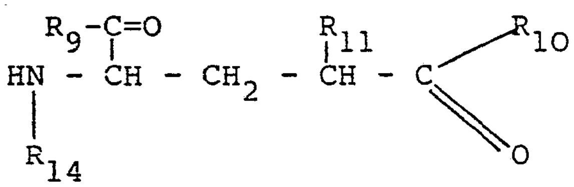

in which the

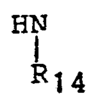

group is optionally in activated form and the remaining substituents have the meanings given above, functional groups present therein optionally being in protected form, and, if desired, at least one protecting group that may be present is removed and/or, if desired, a resulting compound having at least one salt-forming group is converted into its salt, or

b) in a compound of the formula II, in which functional groups are optionally in protected form and in which $R_8$ represents a lower alkyl or phenyl-lower alkyl radical which may also be bonded to $R_7$ and which carries a hydroxy, mercapto, amino or carboxy group optionally present in reactive form, the radical $R_o$ defined according to any one of claims 1, 10, 12 to 14 and 16 to 18 is introduced, optionally in stages, by way of the oxycarbonyl, mercaptocarbonyl or iminocarbonyl group, and, if desired, at least one protecting group that may be present is removed and/or, if desired, a resulting compound having at least one salt-forming group is converted into a salt, or

c) in a compound of the formula II in which functional groups are optionally in protected form, at least one group that is readily replaceable by hydrogen is removed, or at least one functional group is converted to form the derivatives corresponding to the end products.

30. Process according to claim 29, characterised in that the starting materials are so selected that there manufactured a compound of the formula II that has (L)-configuration at the C-$R_8$ and the (D)configuration at the C-$NR_{14}$, or a salt of such a compound having a salt-forming group.

**Revendications** pour les Etats contractants: BE, CH, DE, FR, IT, LI, LU, NL, SE

1 - Dérivés de pyranose de formule I

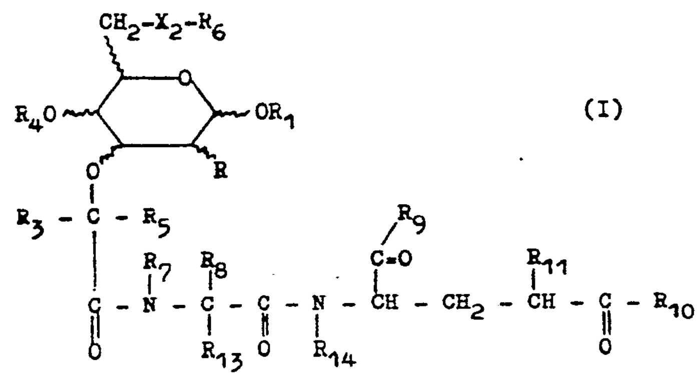

(I)

dans laquelle R représente un hydroxy, amino ou un reste de formule

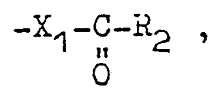

$X_1$ et $X_2$ représentent indépendamment l'un de l'autre $NR_{15}$ ou un atome d'oxygène, dans lequel $R_{15}$ est l'oxygéne ou un alkyle inférieur, $R_1$ et $R_4$ sont indépendamment l'un de l'autre un hydrogène, acyle ou groupe protecteur d'hydroxy, $R_6$ est un hydrogène, acyle ou, si $X_2$ est un atome d'oxygène, un groupe protecteur d'hydroxy, $R_2$ est chaque fois un alkyle le cas echéant substitué, aryle ou alcoxy, $R_3$, $R_5$, $R_7$, $R_{13}$ et $R_{14}$ sont indépendamment l'un de l'autre de l'hydrogène ou um alkyle inférieur et $R_8$ est un reste alkyle inférieur ou phénylalkyle inférieur qui peut aussi être lié, à $R_7$, et qui porte un groupe oxycarbonyle, mercaptocarbonyle ou aminocarbonyle, qui est lié ü un reste hydrocarboné $R_o$ le cas échéant substitué aliphatique cycloaliphatique-aliphatique, cycloaliphatique ou aromatique, ce dernier pouvant être interrompu par un oxycarbonyle, mercaptocarbonyle et/ou iminocarbonyle et qui doit présenter plus de 5 atomes de carbone, quand

$R_1$, $R_4$ et $R_6$ représentent l'hydrogène, dans laquelle les restes $R_9$ et $R_{10}$ sont un hydroxy le cas

échéant éthérifié ou amino le cas échéant substitué, et $R_{11}$ est l'hydrogène ou un reste de formule -C(=O)-$R_{12}$ (Ia), dans laquelle $R_{12}$ est um hydroxy le cas échéant éthérifié ou amino le cas échéant substitué, dans lesquelles les restes qualifiés d' "inférieurs" contiennent jusqu'à 7 atomes de carbone, et des sels de composés de formule I avec au moins un groupe formateur de sel.

2 - Composés selon la revendication 1, dont la partie pyranose dérive du D-glucose.

3 - Composés selon la revendication 1, dont la partie pyranose dérive du D-mannose ou du D-galactose.

4 - Composés de formule I selon l'une des revendications 1-3, qui en cas de substitution asymétrique sur le C-$NR_{14}$ présentent la configuration (D).

5 - Composés de formule I selon la revendication 4, qui en cas de substitution asymétrique sur le C-$R_3$ et le C-$R_8$ présentent respectivement la configuration (D) et (L).

6 - Composés de formule I selon l'une des revendications 1-5, dans lesquels R est un reste de formule

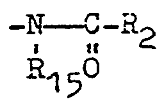

et $X_2$ est un atome d'oxygène.

7 - Composés de formule I selon l'une des revendications 1-6, dans lesquels $R_5$, $R_{13}$, $R_{14}$ et $R_{15}$ sont l'hydrogène.

8 - Composés de formule I selon l'une des revendications 1-7, dans lesquels $R_1$, $R_4$ et $R_6$ sont l'hydrogène, le cas échéant un alcanoyle insaturé avec 2-25 atomes de C ou un benzoyle le cas échéant substitué.

9 - Composés de formule I selon l'une des revendications 1-7, dans lesquels $R_6$ est le reste

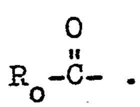

10 - Dérivés peptidiques selon la revendication 1 de composés glucosamine de formule

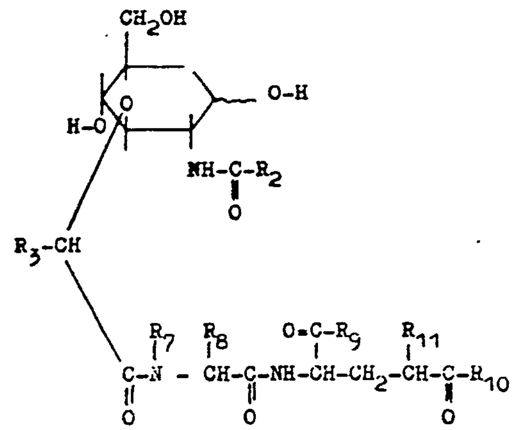

(Ic)

dans laquelle $R_2$ est un alkyle inférieur le cas échéant substitue, phényle, alcoxy inférieur ou phénylalcoxy in férieur $R_3$ est l'hydrgène ou alkyle inférieur, $R_7$ l'hydrogène ou alkyle inférieur et $R_8$ un reste alkyle inférieur ou phénylalkyl inférieur qui peut aussi être lié à $R_7$ et qui porte un groupe oxycarbonyle, mercaptocarbonyle ou aminocarbonyle, qui est lié avec un reste hydrocarboné aliphatique à longue chaîne, le cas échéant substitué ou cycloaliphatique-aliphatique ce dernier pouvant être interrompu par un oxycarbonyle, mercaptocarbonyle et/ou iminocarbonyle, dans laquelle $R_9$ et $R_{10}$ sont un hydroxy le cas échéant éthérifié ou amino le cas échéant substitue, et $R_{11}$ est l'hydrogene ou un reste de formule -C(=O)-$R_{12}$ (Ia) dans laquelle $R_{12}$ est un hydroxy le cas échéant etherifie ou amino le cas échéant substitué, et des sels de composés de formule $I_c$ avec au moins un groupe formateur de sel.

11 - Composés de formule Ic selon la revendication 10 caractérisé en ce que le reste de l'acide hydroxyacétique avec le groupement de formule -CH($R_3$)-, dans lequel $R_3$ représente un alkyle inférieur, est dans la forme D, le reste anino-acétique avec le groupement de formule -CH($R_8$)- est dans la forme L, et le reste du composé acide α-aminoglutarique terminal est sous la forme D ou les sels de tels composés avec des groupes formateurs de sels.

12 - Composés de formule Ic selon la revendication 10 dans lesquels $R_2$ est un alkyle inférieur substitué le cas échéant par un hydroxy, alcoxy inferieur, alcanoyloxy inférieur ou halogène ou un alcoxy inférieur, ou un phényle substitué le cas échéant par un alkyle inférieur, hydroxy, alcoxy inférieur, alcanoyloxy inférieur ou halogène ou un phénylalcoxy inferieur, $R_3$ est l'hydrogène ou un alkyle inférieur, $R_7$ est l'hydrogène ou un

alkyle inférieur et $R_8$ est un reste alkyle inférieur ou phénylalkyle inférieur, qui porte comme substituant un groupe oxycarbonyle, mercaptocarbonyle ou aminocarbonyle, qui à son tour est lié à un reste alkyle ou alcényle substitué le cas échéant par un hydroxy alcoxy inférieur, alcanoyloxy inférieur oxo ou halogène avec plus de 6 et jusqu'à 90 atomes de carbone ou à un reste alkyle ou alcényle substitué cycloaliphatique avec par exemple jusqu'à 30 atomes de carbone, et dans lesquels les restes alkyle, alcényle et alkyle ou alcényle substitués cycloaliphatique peuvent aussi être interrompus par un ou deux groupes oxycarbonyle ou imidocarbonyle, $R_{11}$ est l'hydrogène ou le reste de la formule Ia, et chacun des restes $R_9$, $R_{10}$ et $R_{12}$ est un hydroxy, alcoxy inférieur, amino ou amino substitué le cas échéant par un alkyle inférieur renfermant un carbamoyle, alcoxycarbonyle inférieur ou carboxy, et où dans de tels composés, par exemple le reste de l'acide hydroxy acétique avec le groupement de formule -CH($R_3$)-, dans lequel $R_3$ est un alkyle inferieur, est dans la forme D, le reste de l'acide aminoacétique avec le groupement de formule -CH($R_8$)-, est dans la forme L, et le reste du composé acide α-aminoglutarique terminal est dans la forme D, ainsi que les sels de tels composés avec des groupes formateurs de sel.

13 - Composés de formule Ic selon la revendication 10 dans lesquels $R_2$ est un alkyle inférieur avec jusqu'à 4 atomes de carbone ou un phényle, $R_3$ est l'hydrogène ou un alkyle inférieur avec jusqu'à 4 atomes de carbone, $R_7$ est l'hydrogène et $R_8$ est un reste alkyle inférieur avec 1-4 atomes de carbone ou un reste phényl alkyle inférieur, qui porte comme substituant un groupeoxycarbonyle, mercaptocarbonyle ou aminocarbonyle qui à son tour est lié à un alkyle ou alcényle substitué le cas échéant par un hydroxv, alcoxy inférieur, oxo ou halogène avec plus de 10 et jusqu'à 50 atomes de carbone ou à un cycloalkyl-alkyle ou alcényl tétracyclique substitué le cas échéant par un hydroxy, alcoxy inférieur, oxo ou halogène avec plus de 20 et jusqu'à 50 atomes de carbone, et où de tels restes peuvent aussi être interrompus par 1 ou 2 groupes oxycarbonyle ou iminocarbonyle, $R_9$ est un amino ou alkylamino inférieur renfermant le cas échéant un carboxy ou carbamoyle, $R_{10}$ est un hydroxy et $R_{11}$ est l'hydrogène, où dans de tels composés, par exemple le reste de l'acide hydroxyacétique avec le groupement de formule -CH($R_3$)-, dans lequel $R_3$ est un alkyle inférieur, est dans la forme D, le reste de l'acide aminoacétique avec le groupement de formule -CH($R_8$)-, est dans la forme L, et le reste du composé acide α-aminoglutarique terminal est dans la forme D, et les sels pharmaceutiquement utilisables de tels composés avec des groupes formateurs de sels.

14 - Composés de formule Ic selon la revendication 10 dans lesquels $R_2$ est un alkyle inférieur avec jusqu,à 4 atomes de carbone, ou un phényle, $R_3$ est l'hydrogène, ou encore un alkyle inférieur avec jusqu'à 4 atomes de carbone, $R_7$ est l'hydrogène, $R_8$ est un reste alkyle inférieur avec 1-4 atomes de carbone ou benzyle qui portent comme substituant un groupe oxycarbonyle, mercaptocarbonyle ou aminocarbonyle, qui est lié de préférence par l'intermédiaire de l'hétéroatome au reste alkyle inférieur ou benzyle, et qui à son tour est lié à un alkyle ou alcényle substitué le cas échéant par un hydroxy, alcoxy inférieur ou oxo avec plus de 10 et jusqu'à 50 atomes de carbone ou à un reste cycloalkylalkyle ou cycloalcénylalkyle tétracyclique substi- tué le cas échéant par un hydroxy ou alcoxy inférieur avec plus de 20 et jusqu'à 50 atomes de carbone, ces restes peuvent aussi être interrompus psr un groupe oxycarbonyle ou iminocarbonyle, $R_9$ est un amino, $R_{10}$ est un hydroxy et $R_{11}$ est l'hydrogène, où dans de tels composés le reste de l'acide hydroxyacétique avec le groupement de formule -CH($R_3$)-, dsns lequel $R_3$ est un alkyle inférieur, est dans la forme D, le reste de l'acide aminoacétique avec le groupement de formule - CH($R_8$)-est dans la forme L et le reste du composé acide α-aminoglutarique terminal est dans la forme D, et les sels pharmaceutiquement utilisables de tels composés avec des groupes formateurs de sels.

15 - Composés de formule Ic selon la revendication 14, dans lesquels $R_2$ est méthyle ou phényle, $R_3$ est l'hydrogène ou méthyle et les autres restes ont les significations données dans la revendication 14.

16 - Composés de formule Ic selon la revendication 10, dans lesquels $R_2$ est un alkyle inférieur avec jusqu'à 3 atomes de carbone ou un phényle, $R_3$ est l'hydrogène ou un méthyle, $R_7$ est l'hydrogène, $R_8$ est un alkyle inférieur avec 1-3 atomes de carbone, qui porte comme substituant un groupe oxycarbonyle, mercaptocarbonyle ou aminocarbonyle, qui est lié par l'intermédiaire de l'hétéroatome au reste alkyle inférieur, et qui à son tour est lié à un alkyle ou alcényle substitué le cas échéant par un hydroxy, alcoxy inférieur ou oxo avec plus de 10 et jusqu'à 50 atomes de carbone ou à un reste cycloalkylalkyle ou cycloalcénylalkyl tétracyclique substitué le cas échéant par un hydroxy ou alcoxy inférieur avec plus de 20 et jusqu'à 50 atomes de carbone, ces restes peuvent aussi être interrompus par un groupe oxycarbonyle ou iminocarbonyle, $R_9$ est un amino, alkylamino inférieur ou carbamoylalkylamino ou hydroxy, $R_{10}$ est un hydroxy, et $R_{11}$ est l'hydrogène, où dans de tels composés le reste de l'acide hydroxyacétique avec le groupement de formule -CH($R_3$)-, dans lequel $R_3$ est le methyle, est dans la forme D, le reste de l'acide aminoacétique avec le groupement de formule -CH($R_8$)- est dans la forme L et le reste du composé acide α-aminoglutarique terminal est dans la forme D et les sels pharmaceutiquement utilisables de tels composés avec des groupes formateurs de sel.

17 - Composés de formule Ic selon la revendication 11 dans lesquels $R_2$ est un alkyle inférieur avec jusqu'à 3 atomes de carbone, $R_3$ est l'hydrogène ou un méthyle, $R_7$ est l'hydrogène, $R_8$ est un alkyle inférieur avec 1-4 atomes de carbone

qui porte comme substituant un groupe oxycarbonyle, mercaptocarbonyle ou aminocarbonyle qui est lié par l'intermédiaire de l'hétéroatome au reste alkyle inférieur et qui à son tour est lié à un alkyle avec plus de 10 et jusqu'à 50 atomes de carbone, qui peut aussi être interrompu par un iminocarbonyle, $R_9$ est un amino, hydroxy ou alcoxy inférieur avec 1-3 atomes de carbone, $R_{10}$ est un hydroxy, alcoxy inférieur avec 1-3 atomes de carbone ou un amino et $R_{11}$ est l'hydrogène ou un carboxy et les sels pharmaceutiquement utilisables de tels composés avec des groupes formateurs de sel.

18 - Composés de formule Ic selon la revendication 17 dans lesquels $R_8$ est un alcanoyloxyméthyle ou alcanoyloxyéthyle avec plus de 10 et jusqu'à 30 atomes de carbone dans la partie alkyle du reste alcanoyle, $R_9$ est amino et $R_{10}$ est un hydroxy, et leurs sels pharmaceutiquement utilisables.

19 - N-acétyl-normuramyl-L-O-(N-béhénoylglycyl)-séryl-D-isoglutamine ou un de ses sels pharmaceutiquement utilisable selon la revendication 1.

20 - N-acétyl-normuramyl-L-O-béhénoyl-séryl-D-isoglutamine ou un de ses sels pharmaceutiquement utilisable selon la revendication 1.

21 - N-acétyl-muramyl-L-O-[N-(D,L-2-n-hexadécanoyl-amino-n-hexadécanoyl)-L-alanyl]-thréonyl-Disoglutamine ou un de ses sels pharmaceutiquement utilisable selon la revendication 1.

22 - N-acétyl-muramyl-L-S-stéaroyl-cystéinyl-D-isoglutamine ou un de ses sels pharmaceutiquement utilisable selon la revendication 1.

23 - Un composé selon la revendication 1, choisi dans le groupe des composés suivants et leurs sels

N-acétyl-normuramyl-L-O-[N-(12-hydroxy-cis-9-octadécénoyl)-glycyl]-séryl-D-isoglutamine,

N-benzoyl-normuramyl-L-O-stéaroyl-séryl-D-isoglutamine,

N-acétyl-muramyl-L-O-(ω -n-stéaroylamino-n-undécanoyl)thréonyl-D-isoglutamine,

N-acétyl-muramyl-L-L-[N-(3-hydroxy-étio-cholénoyl)-8-amino-hexanoyl]-γ -hydroxyprolyl-D-isoglutamine,

N-acétyl-muramyl-L-O-béhénoyl-tyrosyl-D-isoglutamine,

N-acétyl-muramyl-L-($C_\gamma$)-[tétracosylamido-glycyl]glutamyl-D-isoglutamine,

N-acétyl-muramyl-L-[(Cγ)-lauryl)-α-glutamyl-D-isoglutamine,

N-acétyl-normuramyl-L-γ -stéaroylamino-α-amino-butanoyl-D-isoglutamine,

N-acétyl-muramyl-L-(O-béhénoyl)-N-méthyl)-séryl-Disoglutamine,

diméthylester d'acide N-acétyl-muramyl-L-($N^\varepsilon$ -palmitoyl)lysyl-D-glutamique.

24 - Diméthylester d'acide N-benzoyl-normuramyl-L-(O-stéaroyl)-séryl-D-glutamique selon la revendication 1.

25 - N-benzoyl-normuramyl-L-(O-béhénoyl)-séryl-D-isoglutamine ou un de ses sels pharmaceutiquement utilisable selon la revendication 1.

26 - N-acétyl-isomuramyl-L-(O-béhénoyl)-séryl-D-isoblutamine ou un de ses sels pharmaceutiquement utilisable selon la revendication 1.

27 - N-acétyl-muramyl-L-(O-béhénoyl)-séryl-Disoglutamine ou un de ses sels pharmaceutiquement utilisable selon la revendication 1.

28 - Composé selon la revendication 1 choisi dans le groupe des composés suivants et de leurs sels

N-acétyl-muramyl-D-(O-oléoyl)-séryl-D-isoglutamine,

N-acétyl-1,4,8,0-triacétyl-normuramyl-L-(O-béhénoyl)séryl-D-isoglutamine,

N-acétyl-muramyl-L-(4-béhénoyloxy)-prolyl-D-isoglutami-nyl-γ -L-alanine, et

N-acétyl-muramyl-L-(O-béhénoyl)-séryl-D- γ -carboxy-α-glutamyl-glycinamide.

29 - Composés de formule I et leurs sels selon l'une des revendications 1-28 pour l'application dans un procédé de traitement therapeutique du corps humain ou animal.

30 - Les composés immunopotentialisant de la formule Ic selon l'une des revendications 10-22 et des sels de tels composés avec des groupes formateurs de sels.

31 - Composés de formule I selon l'une des revendications 1-28 comme moyen immunomodulateur.

32 - Préparations pharmaceutiquement utilisables contenant des composés de formule Ic selon l'une des revendications 10-22 ou des sels pharmaceutiquement utilisables de tels composés avec des groupes formateurs de sels.

33 - Preparations pharmaceutiques qui contiennent des composés pharmacologiquement éfficaces de formule I selon l'une des revendications 1-9 et 23-28 ou des sels pharmaceutiquement utilisables de tels composes avec des groupes formateurs de sels.

34 - Utilisation de composés pharmacologiquement utilisables selon l'une des revendications 1-28 et leurs sels à la production de préparations pharmaceutiques.

35 - Utilisation de composés de formule Ic selon l'une des revendications 10-22 et de sels pharmaceutiquement utilisables de tels composés avec des groupes formateurs de sels comme composés à effet immunopotentialisant.

36 - Préparations pharmaceutiques, qui contiennent au moins un antibiotique et au moins un dérivé de pyranose de formule I selon l'une des revendications 1-28 et/ou un de ses sels.

37 - Aliment du bétail et additifs d'aliment du bétail, qui contiennent au moins un antibiotique et au moins un muramylpeptide selon l'une des revendications 1-28 et/ou un de ses sels.

38 - Procédé de préparation de composés de formule I selon l'une des revendications 1-28 et des sels de tels composés avec des groupes formateurs de sels, caractérisé en ce que

a) dans un composé de formule I, dans lequel les groupes fonctionnels sont présents le cas échéant sous forme protégée, on sépare au moins un groupe facilement remplaçable par l'hydrogène, ou on transforme au moins un groupe fonctionnel dans les dérivés correspondant aux produits finaux ou

b) dans un composé de formule I, dans lequel les groupes fonctionnels sont présents le cas échéant sous forme protégée et dans lequel $R_8$ est un reste alkyle inférieur ou phénylalkyl inférieur, qui peut aussi être lié à $R_7$ et qui porte un groupe protecteur hydroxy-, mercapto-, amino- ou carboxy, qui le cas echeant se trouvent sous une forme permettant de réagir, et dans lequel les autres substituants qui ont la significationcitée dans la revendication à laquelle cette revendication renvoie, on introduit, le cas échéant par étape le reste $R_0$ par l'intermédiaire d'un groupe oxycarbonyle, mercaptocarbonyle et/ou iminocarbonyle,

c) dans un composé de formule

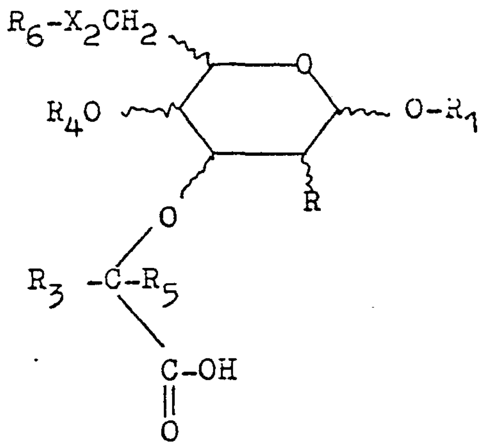
(IV)

dans lequel les substituants ont la signification citée dans la revendication à laquelle cette revendication renvoie dans lequel les groupes fonctionnels libres sont le cas échéant sous forme protégée, et dans lequel le groupe carboxy peut se présenter sous une forme dérivée, le groupe carboxy est amidé par des moyens de transfert le cas echéant par étape, avec l'un des restes de formule

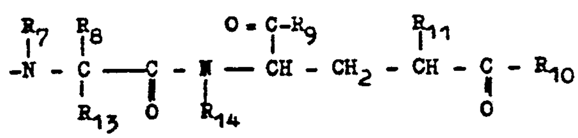
(IVa)

dans lequel les substituants ont la signification citée dans la revendication à laquelle cette revendication renvoie dans lequel les groupes fonctionnels sont le cas échéant sous forme protégée, et, si nécessaire dans un composé obtenu on transforme des groupes fonctionnels protégés en les groupes fonctionnels libres,

d) ou un composé de formule

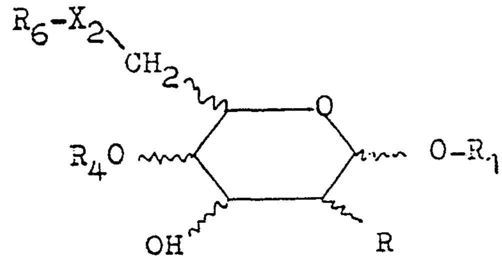
(VII),

dans lequel les substituants ont la signification citée dans la revendication à laquelle cette revendication renvoie dans lequel les groupes fonctionnels sont de préférence sous forme protégée, ou on condense un derivé pouvant réagir avec un composé de formule

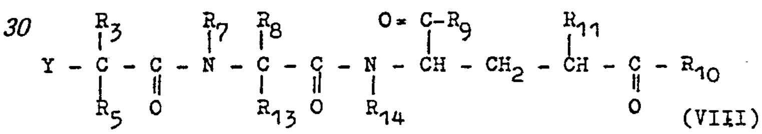
(VIII),

dans laquelle Y est un groupe hydroxy estérifié pouvant réagir et les autres substituants ont la signification citée dans la revendication à laquelle cette revendication renvoie, dans laquelle les groupes fonctionnels sont de préférence sous forme protégée, et, si nécessaire, dans un composé obtenu on transforme des groupes fonctionnels protégés en les groupes fonctionnels libres, et, si nécessaire, on transforme un composé de formule I obtenu selon ce procédé en un autre composé de formule I et/ou, si c'est souhaité, on transforme un sel d'un composé de formule I obtenu selon ce procédé avec un groupe formateur de sel dans le composé libre correspondant de formule I ou en un autre sel, et/ou si c'est souhaité on transforme un composé de formule I obtenu selon ce procédé en un sel avec un groupe formateur de sel, et/ou, si c'est souhaité on sépare un mélange d'isomères de composés de formule I obtenu selon ce procédé en les composés isomères de formule I.

39 - Procédé de préparation de composés qui ont la formule I décrite dans la revendication 1 selon l'une des revendications 2 ou 10-22 ou de sels de tels composés avec au moins un groupe formateur de sel, dans lesquels les substituants ont les significations citées dans la revendication concernée, avec la condition que R est un reste de formule

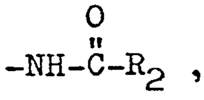

$R_1$, $R_4$ et $R_6$ sont l'hydrogène et $X_2$ est l'oxygène et que la partie pyranose dérive du D-glucose caractérisé en ce que, dans un composé de formule

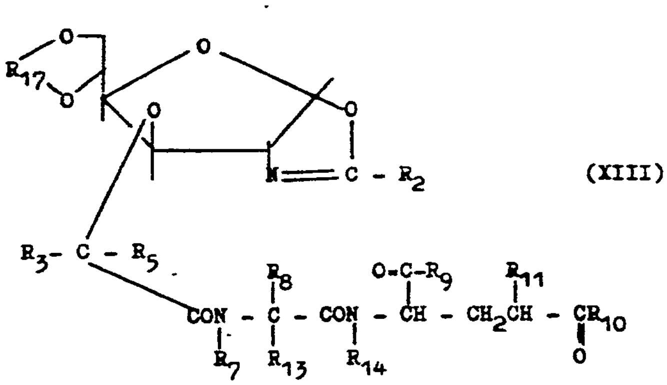

(XIII)

dans lequel $R_{17}$ est un groupe alkylidène ou cycloalkylidène et les autres substituants ont les significations citées plus haut, on sépare par traitement acide le cycle oxazoline et dioxol, et le cas échéant sépare le groupe protecteur présents, et transforme si c'est souhaité, un composé de formule Ic obtenu selon ce procédé en un autre composé de formule Ic, et/ou si c'est souhaité, on transforme un sel de composé de formule Ic obtenu selon ce procédé avec un groupe formateur de sel en le composé libre correspondant de formule Ic ou en un autre sel, et/ou, si c'est souhaité on sépare un mélange d'isomères de composés de formule Ic obtenus selon ce procédé en les composés isomères de formule Ic.

40 - Les composés qu'on peut obtenir selon les procédés des revendications 38 et 39.

41 - Peptides de formule II

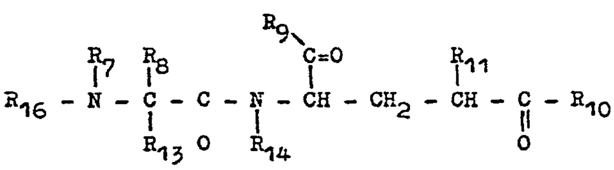

(II)

dans lesquels $R_{16}$ est l'hydrogène ou un groupe protecteur d'amino ou le reste

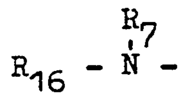

est un groupe amino activé et les autres substituants ont la signification citée dans l'une des revendications 1, 7, 10, 12-14 et 16, et des sels de composés avec des groupes formateurs de sel.

42 - Procédé de préparation de composés de formule (II), selon la revendication 41 et leurs sels, caractérisé en ce qu'on condense

a) un acide aminé de formule

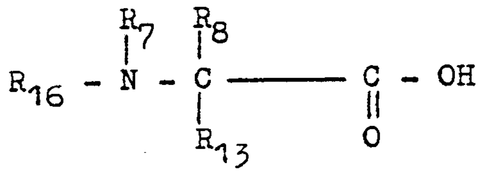

dans lequel $R_{16}$ est un groupe protecteur d'amino tandis que les autres substituants ont la signification citée dans la revendication 41, et le groupe carboxyle est le cas échéant sous forme activée, tandis que d'autres groupes fonctionnels sont le cas échéant sous forme protégée, avec un aminoacide de formule

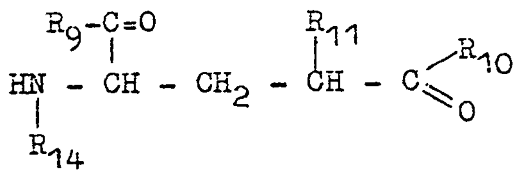

dans lequel le groupe

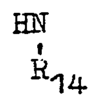

est le cas échéant sous forme activée, et les autres substituants ont la signification citee dans la revendication 41, où les groupes fonctionnels qui y sont présents peuvent être le cas échéant sous forme protégée, et, si c'est souhaité on separe au moins un groupe protecteur présent le cas échéant, et/ou, si c'est souhaité, on transforme une combinaison obtenue en son sel avec au moins un groupe formateur de sel, ou

b) dans un composé de formule II, dans lequel des groupes fonctionnels sont le cas échéant sous forme protégée, et dans lequel $R_8$ est un reste alkyle inférieur ou phénylalkyle inférieur qui peut aussi être lié à $R_7$ et porte un groupe hydroxy, mercapto, amino ou carboxy présent le cas échéant sous forme pouvant reagir, on introduit, le cas échéant par étape, par l'intermédiaire du groupe oxycarbonyle, mercaptocarbonyle ou iminocarbonyle le reste $R_o$,

qui est défini selon l'une des revendications 1, 7, 10, 12-14 et 16, et, si c'est souhaité, on sépare au moins un groupe protecteur présent le cas échéant, et/ou si c'est sou; haité, on transforme un composé obtenu en un sel avec au moins un groupe formateur de sel, ou

c) dans un composé de formule II, dans lequel des groupes fonctionnels sont le cas échéant sous forme protégée, on sépare au moins un groupe facilement remplaçable par l'hydrogène, on transforme au moins un groupe fonctionnel en les dérivés correspondant aux produits finaux.

43 - Les composés qu'on peut obtenir selon le procédé de la revendication 42 et leurs sels.

44 - Utilisation d'un peptide de formule II selon la revendication 41 comme produit intermédiaire pour la préparation de composés de formule I selon l'une des revendications 1-16.

**Revendications** pour l'Etat contractant: AT

1 - Procédé de préparation de dérivés pyranose de formule I

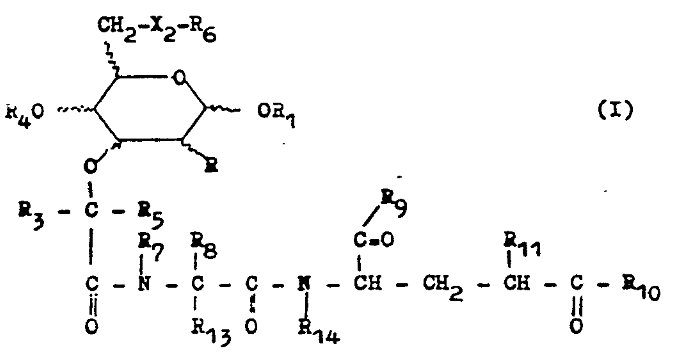

(I)

dans laquelle R représente un hydroxy, amino ou un reste de formule

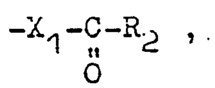

$X_1$ et $X_2$ représentent indépendamment l'un de l'autre $NR_{15}$ ou un atome d'oxygène, dans lequel $R_{15}$ est l'oxygène ou un alkyle inférieur, $R_1$ et $R_4$ sont indépendamment l'un de l'autre un hydrogene, acyle ou groupe protecteur d'hydroxy, $R_6$ est un hydrogène, acyle ou, si $X_2$ est un atome d'oxygène, un groupe protecteur d'hydroxy, $R_2$ est chaque fois un alkyle le cas échéant substitué, aryle ou alcoxy, $R_3$, $R_5$, $R_7$, $R_{13}$ et $R_{14}$ sont indépendamment l'un de l'autre de l'hydrogène ou un alkyle inférieur et $R_8$ est un reste alkyle inférieur ou phénylalkyl inférieur qui peut aussi être lié à $R_7$, et qui porte un groupe oxycarbonyle, mercaptocarbonyle ou aminocarbonyle, qui est lié à un reste hydrocarboné $R_0$ le cas échéant substitué aliphatique, cycloaliphatique-aliphatique, cycloaliphatique ou aromatique, ce dernier pouvant être interrompu par un oxycarbonyle, mercaptocarbonyle et/ou iminocarbonyle et qui doit présenter plus de 5 atomes de carbone, quand $R_1$, $R_4$ et $R_6$ représentent l'hydrogène, dans laquelle les restes $R_9$ et $R_{10}$ sont un hydroxy le cas èchéant éthérifié ou amino le cas échéant substitue, et $R_{11}$ est l'hydrogène ou un reste de formule $-C(=O)-R_{12}$ (Ia), dans laquelle $R_{12}$ est un hydroxy le cas échéant éthérifié ou amino le cas échéant substitué, dans lesquelles les restes qualifiés d'"inférieurs" contiennent jusqu'à 7 atomes de carbone, et des sels de composés de formule I avec au moins un groupe formateur de sel caractérisé en ce que

a) dans un composé de formule I, dans lequel les groupes fonctionnels sont présents le cas échéant sous forme protégée, on sépare au moins un groupe facilement remplaçable par l'hydrogène, ou on transforme aumoins un groupe fonctionnel dans les dérivés correspondant aux produits finaux,

b) dans un composé de formule I, dans lequel les groupes fonctionnels sont présents le cas échéant sous forme protégée et dans lequel $R_8$ est un reste alkyle inférieur ou phénylalkyle inférieur, qui peut aussi être lié à $R_7$ et qui porte un groupe protecteur hydroxy-, mercapto-, amino- ou carboxy, qui le cas échéant se trouvent sous une forme permettant de réagir, et dans lequel les autres substituants qui ont la signification citée plus haut, on introduit, le cas échéant par étape le reste $R_0$ par l'intermédiaire d'un groupe oxycarbonyle, mercaptocarbonyle et/ou iminocarbonyle, et, si nécessaire, dans un composé obtenu on transforme les groupes fonctionnels protégés en les groupes fonctionnels libres, ou

c) dans un composé de formule

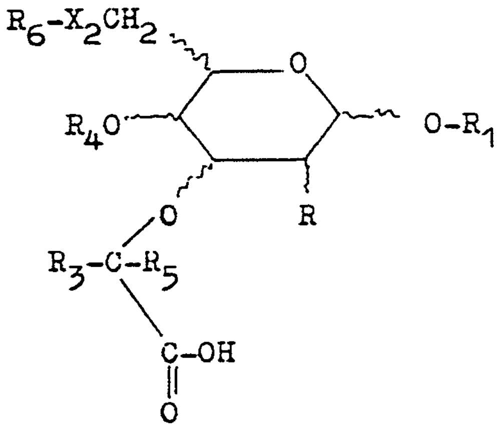

(IV)

dans lequel les substituants ont la signification citée plus haut, dans lequel les groupes fonctionnels libres sont le cas échéant sous forme protégée, et dans lequel le groupe carboxy peut se présenter sous une forme dérivée, le groupe carboxy est amidé par des moyens de transfert le cas échéant par étape, avec l'un des restes de formule

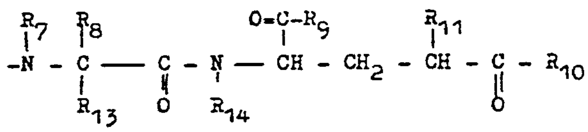

(IVa)

dans lequel les substituants ont la signification citée plus haut, dans lequel les groupes fonctionnels sont le cas echéant sous forme protégée, et, si nécessaire dans un composé obtenu on transforme des groupes fonctionnels protégés en les groupes fonctionnels libres,

d) ou un composé de formule

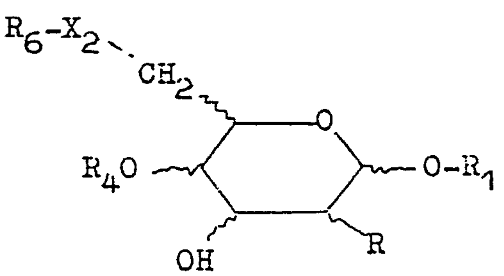

(VII),

dans lequel les substituants ont la signification citée plus haut, dans lequel les groupes fonctionnels sont de préférence sous forme protégée, ou d'un dérivé pouvant réagir avec un composé de formule

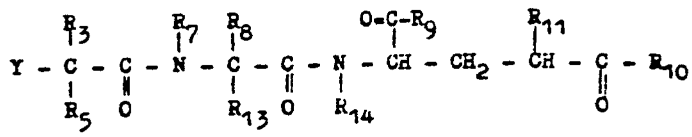

(VIII),

dans laquelle Y est un groupe hydroxy estérifié pouvant réagir et les autres substituants ont la signification citée plus haut

dans laquelle les groupes fonctionnels sont de préférence sous forme protégée, et, si nécessaire, dans un composé obtenu on transforme des groupes fonctionnels protégés en les groupes fonctionnels libres, ou

e) pour la préparation d'un composé de formule I, dans lequel R est un reste de formule

$$-NH-\overset{O}{\overset{\parallel}{C}}-R_2 \ ,$$

$R_1$, $R_4$ et $R_6$ sont l'hydrogène et $X_2$ est l'oxygène, et dans lequel la partie pyranose dérive du D-glucose et les autres substituants ont les significations citées plus haut, ou d'un sel d'un tel composé avec au moins un groupe formateur de sel un composé de formule

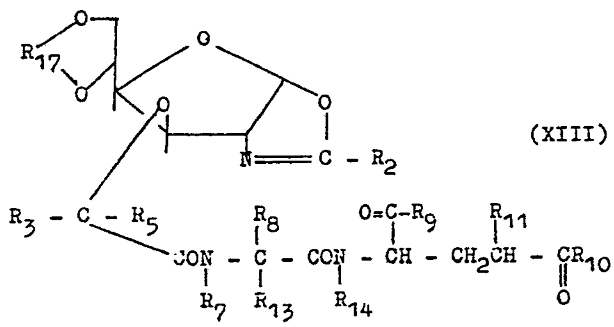

(XIII)

dans lequel $R_{17}$ est un groupe alkylidène ou cycloalkylidène et les autres substituants ont les significations citées plus haut, on sépare par traitement acide le cycle oxazoline et dioxol, et le cas échéant sépare le groupe protecteur présents, et transforme si c'est souhaité, un composé de formule I obtenu selon ce procédé en un autre composé de formule I, et/ou si c'est souhaité, on transforme un sel de composé de formule I obtenu selon ce procédé avec un groupe formateur de sel en le composé libre

correspondant de formule I ou en un autre sel, et/ou, si c'est souhaité on sépare un mélange d'isomères de composés de formule I obtenus selon ce procédé en les composés isomères de formule I.

2 - Procédé selon la revendication 1, caractérisé en ce qu'on prèpare des composés de formule I, dont la partie pyranose dérive du D-glucose.

3 - Procédé selon la revendication 1, caractérisé en ce qu'on prépare des composés de formule I, dont la partie pyranose dérive du D-mannose ou du D-galactose.

4 - Procédé selon l'une des revendications 1-3, caractérisé en ce qu'on prépare des composés de formule I, qui en cas de substitution asymétrique sur le C-$NR_{14}$ présentent la configuration (D).

5 - Procédé selon la revendication 4, caractérisé en ce qu'on prépare des composés de formule I, qui en cas de substitution asymétrique sur le C-$R_3$ et le C-$R_8$ présentent respectivement la configuration (D) et (L).

6 - Procédé selon l'une des revendications 1-5, caractérisé en ce qu'on prépare des composés de formule I, dans lesquels R est un reste de formule

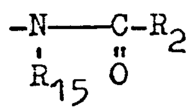

et $X_2$ est un atome d'oxygène.

7 - Procédé selon l'une des revendications 1-6, caractérisé en ce qu'on prépare des composés de formule I, dans lesquels $R_5$, $R_{13}$, $R_{14}$ et $R_{15}$ sont l'hydrogène.

8 - Procédé selon l'une des revendications 1-7, caractérisé en ce qu'on prépare des composés de formule I dans lesquels $R_1$, $R_4$ et $R_6$ sont l'hydrogène, le cas échéant un alcanoyle insaturé avec 2-25 atomes de C ou un benzoyle le cas échéant substitué.

9 - Procédé selon l'une des revendications 1-7, caractérisé en ce qu'on prépare des composés de formule I, dans lesquels $R_6$ est le reste

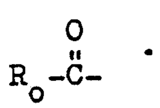

10 - Procédé selon la revendication 1, caractérisé en ce cu'on prépare des dérivés peptidiques de composés glucosamine de formule I

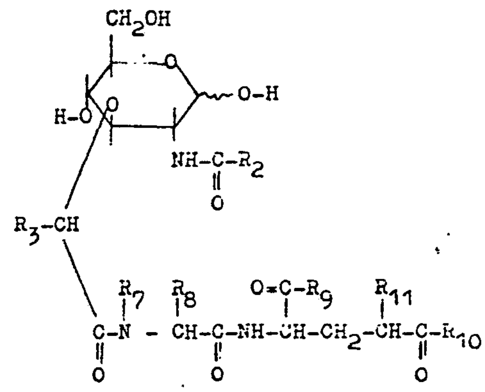

(Ic)

dans laquelle $R_2$ est un alkyle inférieur le cas échéant substitué, phényle, alcoxy inférieur ou phénylalcoxy inférieur, $R_3$ est l'hydrogène ou alkyle inférieur, $R_7$ l'hydrogène ou alkyle inférieur et $R_8$ un reste alkyle inférieur ou phénylalkylinférieur qui peut aussi ête lié à $R_7$ et qui porte un groupe oxycarbonyle, mercaptocarbonyle ou aminocarbonyle, qui est lié avec un reste hydrocarbonè aliphatique à longue chaine, le cas échéant substitué ou cycloaliphatique-aliphatique ce dernier pouvant être interrompu par un oxycarbonyle, mercaptocarbonyle et/ou iminocarbonyle, dans laquelle $R_9$ et $R_{10}$ sont un hydroxy le cas échéant éthérifié ou amino le cas échéant substitué, et $R_{11}$ est l'hydrogène ou un reste de formule $-C(=O)-R_{12}$ (Ia) dans laquelle $R_{12}$ est un hydroxy le cas échéant éthérifié ou amino le cas échéant substitué, ou des sels de composés de formule $I_c$ avec au moins un groupe formateur de sel.

11 - Procédé selon la revendication 10 caractérisé en ce qu'on prépare des composés de formule Ic dans lesquels le reste de l'acide hydroxyacétique avec le groupement de formule $-CH(R_3)-$, dans lequel $R_3$ represente un alkyle inférieur, est dans la forme D, le reste aminoacétique avec le groupement de formule $-CH(R_8)-$ est dans la forme L, et le reste du composé acide α-aminoglutarique terminal est sous la forme D ou les sels de tels composés avec au moins un groupe formateur de sel.

12 - Procédé selon la revendication 10 caractérisé en ce qu'on prépare des composés de formule Ic dans lesquels $R_2$ est un alkyle inférieur substitué le cas échéant par un hydroxy, alcoxy inférieur, alcanoyloxy inférieur ou halogène ou un alcoxy inférieur, ou un phényle substitué le cas échéant par un alkyle inférieur, hydroxy, alcoxy inférieur, alcanoyloxy inférieur ou halogène ou un phénylalcoxy inférieur, $R_3$ est l'hydrogène ou un alkyle inférieur, $R_7$ est l'hydrogène ou un alkyle

inférieur et $R_8$ est un reste alkyle inférieur ou phényl alkyle inférieur, qui porte comme substituant un groupe oxycarbonyle, mercaptocarbonyle ou aminocarbonyle, qui à son tour est lié à un reste alkyle ou alcényle substitué le cas échéant par un hydroxy alcoxy inférieur, alcanoyloxy inférieur, oxo ou halogène avec plus de 6 et jusqu'à 90 atomes de carbone ou à un reste alkyle ou alcényle substitué cycloaliphatique avec par exemple jusqu'à 30 atomes de carbone, et dans lesquels les restes alkyle, alcényle et alkyle ou alcényle substitués cycloaliphatique peuvent aussi être interrompus par un ou deux groupes oxycarbonyle ou imidocarbonyle, $R_{11}$ est l'hydrogène ou le reste de la formule Ia, et chacun des restes $R_9$, $R_{10}$ et $R_{12}$ est un hydroxy, alcoxy inférieur, amino ou amino substitué le cas échéant par un alkyle inférieur renfermant un carbamoyle, alcoxycarbonyle inférieur ou carboxy, et où dans de tels composés, par exemple le reste de l'acide hydroxyacétiqueavec le groupement de formule -CH($R_3$)-, dans lequel $R_3$ est un alkyle inférieur, est dans la forme D, le reste de l'acide aminoacétique avec le groupement de formule -CH($R_8$)-, est dans la forme L, et le reste du composé acide α-aminoglutarique terminal est dans la forme D, ainsi que les sels de tels composés avec au moins un groupe formateur de sel.

13 - Procédé selon la revendication 10 caractérisé en ce qu'on prépare des composés de formule Ic dans lesquels $R_2$ est un alkyle inférieur avec jusqu'à 4 atomes de carbone ou un phényle, $R_3$ est l'hydrogène ou un alkyle inférieur avec jusqu'à 4 atomes de carbone, $R_7$ est l'hydrogène et $R_8$ est un reste ülkyle inférieur avec 1-4 atomes de carbone ou un reste phénylalkyle inférieur, qui porte comme substituant un groupe oxycarbonyle, mercaptocarbonyle ou aminocarbonyle qui à son tour est lié à un alkyle ou alcényle substitué le cas échéant par un hvdroxy, alcoxy inférieur, oxo ou halogène avec plus de 10 et jusqu'à 50 atomes de carbone ou à un cycloalkyl-alkyle ou alcényl tétracyclique substitué le cas échéant par un hydroxy, alcoxy inférieur, oxo ou halogène avec plus de 20 et jusqu'à 50 atomes de carbone, et où de tels restes peuvent aussi etre interrompus par 1 ou 2 groupes oxycarbonyle ou iminocarbonyle, $R_9$ est un amino ou alkyl amino inférieur renfermant le cas échéant un carboxy ou carbamoyle, $R_{10}$ est un hydroxy et $R_{11}$ est l'hydrogène, où dans de tels composés, par exemple le reste de l'acide hydroxyacétique avec le groupement de formule -CH($R_3$)-, dans lequel $R_3$ est un alkyle inférieur, est dans la forme D, le reste de l'acide aminoacétique avec le groupement de formule -CH($R_8$)-, est dans la forme L, et le reste du composé acide α-aminoglutarique terminal est dans la forme D, ainsi que les sels pharmaceutiquement utilisables de tels composés avec au moins ungroupe formateur de sel.

14 - Procedé selon la revendication 10 caractérisé en ce qu'on prépare des composés de formule Ic dans lesquels $R_2$ est un alkyle inferieur avec jusqu'à 4 atomes de carbone, ou un phényle, $R_3$ est l'hydrogène, ou encore un alkyle inférieur avec jusqu'à 4 atomes de carbone, $R_7$ est l'hydrogene, $R_8$ est un reste alkyle inférieur avec 1-4 atomes de carbone ou benzyle qui portent comme substituant un groupe oxycarbonyle, mercaptocarbonyle ou aminocarbonyle, qui est lié de preférence par l'intermédiaire de l'hétéroatome au reste alkyle inférieur ou benzyle, et qui à son tour est lié à un alkyle ou alcényle substitué le cas échéant par un hydroxy, alcoxy inférieur ou oxo avec plus de 10 et jusqu'à 50 atomes de carbone ou à un reste cycloalkylalkyle ou cycloalcénylalkyle tétracyclique substitué le cas échéant par un hydroxy ou alcoxy inférieur avec plus de 20 et jusqu'à 50 atomes de carbone, ces restes peuvent aussi être interrompus par un groupe oxycarbonyle ou iminocarbonyle, $R_9$ est un amino, $R_{10}$ est un hydroxy et $R_{11}$ est l'hydrogéne, où dans de tels composés le reste de l'acide hydroxyacétique avec le groupement de formule -CH($R_3$)-, dans lequel $R_3$ est un alkyle inférieur, est dans la forme D, le reste de l'acide aminoacétique avec le groupement de formule -CH($R_8$)- est dans la forme L et le reste du composé acide α-amino-glutarique terminal est dans la forme D, et les sels pharmaceutiquement utilisables de tels composés avec au moins un groupe formateur de sels.

15 - Procédé selon la revendication 14, caractérisé en ce qu'on prépare des composés de formule Ic, dans lesquels $R_2$ est méthyle ou phényle, $R_3$ est l'hydrogéne ou méthyle et les autres restes ont les significations données dans la revendication 14.

16 - Procédé selon la revendication 10, caractérisé en ce qu'on prépare des composés de formule Ic dans lesquels $R_2$ est un alkyle inférieur avec jusqu'à 3 atomes de carbone ou un phényle, $R_3$ est l'hydrogéne ou un méthyle, $R_7$ est l'hydrogène, $R_8$ est un alkyle inférieur avec 1-3 atomes de carbone, qui porte comme substituant un groupe oxycarbonyle, mercaptocarbonyle ou aminocarbonyle, qui est lié par l'intermédiaire de l'hétéroatome au reste alkyle inférieur, et qui à son tour est lié à un alkyle ou alcényle substitué le cas échéant par un hydroxy, alcoxy inférieur ou oxo avec plus de 10 et jusqu'à 50 atomes de carbone ou à un reste cycloalkylalkyle ou cycloalcénylalkyl tétracyclique substitué le cas échéant par un hydroxy ou alcoxy inférieur avec plus de 20 et jusqu'à 50 atomes de carbone, ces restes peuvent aussi être interrompus par un groupe oxycarbonyle ou iminocarbonyle, $R_9$ est un amino, alkylamino inférieur ou carbamoylalkylamino ou hydroxy, $R_{10}$ est un hydroxy, et $R_{11}$ est l'hydrogène, où dans de tels composés le reste de l'acide hydroxyacétique avec le groupement de formule -CH($R_3$)-, dans lequel $R_3$ est le méthyle, est dans la forme D, le reste de l'acide aminoacétique avec le groupement de formule -CH($R_8$)-est dans la forme L et le reste du compose acide α-aminoglutarique terminal est dans la forme D et les sels pharmaceutiquement utilisables de tels composés avec au moins un groupe formateur de

sel.

17 - Procédé selon la revendication 11, caractérisé en ce qu'on prépare des composés de formule Ic dans lesquels $R_2$ est un alkyle inférieur avec jusqu'à 3 atomes de carbone, $R_3$ est l'hydrogène ou un methyle, $R_7$ est l'hydrogène, $R_8$ est un alkyle inférieur avec 1-4 atomes de carbone qui porte comme substituant un groupe oxycarbonyle, mercaptocarbonyle ou aminocarbonyle qui est lié par l'intermédiaire de l'hétéroatome au reste alkyle inférieur et qui à son tour est lié à un alkyle avec plus de 10 et jusqu'à 50 atomes de carbone, qui peut aussi être interrompu par un iminocarbonyle, $R_9$ est un amino, hydroxy ou alcoxy inférieur avec 1-3 atomes de carbone, $R_{10}$ est un hydroxy, alcoxy inferieur avec 1-3 atomes de carbone ou un amino et $R_{11}$ est l'hydrogène ou un carboxy et les sels pharmaceutiquement utilisables de tels composés avec au moins un groupe formateur de sel.

18 - Procédé selon la revendication 17, caractérisé en ce qu'on prépare des composés de formule I dans lesquels $R_8$ est un alcanoyloxymethyle ou alcanoyloxyéthyle avec plus de 10 et jusqu'à 30 atomes de carbone dans la partie alkyle du reste alcanoyle, $R_0$ est amino et $R_{10}$ est un hydroxy, ou leurs sels pharmaceutiquement utilisables.

19 - Procédé selon la revendication 10, caractérisé en ce qu'on prépare la N-acétyl-normuramyl-L-O(N-behénoyl-glycyl)-séryl-D-isoglutamine ou un de ses sels pharmaceutiquement utilisable.

20 - Procédé selon la revendication 10, caractérisé en ce qu'on prépare la N-acétyl-normuramyl-L-Obéhénoyl-séryl-D-isoglutamine ou un de ses sels pharmaceutiquement utilisable.

21 - Procédé selon la revendication 10, caractérisé en ce qu'on prépare la N-acétyl-muramyl-L-O-[N-(D,L-2-n-hexadécanoyl-amino-n-hexadécanoyl)-L-alanyl) -thréonyl-D-isoglutamine ou un de ses sels pharmaceutiquement utilisable.

22 - Procédé selon la revendication 10, caractérisé en ce qu'on prépare la N-acétyl-muramyl-L-S-stéaroyl-cystéinyl-D-isoglutamine ou un de ses sels pharmaceutiquement utilisable.

23 - Procédé selon la revendication 10, caractérisé en ce qu'on prépare un composé choisi dans le groupe des composés suivants et leurs sels:

N-acétyl-normuramyl-L-O-[N-(12-hydroxy-cis-9-octadécénoyl)-glycyl)-séryl-D-isoglutamine,

N-benzoyl-normuramyl-L-O-stéaroyl-séryl-D-isoglutamine,

N-acétyl-muramyl-L-O-(ε -n-stéaroylamino-n-undécanoyl)thréonyl-D-isoglutamine,

N-acétyl-muramyl-L-O-[N-(3-hydroxy-étio-cholénoyl)-6-amino-hexanoyl)- γ -hydroxyprolyl-D-isoglutamine,

N-acétyl-muramyl-L-O-béhénoyl-tyrosyl-D-isoglutamine,

N-acétyl-mursmyl-L-($C_\gamma$)-[tétracosylamido-glicyl)glutamyl-D-isoglutamine,

N-acétyl-muramyl-L-[($C_\gamma$)-lauryl)-α-glutamyl-D-isoglutamine,

N-acétyl-normuramyl-L-γ -stéaroylamino-α-amino-butanoyl-D-isoglutamine,

N-acétyl-muramyl-L-(O-béhénoyl-N-méthyl)-séryl-D-isoglutamine,

diméthylester d'acide N-acétyl-muramyl-L-($N^\delta$ -palmitoyl)-lysyl-D-glutamique.

24 - Diméthylester d'acide N-benzoyl-normuramyl-L-(O-stéaroyl)-séryl-D-glutamique.

25 - Procédé selon la revendication 10, caractérisé en ce qu'on prépare la N-benzoyl-normuramyl-L(O-béhénoyl)-séryl-D-isoglutamine ou un de ses sels pharmaceutiquement utilisable.

26 - Procédé selon la revendication 10, caractérisé en ce qu'on prépare la N-acétyl-isomuramyl-L-(O-béhénoyl)-séryl-D-isoglutamine ou un de ses sels pharmaceutiquement utilisable.

27 - Procédé selon la revendication 10, caractérisé en ce qu'on prépare la N-acétyl-muramyl-L-(Obéhénoyl)-séryl-D-isoglutamine ou un de ses sels pharmaceutiquement utilisable.

28 - Procédé selon la revendication 10, caractérisé en ce qu'on prépare un composé choisi dans le groupe des composés suivants et de leurs sels:

N-acétyl-muramyl-D-(O-oléoyl)-séryl-D-isoglutamine,

N-acétyl-1,4,6,0-triacétyl-normuramyl-L-(O-béhénoyl)séryl-D-isoglutamine,

N-acétyl-muramyl-L-(4-béhénoyloxy)-prolyl-D-isoglutami-nyl-γ -L-alanine et

N-acétyl-muramyl-L-(O-béhénoyl)-séryl-D-γ -carboxy-α-glutamyl-glycinamide.

29 - Procédé de préparation de peptides de formule II,

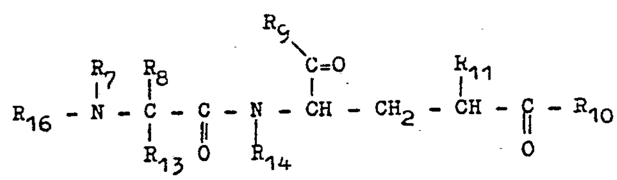

(II)

dans lequel $R_{16}$ est l'hydrogène ou un groupe protecteur d'amino et les autres substituants ont la signification citée dans l'une des revendications 1, 7, 10, 12-14 et 16-18 et de sels de tels composés avec des groupes formateurs de sels, caractérisé en ce qu'on condense

a) un acide amine de formule

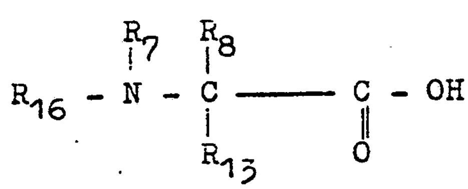

dans lequel $R_{16}$ est un groupe protecteur d'amino tandis que les autres substituants ont la signification citée plus haut et le groupe

carboxyle est le cas echeant sous forme activée, tandis que d'autres groupes fonctionnels sont le cas échéant sous forme protégée, avec un aminoacide de formule

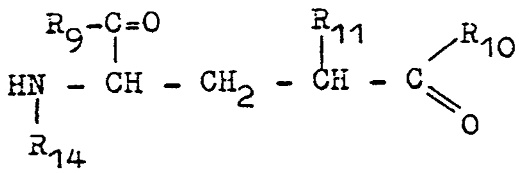

dans lequel le groupe

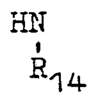

est le cas échéant sous forme activée, et les autres substituants ont la signification citée plus haut, où les groupes fonctionnels qui y sont présents peuvent être le cas échéant sous forme protegee, et, si c'est souhaité on sépare au moins un groupe protecteur présent le cas échéant, et/ou, si c'est souhaité, on transforme une combinaison obtenue en son sel avec au moins un groupe formateur de sel, ou

b) dans un composé de formule II, dans lequel des groupes fonctionnels sont le cas échéant sous forme protégée, et dans lequel $R_8$ est un reste alkyle inférieur ou phénylalkyle inférieur qui peut aussi être lié à $R_7$ et porte un groupe hydroxy, mercapto, amino ou carboxy présent le cas échéant sous forme pouvant réagir, on introduit, le cas échéant par étape, par l'intermediaire du groupe oxycarbonyle, mercaptocarbonyle ou iminocarbonyle le reste $R_o$, qui est défini selon l'une des revendications 1, 10, 12-14 et 16-18, et, si c'est souhaité, on sépare au moins un groupe protecteur present le cas échéant, et/ou si c'est souhaité, on transforme un composé obtenu en un sel avec au moins un groupe formateur de sel, ou

c) dans un composé de formule II, dans lequel des groupes fonctionnels sont le cas échéant sous forme protégée, on sépare au moins un groupe facilement remplaçable par l'hydrogène, on transforme au moins un groupe fonctionnel en les dérivés correspondant aux produits finaux.

30 - Procédé selon la revendication 29, caractérisé en ce qu'on choisisse les produits de départ de telle façon qu'on prépare un composé de formule II, qui sur le <u>C</u>-$R_8$ et le <u>C</u>-$NR_{14}$ présente respectivement la configuration (L) et (D).